(19) Europäisches Patentamt
European Patent Office
Office européen des brevets

(11) Veröffentlichungsnummer: **0 030 030**
**A1**

(12) # EUROPÄISCHE PATENTANMELDUNG

(21) Anmeldenummer: 80107499.8

(22) Anmeldetag: 01.12.80

(51) Int. Cl.³: C 07 C 143/80
C 07 C 103/29, C 07 C 143/74
C 07 D 317/58, C 07 C 103/85
A 61 K 31/16

(30) Priorität: 04.12.79 CH 10744/79

(43) Veröffentlichungstag der Anmeldung:
10.06.81 Patentblatt 81/23

(84) Benannte Vertragsstaaten:
AT BE CH DE FR IT LI LU NL SE

(71) Anmelder: CIBA-GEIGY AG
Patentabteilung Postfach
CH-4002 Basel(CH)

(72) Erfinder: Ostermayer, Franz, Dr.
Am Hang 5
CH-4125 Riehen(CH)

(72) Erfinder: Zimmermann, Markus, Dr.
Steinbrecheweg 8
CH-4125 Riehen(CH)

(72) Erfinder: Fuhrer, Walter, Dr.
Munzackerweg 11
CH-4402 Frenkendorf(CH)

(74) Vertreter: Zumstein sen., Fritz, Dr. et al,
Bräuhausstrasse 4
D-8000 München 2(DE)

(54) **Derivate des 2-Amino-äthanols, Verfahren zu ihrer Herstellung, pharmazeutische Präparate enthaltend solche Verbindungen und Verwendung von letzteren.**

(57) Neue Derivate des 2-Aminoäthanols der Formel

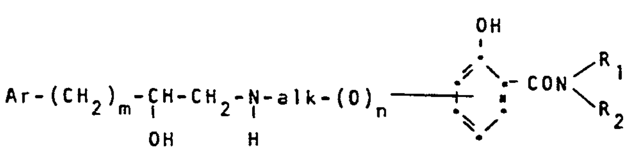

(1)

in welcher Ar gegebenenfalls substituiertes Phenyl, m eine Zahl von 0 bis 3, n die Zahl 0 oder 1 und alk Alkylen mit 2-5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind, und $R_1$ und $R_2$ unabhängig voneinander je Wasserstoff oder Niederalkyl, oder zusammen Niederalkylen, Oxaniederalkylen, Thianiederalkylen, Azaniederalkylen oder N-Niederalkylazaniederalkylen darstellt mit der Massgabe, das falls m für 0 steht, der Phenylrest Ar mindestens einen Substituenten enthält, und ein durch eine oder zwei Hydroxygruppen oder geschützte Hydroxygruppen substituierter Phenylrest Ar mindestens einen zusätzlichen, von diesen ver-

./...

EP 0 030 030 A1

schiedenen Substituenten enthält, in der Form von Racematgemischen, Racematen, optischen Antipoden oder deren Salzen. Solche Verbindungen wirken teilweise als Blocker, teilweise als Stimulatoren von $\beta$-adrenergen Rezeptoren mit mehr oder weniger ausgeprägter Cardioselektivität und können demnach einesteils bei den für $\beta$-Blocker üblichen Indikationen, anderenteils zur Behandlung der insuffizienten Herzleistung, von Asthma und Durchblutungsstörungen eingesetzt werden.

CIBA-GEIGY AG 4-12619/+

Basel (Schweiz)

Derivate des 2-Amino-äthanols, Verfahren zu ihrer Herstellung, pharmazeutische Präparate enthaltend solche Verbindungen und Verwendung von letzteren

Die Erfindung betrifft neue Derivate des 2-Aminoäthanols, Verfahren zu ihrer Herstellung und pharmazeutische Präparate enthaltend solche Verbindungen und ihre Verwendung zur Herstellung von pharmazeutischen Präparaten oder als pharmakologisch wirksame Verbindungen.

Die erfindungsgemässen neuen Derivate des 2-Amino-äthanols entsprechen der Formel

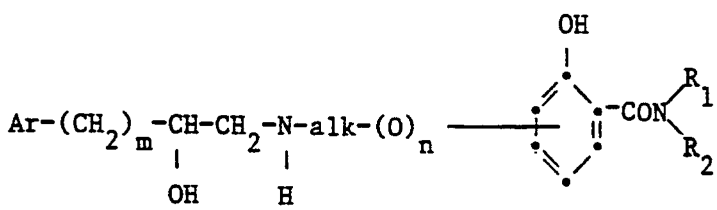

(I),

in welcher Ar gegebenenfalls substituiertes Phenyl, m eine Zahl von 0 bis 3, n die Zahl 0 oder 1 und alk Alkylen mit 2-5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind, und $R_1$ und $R_2$ unabhängig voneinander je Wasserstoff oder Niederalkyl, oder zusammen Niederalkylen, Oxaniederalkylen, Thianiederalkylen, Azaniederalkylen oder N-Niederalkylazaniederalkylen darstellt mit der Massgabe, dass falls m für 0 steht, der Phenylrest Ar mindestens einen Substituenten enthält und ein durch eine oder zwei Hydroxygruppen oder geschützte Hydroxygruppen substituierter Phenylrest Ar mindestens einen zusätzlichen, von diesen verschiedenen Substituenten enthält,

in der Form von Racematgemischen, Racematen, optischen Antipoden oder deren Salzen, insbesondere Säureadditionssalzen, und vor allem pharmazeutisch annehmbaren, nicht-toxischen Säureadditionssalzen.

Als Substituenten des Restes Ar können beispielsweise gegebenenfalls, insbesondere in untenstehend angegebener Weise, substituiertes Niederalkyl, Niederalkenyl oder Niederalkoxy, weiter Niederalkenyloxy, Niederalkinyl, Niederalkinyloxy, Niederalkylidendioxy, Cyan und/oder Nitro und/oder direkt oder an vorgenanntes Niederalkyl, Niederalkenyl oder Niederalkoxy gebundenes, d.h. eine dieser Gruppen substituierendes Niederalkanoyl, verestertes oder amidiertes Carboxyl, insbesondere Niederalkoxycarbonyl bzw. gegebenenfalls substituiertes Carbamoyl, z.B. Carbamoyl, Niederalkylcarbamoyl, Diniederalkylcarbamoyl, oder (Hydroxyniederalkyl)-carbamoyl, Niederalkylthio, Niederalkylsulfinyl, Niederalkylsulfonyl, Sulfamoyl oder Niederalkylsulfamoyl, Diniederalkylsulfamoyl, oder direkt oder an vorgenanntes Niederalkyl oder in höherer als der 1-Stellung an vorgenanntes Niederalkoxy gebundenes Halogen, veräthertes Mercapto, wie Niederalkylthio, gegebenenfalls substituiertes Amino, wie Amino, Niederalkylamino, Diniederalkylamino, Alkylen- oder Oxaalkylenamino, Pyrrol-1-yl, Acylamino, wie Niederalkanoylamino oder Niederalkoxycarbonylamino, gegebenenfalls, insbesondere durch ein oder zwei Niederalkyl, durch Hydroxyniederalkyl oder Cycloalkyl substituiertes Ureido, Niederalkylsulfonylamino oder gegebenenfalls veräthertes oder verestertes Hydroxy, wie Hydroxy, Phenylniederalkoxy bzw. Niederalkanoyloxy oder, als nicht direkt gebundender Substituent, wiederum Niederalkoxy vorliegen, mit der Massgabe, dass, falls m für 0 steht, der Arylrest Ar mindestens einen Substituenten und ein durch eine oder zwei Hydroxy-, 1-Phenylniederalkoxy- oder Niederalkanoyloxygruppen substituierter Phenylrest mindestens einen zusätzlichen, von diesen verschiedenen Substituenten enthält.

Die im Zusammenhang mit der vorliegenden Beschreibung mit "nieder" bezeichneten Reste und Verbindungen enthalten vorzugsweise

bis 7 und in erster Linie bis 4 Kohlenstoffatome.

Die in der Aufzählung von Substituenten des Restes Ar verwendeten Allgemeinbegriffe können z.B. die folgenden spezifischen Bedeutungen haben. Niederalkyl ist z.B. Methyl, Aethyl, n-Propyl, Isopropyl, n-Butyl, Isobutyl oder tert.-Butyl; substituiertes Niederalkyl ist insbesondere entsprechendes Methyl oder 1- oder 2-substituiertes Aethyl, Niederalkenyl ist z.B. Vinyl, Allyl, 2- oder 3-Methallyl oder 3,3-Dimethylallyl, und substituiertes Niederalkenyl insbesondere 2-substituiertes Vinyl oder 3-substituiertes Allyl. Niederalkoxy ist z.B. Methoxy, Aethoxy, n-Propyloxy, Isopropyloxy, n-Butyloxy oder Isobutyloxy, und substituiertes Niederalkoxy z.B. substituiertes Methoxy- oder 1- oder 2-substituiertes Aethoxy. Niederalkenyloxy ist z.B. Allyloxy, 2- oder 3-Methallyloxy oder 3,3-Dimethylallyloxy. Niederalkinyl ist z.B. Propargyl, und Niederalkinyloxy insbesondere Propargyloxy; Niederalkylidendioxy ist z.B. Isopropylidendioxy, Aethylidendioxy und insbesondere Methylendioxy, und Alkylendioxy insbesondere Aethylendioxy. Niederalkanoyl ist z.B. Acetyl, Propionyl oder Butyryl. Niederalkoxycarbonyl ist z.B. Methoxycarbonyl oder Aethoxycarbonyl. Niederalkyl- oder Diniederalkylcarbamoyl ist z.B. Methylcarbamoyl, Dimethylcarbamoyl, Aethylcarbamoyl oder Diäthylcarbamoyl, und Hydroxyniederalkylcarbamoyl z.B. (2-Hydroxyäthyl)-carbamoyl. Niederalkylthio ist z.B. Methylthio, Aethylthio, n-Propylthio oder Isopropylthio. Niederalkylsulfinyl ist z.B. Methyl- oder Aethylsulfinyl, Niederalkylsulfonyl z.B. Methyl-, Aethyl- oder n-Propylsulfonyl, und Niederalkylsulfamoyl z.B. Methyl-, Aethyl- oder Isopropylsulfamoyl, und Diniederalkylsulfamoyl z.B. Dimethylsulfamoyl. Halogen ist Brom oder Jod und vorzugsweise Fluor oder Chlor. Phenylniederalkoxy ist z.B. Benzyloxy oder 1- oder 2-Phenyläthoxy, und Niederalkanoyloxy z.B. Formyloxy, Acetoxy; Niederalkylamino und Diniederalkylamino sind z.B. Methylamino, Aethylamino,

Dimethylamino oder Diäthylamino. Alkylenamino und Oxaalkylenamino sind z.B. Pyrrolidino oder Piperidino bzw. Morpholino. Niederalkanoylamino ist z.B. Acetylamino oder Butyrylamino, und Niederalkoxycarbonylamino z.B. Methoxycarbonylamino oder Aethoxycarbonylamino. Durch ein oder zwei Niederalkyl, durch Hydroxyniederalkyl oder durch Cycloalkyl, vorzugsweise solches mit 5 bis 7 Ringgliedern, substituiertes Ureido ist z.B. 3-Methylureido, 3,3-Dimethylureido, 3-(2-Hydroxyäthyl)-ureido bzw. 3-Cyclohexylureido. Niederalkylsulfonylamino ist z.B. Aethylsulfonylamino und insbesondere Methylsulfonylamino.

Wie oben angegeben, können Substituenten von Ar aus einem der vorgenannten Reste, der nicht direkt, sondern über Niederalkyl, Niederalkoxy oder gegebenenfalls über Niederalkenyl gebunden ist, bestehen. Nachstehend werden eine Auswahl solcher Substituenten allgemein und spezifisch als Beispiele genannt, ohne die Kombinationsmöglichkeiten darauf zu beschränken. Niederalkanoylalkyl ist z.B. 2-Oxopropyl (Acetonyl) oder 3-Oxobutyl, Niederalkanoylniederalkenyl z.B. 3-Oxo-1-butenyl, und Niederalkanoylalkoxy z.B. 2-Oxopropoxy (Acetonyloxy) oder 3-Oxobutoxy. Gegebenenfalls substituiertes Carbamoylniederalkyl ist z.B. Carbamoylmethyl oder [(Hydroxyniederalkyl)-carbamoyl)]-niederalkyl, wie [(2-Hydroxyäthyl)-carbamoyl]-methyl. Niederalkoxy-carbonylniederalkoxy ist z.B. Aethoxycarbonylmethoxy. Gegebenenfalls substituiertes Carbamoylniederalkoxy ist z.B. Carbamoylniederalkoxy, wie Carbamoylmethoxy, oder [(Hydroxyniederalkyl)-carbamoyl]-niederalkoxy, wie [(2-Hydroxyäthyl)-carbamoyl]-methoxy. Halogenniederalkyl ist insbesondere Halogenmethyl, z.B. Trifluormethyl. Niederalkylthioniederalkoxy ist, z.B. 2-Methylthioäthoxy oder 2-Aethylthio-äthoxy. Acylaminoniederalkyl ist z.B. Niederalkanoylaminoniederalkyl, insbesondere Niederalkanoylaminomethyl oder 1- und in erster Linie 2-Niederalkanoylamino-äthyl, z.B. Acetylaminomethyl, 2-Acetylamino-äthyl oder 2-Propionylaminoäthyl, oder Niederalkoxycarbonylaminoniederalkyl, insbesondere Niederalkoxycarbonylaminomethyl oder 1- und in erster Linie 2-Niederalkoxycarbonylamino-äthyl, z.B. Methoxycarbonylaminomethyl, 2-Methoxycarbonylamino-äthyl oder 2-Aethoxycarbonylamino-äthyl. Acylaminoniederalkoxy ist z.B. Niederalkanoylaminoniederalkoxy, insbesondere 2-Niederalkanoylaminoäthoxy, z.B. 2-(Acetylamino)-äthoxy, oder Niederalkoxycarbonylaminoniederalkoxy, insbesondere 2-(Niederalkoxycarbonylaminoäthoxy, z.B. 2-(Methoxycarbonylamino)-äthoxy oder 2-(Aethoxycarbonylamino)-äthoxy. Hydroxyniederalkyl ist vorzugsweise Hydroxymethyl oder 1- und in erster Linie 2-Hydroxyäthyl. Niederalkoxyniederalkyl ist vorzugsweise Niederalkoxymethyl oder 1- und in erster Linie 2-Niederalkoxyäthyl, z.B. Methoxymethyl, Aethoxymethyl, 2-Methoxyäthyl oder 2-Aethoxyäthyl. Niederalkoxyniederalkoxy ist insbesondere 2-Niederalkoxyäthoxy, wie 2-Methoxyäthoxy oder 2-Aethoxyäthoxy.

Alkylen alk kann geradkettig oder verzweigt sein und ist z.B. 1,2-Aethylen, 1,2-, 2,3- oder 1,3-Propylen, 1,4- oder 2,4-Butylen, 2-Methyl-2,4-butylen, oder 1,1-Dimethyläthylen.

$R_1$ und $R_2$ in der Bedeutung von Niederalkyl sind beispielsweise Propyl, Isopropyl, Butyl, Isobutyl, Sek.-butyl, Pentyl, Isopentyl, Neopentyl, Hexyl oder Heptyl, und vor allem Methyl oder Aethyl. Zusammen mit dem Stickstoffatom der Amidgruppe sind $R_1$ und $R_2$ als Niederalkylen beispielsweise 1-Aziridinyl, 1-Azetidinyl, 1-Pyrrolidinyl, Piperidino, Hexahydro-1H-azepin-1-yl, als Oxaniederalkylen z.B. Morpholino, als Thianiederalkylen z.B. Thiomorpholino; als Azaniederalkylen z.B. 1-Piperazinyl oder Hexahydro-1H-1,4-diazepin-1-yl, wobei die beiden letztgenannten Gruppen entsprechend der Bedeutung von N-Niederalkylazaniederalkylen für $R_1$ und $R_2$ in 4-Stellung, d.h. in der Iminogruppe, z.B. durch Niederalkyl, wie Methyl, Aethyl, Propyl, Isopropyl, Butyl oder Isobutyl, substituiert sein können.

Das die Amid- und die benachbarte Hydroxygruppe tragende Phenyl kann in beliebiger Stellung mit dem restlichen Molekül verbunden sein, vorzugsweise ist letzteres in der 4-Stellung des genannten Phenyls, d.h. in der para-Stellung zur Amidgruppe, und vor allem in der 5-Stellung des genannten Phenyls, d.h. in para-Stellung zur Hydroxygruppe gebunden.

Die neuen Verbindungen können in Form ihrer Salze, wie ihrer Säureadditionssalze und in erster Linie ihrer pharmazeutisch verwendbaren, nicht-toxischen Säureadditionssalze vorliegen. Geeignete Salze sind z.B. solche mit anorganischen Säuren, wie Halogenwasserstoffsäuren, z.B. Chlorwasserstoffsäure oder Bromwasserstoffsäure, Schwefelsäure, oder Phosphorsäure, oder mit organischen Säuren, wie aliphatischen, cycloaliphatischen, aromatischen oder heterocyclischen Carbon- oder Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, Embon-, Methansulfon-, Aethansulfon-, 2-Hydroxyäthansulfon-, Aethylensulfon-, Toluolsulfon-, Naphthalinsulfon- oder Sulfanilsäure, oder mit anderen sauren organischen Stoffen, wie Ascorbinsäure.

Die Erfindung betrifft insbesondere Verbindungen der Formel I, worin Ar gegebenenfalls ein- oder mehrfach, vorzugsweise höchstens dreifach, substituiert ist, wobei als Substituenten gegebenenfalls, insbesondere in untenstehend angegebener Weise, substituiertes Niederalkyl, Niederalkenyl oder Niederalkoxy, weiter Niederalkenyloxy, Niederalkinyl, Niederalkinyloxy, Niederalkylidendioxy, Niederalkylendioxy, Cyan und/oder Nitro, und/oder direkt oder an vorgenanntes Niederalkyl, Niederalkenyl oder Niederalkoxy gebundenes Niederalkanoyl, verestertes oder amidiertes Carboxyl, insbesondere Niederalkoxycarbonyl, Carbamoyl, Niederalkylcarbamoyl oder (Hydroxyniederalkyl)-carbamoyl, Niederalkylsulfinyl, Niederalkylsulfonyl, Sulfamoyl oder Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Acylamino, wie Niederalkanoylamino oder Niederalkoxycarbonylamino, gegebenenfalls durch Niederalkyl, Hydroxyniederalkyl oder Cycloalkyl substituiertes Ureido, Niederalkylsulfonylamino, Hydroxy, Phenylniederalkoxy, z.B.

Benzyloxy, Niederalkanoyloxy oder, als nicht direkt gebundener Substituent, wiederum Niederalkoxy vorliegen können, m für eine Zahl von 0 bis 3 steht, n für die Zahl 0 oder 1 steht und alk einen Alkylenrest mit 2 bis 4 Kohlenstoffatomen bedeutet, wobei das Stickstoffatom und das Sauerstoffatom oder, falls n für 0 steht, der Phenylrest, durch 2 bis 3 Kohlenstoffatome der Reste alk voneinander getrennt sind, $R_1$ und $R_2$ die unter Formel I angegebene Bedeutung haben, jedoch vorzugsweise für Wasserstoff oder Niederalkyl, z.B. Methyl oder Aethyl, stehen, oder zusammen mit dem Stickstoffatom der Amidgruppe Morpholino oder Alkylenamino mit 5 bis 6 Ringgliedern, wie 1-Pyrrolidinyl oder Piperidino bilden, mit der Massgabe, dass, falls m für 0 steht, der Phenylrest Ar mindestens einen Substituenten und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft vorzugsweise Verbindungen der Formel I, worin Ar gegebenenfalls ein- bis dreifach substituiert ist, wobei als Substituenten gegebenenfalls in untenstehend angegebener Weise substituiertes Niederalkyl, Niederalkenyl oder Niederalkoxy, weiter Niederalkenyloxy, Niederalkinyl, Niederalkinyloxy, Niederalkylidendioxy, Cyan und/oder Nitro und/oder direkt oder an vorgenanntes Niederalkyl, Niederalkenyl oder Niederalkoxy gebundenes Niederalkanoyl, Niederalkoxycarbonyl, Carbamoyl, Niederalkylcarbamoyl, (Hydroxyniederalkyl)-carbamoyl, Niederalkylsulfinyl, Niederalkylsulfonyl, Sulfamoyl, Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl,

Niederalkanoylamino, Niederalkoxycarbonylamino, gegebenenfalls durch Niederalkyl, Hydroxyniederalkyl oder Cycloalkyl substituiertes Ureido, Niederalkylsulfonylamino, Phenylniederalkoxy, z.B. Benzyloxy, Niederalkanoyloxy oder, als nicht direkt gebundener Substituent wiederum Niederalkoxy vorliegen können, m für eine Zahl von 0 bis 3 steht und n die Zahl 0 oder 1, und alk einen Alkylenrest mit 2 bis 4 Kohlenstoffatomen bedeutet, wobei das Stickstoffatom und das Sauerstoffatom, oder, falls n für 0 steht, der Phenylrest, durch 2 bis 3 Kohlenstoffatome des Restes alk voneinander getrennt sind, $R_1$ und $R_2$ die unter Formel I angegebene Bedeutung haben, jedoch vorzugsweise für Wasserstoff oder Niederalkyl, insbesondere Methyl oder Aethyl, stehen oder zusammen mit dem Stickstoffatom der Aminogruppe 1-Pyrrolidinyl, Piperidino oder Morpholino bilden, mit der Massgabe, dass, falls m für 0 steht, der Phenylrest Ar mindestens einen Substituenten und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft besonders Verbindungen der Formel I, worin Ar gegebenenfalls ein- bis dreifach substituiert ist, wobei als Substituenten gegebenenfalls in untenstehend angegebener Weise substituiertes Niederalkyl, Niederalkenyl oder Niederalkoxy, weiter Niederalkenyloxy, Niederalkinyloxy, Niederalkylidendioxy, Nitro und/oder Cyan und/oder direkt oder an vorgenanntes Niederalkyl, Niederalkenyl oder Niederalkoxy gebundenes Niederalkanoyl, Niederalkoxycarbonyl, Carbamoyl, Niederalkylcarbamoyl, (Hydroxyniederalkyl)-carbamoyl, Niederalkylsulfinyl, Niederalkylsulfonyl, Sulfamoyl oder Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino,

Diniederalkylamino, Alkylen- oder Oxaalkylenamino, z.B. 1-Pyrrolidinyl, Piperidino oder Morpholino, Pyrrol-1-yl, Niederalkanoylamino, Niederalkoxycarbonylamino, gegebenenfalls durch Niederalkyl oder Cycloalkyl substituiertes Ureido, Niederalkylsulfonylamino, Hydroxy, Phenylniederalkoxy, z.B. Benzyloxy, Niederalkanoyloxy oder, als nicht direkt gebundener Substituent, wiederum Niederalkoxy vorliegen können, m für eine Zahl von 0 bis 3 steht, mit der Massgabe, dass,falls m für 0 steht, der Phenylrest Ar mindestens einen Substituenten, und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, n die Zahl 0 oder 1 und alk einen Alkylenrest mit 2 bis 4 Kohlenstoffatomen bedeutet, wobei das Stickstoffatom und das Sauerstoffatom, oder, falls n für 0 steht, der Phenylrest, durch 2 bis 3 Kohlenstoffatome des Restes alk voneinander getrennt sind, und $R_1$ und $R_2$ unabhängig voneinander je Wasserstoff oder Niederalkyl, insbesondere Methyl oder Aethyl darstellen, oder zusammen mit dem Stickstoffatom der Amidgruppe Morpholino bilden, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft vor allem Verbindungen der Formel I, worin Ar gegebenenfalls ein- bis dreifach substituiert ist, wobei als Substituenten gegebenenfalls in untenstehend angegebener Weise substituiertes Niederalkyl oder Niederalkoxy, weiter Niederalkenyl, Niederalkenyloxy, Niederalkinyloxy, Niederalkylidendioxy, Nitro und/oder Cyan und/oder direkt oder an vorgenanntes Niederalkyl oder Niederalkoxy gebundenes Niederalkanoyl, Niederalkoxycarbonyl, Carbamoyl, Niederalkalkylcarbamoyl oder (Hydroxyniederalkyl)-carbamoyl, Niederalkylsulfinyl, Niederalkylsulfonyl, Sulfamoyl und/oder direkt oder an vorgenanntes Niederalkyl oder in höherer als der 1-Stellung an vorgenanntes Niederalkoxy gebundenes Halogen,

Niederalkylthio, Amino, Alkylen- oder Oxaalkylenamino, z.B.1-Pyrrolidinyl, Piperidino oder Morpholino, Pyrrol-1-yl,Niederalkanoylamino oder Niederalkoxycarbonylamino, oder gegebenenfalls durch Niederalkyl substituiertes Ureido, Niederalkylsulfonylamino, Hydroxy, weiter Phenylniederalkoxy, z.B. Benzyloxy, oder, als nicht direkt gebundener Substituent, wiederum Niederalkoxy vorliegen können, m für eine Zahl von 0 bis 3 steht, mit der Massgabe dass, falls m für 0 steht, der Phenylrest Ar mindestens einen Substituenten, und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, n die Zahl 0 oder 1 und alk einen Alkylenrest mit 2 bis 4 Kohlenstoffatomen bedeutet, wobei das Stickstoffatom und das Sauerstoffatom oder, falls n für 0 steht, der Phenylrest, durch 2 bis 3 Kohlenstoffatome des Restes alk voneinander getrennt sind, und $R_1$ und $R_2$ unabhängig voneinander je Wasserstoff oder Niederalkyl, jedoch vorzugsweise Wasserstoff oder Methyl darstellen, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft in erster Linie Verbindungen der Formel I, worin Ar gegebenenfalls ein- bis dreifach substituiert ist, wobei als Substituenten gegebenenfalls in untenstehend angegebener Weise substituiertes Niederalkyl, z.B. Methyl, oder Niederalkoxy, z.B. Methoxy oder Aethoxy, weiter Niederalkenyl, z.B. Allyl, Niederalkenyloxy, z.B. Allyloxy, Niederalkinyloxy, z.B. Propargyloxy, Nitro, Niederalkylidendioxy, z.B. Methylendioxy, und/oder Cyan und/oder direkt oder an vorgenanntes Niederalkyl oder Niederalkoxy gebundenes Niederalkanoyl, z.B. Acetyl,Carbamoyl, Niederalkylcarbamoyl, z.B. N-Methylcarbamoyl, oder (Hydroxyniederalkyl)-carbamoyl, z.B. N-Hydroxymethyl-carbamoyl, Niederalkylsulfonyl, z.B. Methylsulfonyl,Sulfamoyl, und/oder direkt oder an

vorgenanntes Niederalkyl, z.B. Methyl, gebundenes Fluor oder Chlor, z.B. Trifluormethyl, Hydroxy, oder als direkt gebundene Substituenten Phenylniederalkoxy z.B. Benzyloxy, Niederalkoxyniederalkoxy, z.B. 2-Methoxyäthoxy, Benzyloxy, Amino, Niederalkanoylamino, z.B. Acetylamino, Niederalkoxycarbonylamino, z.B. Methoxycarbonylamino, oder Niederalkylsulfonylamino, z.B. Methylsulfonylamino vorliegen können, m für eine Zahl von 0 bis 3 steht, mit der Massgabe, dass, falls m für 0 steht, der Phenylrest Ar mindestens einen Substituenten und ein durch ein oder zwei Hydroxy- oder 1-Phenylniederalkoxygruppen substituierter Phenylrest Ar mindestens einen zusätzlichen, von diesen verschiedenen Substituenten enthält, n die Zahl 1 und alk einen Alkylenrest mit 2 bis 4 Kohlenstoffatomen bedeutet, wobei das Stickstoffatom und das Sauerstoffatom durch 2 bis 3 Kohlenstoffatome des Restes alk voneinander getrennt sind, und $R_1$ und $R_2$ Wasserstoff darstellen, und der die Amid- und die benachbarte Hydroxygruppe tragende Phenylrest vorzugsweise in seiner 4- oder 5-Stellung mit dem restlichen Molekül verbunden ist, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft vor allem Verbindungen der Formel I, worin Ar gegebenenfalls ein- bis dreifach substituiert ist, wobei als Substituenten Niederalkyl, Niederalkoxy, Hydroxyniederalkyl, Amino, Niederalkanoylamino, Niederalkylsulfonylamino, Nitro, Phenylniederalkoxy, Ureido, Halogenniederalkyl, Halogen und Hydroxy genannt werden, und m für die Zahl 0 steht mit der Massgabe, dass ein durch ein oder zwei Hydroxygruppen substituierter Phenylrest Ar mindestens einen zusätzlichen von diesen verschiedenen Substituenten enthält, n die Zahl 0 oder 1 und alk einen Alkylenrest mit 2 bis 5 Kohlenstoffatomen bedeuten, wobei das Stickstoffstom und das Sauerstoffatom, oder, falls n für Null steht, der Phenylrest, durch mindestens zwei Kohlenstoffatome voneinander getrennt sind, und $R_1$ und $R_2$ unabhängig von-

einander Niederalkyl mit bis zu 4 Kohlenstoffatomen oder $R_1$ und $R_2$ zusammen Niederalkylen mit 4-6 Kohlenstoffatomen in der unverzweigten Kette, insbesondere aber Wasserstoff, bedeuten, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft insbesondere Verbindungen der Formel I, worin Ar durch Niederalkylsulfonylamino wie Methylsulfonylamino, gegebenenfalls ausserdem noch durch Halogen, wie Chlor, substituiert ist, m für die Zahl 0 steht und n die Zahl 0 oder 1 und alk einen Alkylenrest mit 2 bis 3 Kohlenstoffatomen bedeuten, wobei das Stickstoffatom und das Sauerstoffatom, oder, falls n für Null steht, der Phenylrest durch mindestens zwei Kohlenstoffatome voneinander getrennt sind, und $R_1$ und $R_2$ jeweils Wasserstoff bedeuten, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft vor allem Verbindungen der Formel I, worin Ar durch Niederalkylsulfonylamino, wie Methylsulfonylamino, gegebenenfalls ausserdem noch durch Halogen, wie Chlor oder durch Sulfamoyl, Halogen, wie Chlor, oder Niederalkoxy, wie Methoxy, substituiert ist, m für die Zahl 0 steht, und n die Zahl 0 oder 1 und alk einen Alkylenrest mit 2 bis 3 Kohlenstoffatomen bedeuten, wobei das Stickstoffatom und das Sauerstoffatom, oder falls n für Null steht, der Phenylrest, durch mindestens zwei Kohlenstoffatome voneinander getrennt sind, und $R_1$ und $R_2$ jeweils Wasserstoff bedeuten, in der Form von Racematgemischen, Racematen, optischen Antipoden, als freie Verbindungen oder deren Salze, insbesondere Säureadditionssalze, von allem pharmazeutisch annehmbare, nicht-toxische Säureadditionssalze.

Die Erfindung betrifft insbesondere Verbindungen der Formel I, worin Ar durch Sulfamoyl und Halogen, wie Chlor, oder Niederalkoxy, wie Methoxy substituiert ist, m für die Zahl 0 steht und n die Zahl 1 und alk einen Alkylenrest mit 2 bis 3 Kohlenstoffatomen bedeuten, wobei das Stickstoffatom und das Sauerstoffatom durch mindestens zwei Kohlenstoffatome voneinander getrennt sind, und $R_1$ und $R_2$ jeweils Wasserstoff bedeuten, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die erfindungsgemässen neuen Derivate des 2-Amino-äthanols entsprechen der oben erläuterten Formel I, in welcher Ar gegebenenfalls substituiertes Phenyl, m eine Zahl von 1 bis 3, n die Zahl 0 oder 1 und alk Alkylen mit 2-5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind, und $R_1$ und $R_2$ unabhängig voneinander je Wasserstoff oder Niederalkyl, oder zusammen Niederalkylen, Oxaniederalkylen, Thianiederalkylen, Azaniederalkylen oder N-Niederalkylazaniederalkylen darstellt, in der Form von Racematgemischen, Racematen, optischen Antipoden oder deren Salzen, insbesondere Säureadditonssalzen, und vor allem pharmazeutisch annehmbaren, nicht-toxischen Säureadditionssalzen.

Die Erfindung betrifft insbesondere Verbindungen der Formel I, worin Ar gegebenenfalls ein- oder mehrfach, vorzugsweise höchstens dreifach, substituiert ist, wobei als Substituenten gegebenenfalls, insbesondere in untenstehend angegebener Weise, substituiertes Niederalkyl, Niederalkenyl oder Niederalkoxy, weiter Niederalkenyloxy, Niederalkinyl, Niederalkinyloxy, Niederalkylidendioxy, Niederalkylendioxy, Cyan und/oder Nitro, und/oder direkt oder an

vorgenanntes Niederalkyl, Niederalkenyl oder Niederalkoxy gebundenes Niederalkanoyl, verstertes oder amidiertes Carboxyl, insbesondere Niederalkoxycarbonyl, Carbamoyl, Niederalkylcarbamoyl oder (Hydroxyniederalkyl)-carbamoyl, Niederalkylsulfinyl, Niederalkylsulfonyl, Sulfamoyl oder Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Acylamino, wie Niederalkanoylamino oder Niederalkoxycarbonylamino, gegebenenfalls durch Niederalkyl, Hydroxyniederalkyl oder Cycloalkyl substituiertes Ureido, Niederalkylsulfonylamino, Hydroxy, Phenylniederalkoxy, z.B. Benzyloxy, Niederalkanoyloxy oder, als nicht direkt gebundener Substituent, wiederum Niederalkoxy vorliegen können, m für eine Zahl von 1 bis 3 steht, n für die Zahl 0 oder 1 steht und alk einen Alkylenrest mit 2 bis 4 Kohlenstoffatomen bedeutet, wobei das Stickstoffatom und das Sauerstoffatom oder, falls n für 0 steht, der Phenylrest, durch 2 bis 3 Kohlenstoffatome der Reste alk voneinander getrennt sind, $R_1$ und $R_2$ die unter Formel I angegebene Bedeutung haben, jedoch vorzugsweise für Wasserstoff oder Niederalkyl, z.B. Methyl oder Aethyl, stehen oder zusammen mit dem Stickstoffatom der Amidgruppe, Morpholino oder Alkylenamino mit 5 bis 6 Ringgliedern, wie 1-Pyrrolidinyl oder Piperidino bilden, in der Form von Racematgemischen, Racematen, optischen Antipoden, als freie Verbindungen oder deren Salze, insbesondere Säureadditionssalze, vor allem pharmazeutisch annehmbare, nicht-toxische Säureaddtionssalze.

Die Erfindung betrifft vorzugsweise Verbindungen der Formel I, worin Ar gegebenenfalls ein- bis dreifach substituiert ist, wobei als Substituenten gegebenenfalls in untenstehend angegebener Weise

substituiertes Niederalkyl, Niederalkenyl oder Niederalkoxy, weiter Niederalkenyloxy, Niederalkinyl, Niederalkinyloxy, Niederalkylidendioxy, Cyan und/oder Nitro und/oder direkt oder an vorgenanntes Niederalkyl,Niederalkenyl oder Niederalkoxy gebundenes Niederalkanoyl, Niederalkoxycarbonyl, Carbamoyl, Niederalkylcarbamoyl, (Hydroxyniederalkyl)—carbamoyl, Niederalkylsulfinyl, Niederalkylsulfonyl, Sulfamoyl, Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Niederalkanoylamino, Niederalkoxycarbonylamino, gegebenenfalls durch Niederalkyl, Hydroxyniederalkyl oder Cycloalkyl substituiertes Ureido, Niederalkylsulfonylamino, Phenylniederalkoxy, z.B. Benzyloxy, Niederalkanoyloxy oder, als nicht direkt gebundener Substituent wiederum Niederalkoxy vorliegen können, m für eine Zahl von 1 bis 3 steht und n die Zahl 0 oder 1, und alk einen Alkylenrest mit 2 bis 4 Kohlenstoffatomen bedeutet, wobei das Stickstoffatom und das Sauerstoffatom, oder, falls n für 0 steht, der Phenylrest, durch 2 bis 3 Kohlenstoffatome des Restes alk voneinander getrennt sind, $R_1$ und $R_2$ die unter Formel I angegebene Bedeutung haben, jedoch vorzugsweise für Wasserstoff oder Niederalkyl, insbesondere Methyl oder Aethyl, stehen oder zusammen mit dem Stickstoffatom der Aminogruppe 1-Pyrrolidinyl, Piperidino oder Mopholino bilden, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft besonders Verbindungen der Formel I, worin Ar gegebenenfalls ein- bis dreifach substituiert ist, wobei

als Substituenten gegebenenfalls in untenstehend angegebener Weise substituiertes Niederalkyl, Niederalkenyl oder Niederalkoxy, weiter Niederalkenyloxy, Niederalkinyloxy, Niederalkylidendioxy, Nitro und/oder Cyan und/oder direkt oder an vorgenanntes Niederalkyl, Niederalkenyl oder Niederalkoxy gebundenes Niederalkanoyl, Niederalkoxycarbonyl, Carbamoyl, Niederalkylcarbamoyl, (Hydroxyniederalkyl)-carbamoyl, Niederalkylsulfinyl, Niederalkylsulfonyl, Sulfamoyl oder Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Niederalkanoylamino, Niederalkoxycarbonylamino, gegebenenfalls durch Niederalkyl oder Cycloalkyl substituiertes Ureido, Niederalkylsulfonylamino, Hydroxy, Phenylniederalkoxy, z.B. Benzyloxy, Niederalkanoyloxy oder, als nicht direkt gebundener Substituent, wiederum Niederalkoxy vorliegen können, m für eine Zahl von 1 bis 3 steht, n die Zahl 0 oder 1 und alk einen Alkylenrest mit 2 bis 4 Kohlenstoffatomen bedeutet, wobei das Stickstoffatom und das Sauerstoffatom, oder, falls n für 0 steht, der Phenylrest, durch 2 bis 3 Kohlenstoffatome des Restes alk voneinander getrennt sind, und $R_1$ und $R_2$ unabhängig voneinander je Wassserstoff oder Niederalkyl, insbesondere Methyl oder Aethyl darstellen, oder zusammen mit dem Stickstoffatom oder Amidgruppe Morpholino bilden, in der Form von Racematgemischen, Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft vor allem Verbindungen der Formel I, worin Ar gegebenenfalls ein- bis dreifach substituiert ist, wobei als Substituenten gegebenenfalls in untenstehend angegebener Weise substituiertes Niederalkyl oder Niederalkoxy, weiter Niederalkenyl, Niederalkenyloxy, Niederalkinyloxy, Niederalkylidendioxy, Nitro und/oder Cyan und/oder direkt oder an vorgenanntes Niederalkyl oder

Niederalkoxy gebundenes Niederalkanoyl, Niederalkoxycarbonyl, Carbamoyl, Niederalkalkylcarbamoyl oder (Hydroxyniederalkyl)-carbamoyl, Niederalkylsulfinyl, Niederalkylsulfonyl, Sulfamoyl und/oder direkt oder an vorgenanntes Niederalkyl oder in höherer als der 1-Stellung an vorgenanntes Niederalkoxy gebundenes Halogen, Niederalkylthio, Amino, Alkylen- oder Oxaalkylenamino, z.B. 1-Pyrrolidinyl, Piperidino oder Morpholino, Pyrrol-1-yl, Niederalkanoylamino oder Niederalkoxycarbonylamino, oder gegebenenfalls durch Niederalkyl substituiertes Ureido, Niederalkylsulfonylamino, Hydroxy, weiter Phenylniederalkoxy, z.B. Benzyloxy, oder, als nicht direkt gebundener Substituent, wiederum Niederalkoxy vorliegen können, m für eine Zahl von 1 bis 3 steht, n die Zahl 0 oder 1 und alk einen Alkylenrest mit 2 bis 4 Kohlenstoffatomen bedeutet, wobei das Stickstoffatom und das Sauerstoffatom oder, falls n für 0 steht, der Phenylrest, durch 2 bis 3 Kohlenstoffatome des Restes alk voneinander getrennt sind, und $R_1$ und $R_2$ unabhängig voneinander je Wasserstoff oder Niederalkyl, jedoch vorzugsweise Wasserstoff oder Methyl darstellen, in der Form von Racematgemischen,Racematen, optischen Antipoden, als freie Verbindungen oder deren Salze, insbesondere Säureadditionssalze, vor allem pharmazeutisch annehmbare, nicht-toxische Säureadditionssalze.

Die Erfindung betrifft in erster Linie Verbindungen der Formel I, worin Ar gegebenenfalls ein- bis dreifach substituiert ist, wobei als Substituenten gegebenenfalls in untenstehend angegebener Weise substituiertes Niederalkyl, z.B. Methyl, oder Niederalkoxy, z.B. Methoxy oder Aethoxy, weiter Niederalkenyl, z.B. Allyl, Niederalkenyloxy, z.B. Allyloxy, Niederalkinyloxy, z.B. Propargyloxy, Nitro, Niederalkylidendioxy, z.B. Methylendioxy, und/oder Cyan und/oder direkt oder an vorgenanntes Niederalkyl oder Niederalkoxy gebundenes Niederalkanoyl, z.B. Acetyl, Carbamoyl, Niederalkylcarbamoyl, z.B. N-Methylcarbamoyl, oder (Hydroxyniederalkyl)-carbamoyl, z.B. N-Hydroxymethyl-carbamoyl, Niederalkylsulfonyl, z.B. Methylsulfonyl, Sulfamoyl, und/oder direkt oder an vorgenanntes Niederalkyl, z.B. Methyl, gebundenes Fluor oder Chlor, z.B. Trifluormethyl, Hydroxy,

Die neuen Verbindungen der Formel I besitzen wertvolle pharmakologische Eigenschaften. Insbesondere wirken sie in spezifischer Weise auf β-adrenerge Rezeptoren. Dieser Wirkung liegt als gemeinsame Eigenschaft der Verbindungen der Formel I die Affinität zu diesen Rezeptoren zugrunde, die sich bei fehlender oder sehr geringer stimulierender Eigenwirkung als reine Blockade, bei geringer bis mittelstarker stimulierender Eigenwirkung als Blockade mit gleichzeitiger ISA, d.h. intrinsic sympathomimetic activity, und bei stärkerer Eigenwirkung als überwiegende Stimulierung der β-adrenergen Rezeptoren äussert. Die Grenzen zwischen β-Rezeptoren-Blockern ohne oder mit höchstens mittelstarker ISA sind fliessend, ebenso die therapeutischen Anwendungsbereiche dieser Verbindungstypen. Von den Verbindungen der Formel I, die als β-Rezeptoren-Blocker ohne oder mit ISA wirken, etwa solche, die im Phenylkern durch eine Niederalkylsulfonylaminogruppe substituiert sind, zeigt z.B. der α-[N-[2-(4-Carbamoyl-3-hydroxyphenoxy)-1-methyläthyl]-aminomethyl]-4-(methylsulfonylamino)-benzylalkohol, insbesondere dessen eines Enantiomerenpaar vom Smp. 165-167° als Hauptwirkung eine blockierende Wirkung auf β-Rezeptoren (mit leicht bevorzugter Hemmung der Cardialen Rezeptoren), zu der als zusätzliche Wirksamkeiten schwache blutdrucksenkende und α-Rezeptoren-blockierende Wirkungen hinzukommen. Verbindungen dieser Art, wie z.B. der genannte α-[N-[2-(4-Carbamoyl-3-hydroxyphenoxy)-1-methyläthyl]-aminomethyl]-4-(methylsulfonylamino)-benzylkohol als auch dessen beide Entantiomerenpaare vom Smp. 165-167° bzw. 144-145°, oder der α-[N-[2-(4-Carbamoyl-3-hydroxyphenoxy)-äthyl]-aminomethyl]-4-(methylsulfonylamino)-benzylalkohol oder der α-[N-[2-(3-Carbamoyl-4-hydroxyphenoxy)-äthyl]-aminomethyl]-4-(methylsulfonylamino)-benzylalkohol zeigen ausserdem eine deutliche diuretische und saluretische Wirkung, wie z.B. nach p.o. Verabreichung von 1 oder 5 mg/kg solcher Verbindungen am Hund gezeigt werden kann.

Bei anderen Verbindungen der Formel I lassen sich deutliche β-Rezeptoren-blockierende als auch β-Rezeptoren-stimulierende Wirkungen feststellen. Diese Verbindungen erweisen sich bezüglich ihrer Wirkung an cardialen β-Rezeptoren einerseits bei in vitro-Versuchen am Meerschweinchenherzen als potente Blocker mit relativ deutlicher ISA, andererseits bei in vivo-Versuchen an der narkotisierten Katze überwiegend als stark wirkende Stimulatoren. Bezüglich der Wirkung an vaskulären β-Rezeptoren erweisen sie sich überwiegend als Blocker mit mittelstarker ISA, während Verbindungen der Formel I, die im Phenylkern Ar durch Niederalkyl, z.B. Methyl, substituiert sind, z.B. der α-[N-[2-(3-Carbamoyl-4-hydroxyphenoxy)-1-methyläthyl]-aminomethyl]-4-methylbenzylalkohol an trachealen β-Rezeptoren sich als starke Stimulatroen erweisen.

Zu dieser letzteren Gruppe gehören z.B. auch solche Verbindungen, in denen Ar einen verätherten Hydroxyphenylrest darstellt, wie z.B. der α-[N-[2-(3-Carbamoyl-4-hydroxy-phenoxy)-äthyl]-aminomethyl]-3,4-methylendioxy-benzylalkohol, womit sich bei in vivo Versuchen an der Katze eine cardioselektive β-Stimulation zeigen lässt. Als zusätzliche Wirkungen lassen sich z.B. beim α-[N-[2-(3-Carbamoyl-4-hydroxyphenoxy)-1-methyläthyl]-aminomethyl]-3,4-methylendioxy-benzylalkohol eine Blutdrucksenkung als auch eine schwache Blockade von adrenergen α-Rezeptoren feststellen.

Die vorstehenden Angaben betreffend pharmakologische Eigenschaften beruhen auf den Resultaten von entsprechenden pharmakologischen Versuchen in üblichen Testverfahren. So zeigen die neuen β-blockierenden Verbindungen eine Hemmung der durch Isoproterenol induzierten Tachykardie am isolierten Meerschweinchenherzen in einem Konzentrationsbereich von etwa 0,001 bis etwa 1 μg/ml und an der narkotisierten Katze in einem Dosenbereich von etwa 0,001 mg/kg bis etwa 1 mg/kg bei intravenöser Verabreichung. Die Hemmung der durch Isoproterenol induzierten Vasodilatation an der narkotisierten Katze mit Perfusion der Arteria femoralis ist bei intravenöser Verabreichung in einem Dosenbereich von etwa 0,003 mg/kg bis etwa 3 mg/kg nachweisbar. Die ISA der β-blockierenden Verbindungen der Formel I ergibt sich, sofern eine solche verbunden ist, aus der Zunahme der basalen Herzfrequenz an

der narkotisierten, mit Reserpin vorbehandelten Katze bei intravenöser Verabreichung in einem Dosenbereich von 0,001 bis 1 mg/kg. Die neuen β-blockierenden Verbindungen bewirken ebenfalls in einem Dosenbereich von etwa 0,1 mg/kg bis etwa 10 mg/kg i.v. eine Senkung des arteriellen Blutdrucks bei der narkotisierten Katze. Die zusätzliche α-blockierende Wirksamkeit, welche z.B. eine blutdrucksenkende Wirkung begünstigen kann, geht z.B. aus der Antagonisierung der durch Noradrenalin induzierten Kontraktion des isolierten Vas deferens der Ratte durch solche Verbindungen in einer Konzentration von 0,1 µg/ml bis etwa 10 µg/ml hervor. Die neuen β-blockierend wirksamen Verbindungen der Formel I können als, gegebenenfalls cardioselektive, β-Rezeptoren-Blocker, z.B. zur Behandlung von Angina pectoris und Herzrythmusstörungen, sowie als blutdrucksenkende Mittel verwendet werden. Die bei bestimmten Typen von Verbindungen der Formel I, etwa wie oben gezeigt, vorhandene zusätzliche diuretische und saluretische Wirkung könnte zu einer wesentlichen Verstärkung des antihypertensiven Effektes führen und die Kombination von β-Rezeptoren-Blockern mit Diuretika entbehrlich machen.

Die deutlich β-Rezeptoren-stimulierenden Verbindungen der Formel I, wie der α-[N-[2-(3-Carbamoyl-4-hydroxy-phenoxy)-äthyl]-aminomethyl]-3,4-methylendioxy-benzylalkohol, bewirken eine Zunahme von Herzfrequenz und myocardialer Kontraktionskraft am isolierten Meerschweinchenvorhof in einem Konzentrationsbereich von etwa 0,0001 µg/ml bis 0,1 µg/ml und eine Zunahme von Herzfrequenz und maximaler Druckanstiegsgeschwindigkeit im linken Ventrikel (dp/dt max.) an der narkotisierten Katze in einem Dosenbereich von etwa 0,001 mg/kg bis etwa 1 mg/kg i.v.. Die neuen Verbindungen stimulieren zum Teil selektiv die cardialen β-Rezeptoren ($\beta_1$-Rezeptoren) im Vergleich zu den β-Rezeptoren in den Blutgefässen ($\beta_2$-Rezeptoren) und unterscheiden sich dadurch qualitativ deutlich von Isoproterenol, welches die β-Rezeptoren des Herzens und der Blutgefässe etwa gleich stark stimuliert. Zum Teil besitzen die Verbindungen der Formel I auch relativ deutliche stimulierende Wirkungen an $\beta_2$-Rezeptoren. (Blutgefässe, Trachea).

Die neuen stimulierend wirksamen Verbindungen der Formel I können demnach

1) als Stimulatoren von cardialen β-Rezeptoren zur Behandlung der insuffizienten Herzleistung und von Herzrhythmusstörungen,
2) als Stimulatoren von trachealen und vaskulären β-Rezeptoren als Broncho- und Vasodilatatoren zur Behandlung von Asthma, Herzinsuffizienz und Durchblutungsstörungen verwendet werden.

Die neuen Verbindungen der Formel I werden in an sich bekannter Weise hergestellt. Man kann sie z.B. erhalten, indem man eine Verbindung der Formel

$$\mathrm{Ar-(CH_2)_m-\overset{X_1}{\overset{|}{CH}}-CH_2-Z_1} \qquad (II)$$

mit einer Verbindung der Formel

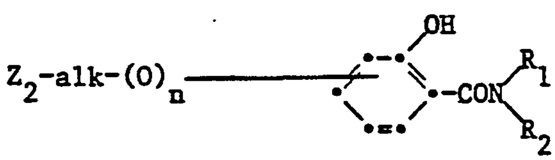

(III)

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, Ar, alk, m und n obige Bedeutung haben, umsetzt, und, wenn erwünscht, eine erhaltene Verbindung in eine andere Verbindung der Formel I umwandelt, und/oder, wenn erwünscht, eine erhaltene freie Verbindung in ein Salz oder ein erhaltenes Salz in eine freie Verbindung überführt, und/oder, wenn erwünscht, ein erhaltenes Racematgemisch in die Racemate oder ein erhaltens Racemat in die optischen Antipoden auftrennt.

Eine reaktionsfähige veresterte Hydroxygruppe $Z_1$ bzw. $Z_2$ ist eine, durch eine starke Säure, insbesondere eine starke anorganische Säure, wie eine Halogenwasserstoffsäure, insbesondere

Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylbenzolsulfonsäure oder 4-Brombenzolsulfonsäure, veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z.B. eines Alkalimetall- oder Erdalkalimetallcarbonats oder -hydroxids, oder eines organischen basischen Mittels, wie eines Alkalimetall-niederalkanolats, und/oder eines Ueberschusses des basischen Reaktionsteilnehmers und üblicherweise in Gegenwart, aber gegebenenfalls auch in Abwesenheit eines Lösungsmittels oder Lösungsmittelgemisches und, wenn notwendig, unter Kühlen oder Erwärmen, z.B. in einem Temperaturbereich von etwa -20°C bis etwa +150°C, in einem offenen oder geschlossenen Gefäss und/oder in einer Inertgasatmosphäre, z.B. in einer Stickstoffatmosphäre, arbeitet.

Ausgangsstoffe der Formeln II oder III sind bekannt oder können in an sich bekannter Weise hergestellt werden. So kann man zur Herstellung eines Ausgangsstoffs der Formel II, worin m für 0 steht, eine Verbindung der Formel Ar-H, worin gegebenenfalls vorhandene Amino- oder Hydroxygruppen durch eine Schutzgruppe, z.B. eine der nachfolgend beschriebenen, geschützt sein können, mit einem Halogenacetylhalogenid, z.B. Chloracetylchlorid, in Gegenwart einer geeigneten Lewissäure, z.B. Aluminiumchlorid, nach der Friedel-Crafts-Methode an einem Kohlenstoffatom des Restes Ar halogenacetylieren und in der so erhältlichen Ar-Halogenacetyl-Verbindung, z.B. durch

Behandeln mit einem geeigneten Hydridreduktionsmittel, die Carbonyl- zur Carbinolgruppe reduzieren; wenn erwünscht, kann man ein Halogen $Z_1$ z.B. durch Behandeln mit Ammoniak, oder einem geeigneten Derivat davon, wie Hexamethylentetramin, und Zersetzung der erhaltenen Verbindung mit verdünnter Mineralsäure, oder durch Umsetzen mit einem Alkalimetallsalz des Phthalimids und Spalten der erhaltenen N-Phthalimid-Verbindung, z.B. mit Hydrazin, in die primäre Aminogruppe $Z_1$ umwandeln. Ausgangsstoffe der Formel II, worin $X_1$ und $Z_1$ zusammen Epoxy bedeuten, können z.B. durch Cyclisierung einer Verbindung der Formel II, worin $X_1$ Hydroxy und $Z_1$ eine reaktionsfähige veresterte Hydroxygruppe, etwa Chlor oder Methansulfonyloxy darstellen, mittels alkalischer Reagentien, z.B. eines Gemisches von verdünnter Natronlauge und Tetrabutylammoniumchlorid in einem geeigneten Lösungsmittel, z.B. Methylenchlorid, erhalten werden.

Ausgangsstoffe der Formel II, worin m obige Bedeutung hat und $X_1$ und $Z_1$ zusammen die Epoxygruppe bedeuten, und gegebenenfalls vorhandene Amino- oder Hydroxygruppen durch eine Schutzgruppe, z.B. wie nachfolgend angegeben geschützt sind, können erhalten werden, indem man in einer Verbindung der Formel $Ar-(CH_2)_m-CH=CH_2$ (IIa), die Gruppe $-CH=CH_2$ durch Einführen der Epoxygruppe in die Gruppe $-CH\overset{O}{-}CH_2$ umwandelt, z.B. durch Umsetzen mit einer Peroxy-Verbindung, wie Wasserstoffperoxid, oder einer organischen Persäure, wie z.B. einer gegebenenfalls substituierten, wie halogenierten, aliphatischen oder aromatischen Persäure, z.B. Peressigsäure oder Trifluorperessigsäure, Perbenzoesäure oder m-Chlorperbenzoesäure, in einem wasserfreiem Lösungsmittel, wie Chloroform. Diese Umsetzung wird in üblicher Weise vorgenommen.

Ausgangsstoffe der Formel III können z.B. durch Umsetzung eines Hydroxysalicylamids mit einem der Bedeutung von alk entsprechenden Dihalogenalkan, etwa einem Chlor-brom- oder Dibromalkan in Gegenwart eines alkalischen Kondensationsmittels, wie einem Alkalicarbonat, und, wenn erwünscht, Ersatz des verbleibenden Halogen $Z_2$ in der oben für $Z_1$ angegebenen Weise durch die primäre Aminogruppe, erhalten werden. Diese Umsetzungen werden in üblicher Weise vorgenommen, wobei an den Amino- bzw. Hydroxygruppen stehende Schutzgruppen gleichzeitig oder nachfolgend, z.B. wie beschrieben, abgespalten werden.

Die Verbindungen der Formel I können weiterhin hergestellt werden, indem man in einer Verbindung der Formel

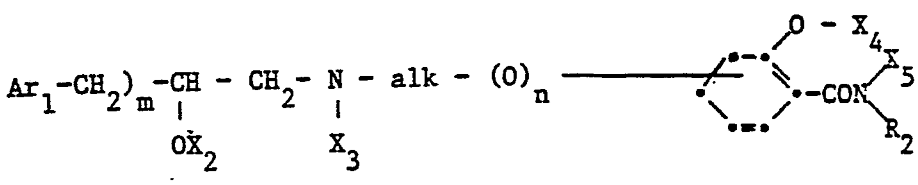 (IV),

worin $Ar_1$ die Bedeutung von Ar hat oder einen Rest Ar bedeutet, der durch jeweils 1 bis 2 in Hydroxy und/oder Amino überführbare Gruppen substituiert ist, $X_2$, $X_3$ und $X_4$ jeweils Wasserstoff oder einen durch Wasserstoff ersetzbaren Substituenten bedeuten und $X_5$ für $R_1$ steht, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$ und $X_4$ von Wasserstoff verschieden ist, oder mindestens $Ar_1$ einen Rest Ar bedeutet, der durch jeweils 1 bis 2 in Hydroxy und/oder Amino überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen, und m und n obige Bedeutungen

haben, oder in einem Salz davon, das von Wasserstoff verschiedene $X_2$, $X_3$, oder $X_4$, bzw. $X_2$ und $X_3$ zusammen oder $X_4$ und $X_5$ zusammen durch Wasserstoff ersetzt, und/oder in einem Rest $Ar_1$ vorhandenes substituiertes Hydroxy und/oder Amino in freies Hydroxy und/oder Amino überführt, und wenn erwünscht, die anschliessend an das erste Verfahren genannten zusätzlichen Verfahrensschritte durchführt.

Die Abspaltung der Gruppen $X_2$, $X_3$ oder $X_4$ oder jeweils $X_2$ und $X_3$ oder $X_4$ und $X_5$ zusammen sowie der gegebenenfalls in einem Rest $Ar_1$ vorhandenen Schutzgruppen von Hydroxy und/oder Amino-Substituenten wird mittels Solvolyse, wie Hydrolyse, Alkoholyse, Aminolyse oder Acidolyse, oder mittels Reduktion einschliesslich Hydrogenolyse vorgenommen.

Besonders geeignete, abspaltbare Gruppen $X_3$ und $X_4$ oder Hydroxy- und/oder Amino-Schutzgruppen in einem Rest $Ar_1$ sind in erster Linie hydrogenolytisch abspaltbare α-Arylniederalkylgruppen, wie eine gegebenenfalls substituierte 1-Polyphenyl- oder 1-Phenylniederalkylgruppen, worin Substituenten, insbesondere des Phenylteils, z.B. Niederalkyl, wie Methyl, oder Niederalkoxy, wie Methoxy sein können, und in erster Linie Benzyl. Gruppen $X_3$ und insbesondere $X_2$ und $X_4$ sowie Hydroxy- und/oder Amino-Schutzgruppen in einem Rest $Ar_1$ können auch solvolytisch, wie hydrolytisch oder acidolytisch, ferner reduktiv, einschliesslich hydrogenolytisch abspaltbare Reste, insbesondere entsprechende Acylreste wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner gegebenenfalls substituierte 1-Polyphenylniederalkylgruppen, worin Substituenten, in erster Linie des Phenylteils, z.B. die oben gegebene Bedeutung haben, und in erster Linie Trityl darstellen.

Ein durch $X_2$ und $X_3$ und/oder $X_4$ und $X_5$ zusammen gebildeter, abspaltbarer Rest ist in erster Linie eine hydrogenolytisch abspaltbare zweiwertige Gruppe, wie gegebenenfalls substituiertes 1-Phenylniederalkyliden, worin Substituenten, insbesondere des Phenylteils, z.B. Niederalkyl oder Niederalkoxy sein können, und insbesondere Benzyliden, sowie solvolytisch, insbesondere hydrolytisch abspaltbare Gruppen, wie Niederalkyliden, z.B. Methylen oder Isopropyliden, oder 1-Phenyl-niederalkyliden, dessen Phenylteil gegebenenfalls durch Niederalkyl, wie Methyl, oder Niederalkoxy, wie Methoxy, substituiert ist, insbesondere Benzyliden, oder Cycloalkyliden, z.B. Cyclopentyliden oder Cyclohexyliden.

In der Form von Salzen verwendbare Ausgangsstoffe werden in erster Linie in der Form von Säureadditionssalzen, z.B. mit Mineralsäuren, sowie mit organischen Säuren verwendet.

Hydrogenolytisch abspaltbare Reste $X_2$, $X_3$ und/oder $X_4$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_2$ und $X_3$ sowie $X_4$ und $X_5$ zusammen gebildete, gegebenenfalls substituierte 1-Phenylniederalkylidengruppen, sowie in einem Rest $Ar_1$ vorhandene Hydroxy- und/oder Amino-Schutzgruppen dieser Art können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators abgespalten werden.

Hydrolytisch abspaltbare Gruppen $X_2$, $X_3$ und/oder $X_4$, wie Acylreste von organischen Carbonsäuren, z.B. Niederalkanoyl, und von Halbestern der Kohlensäure, z.B. Niederalkoxycarbonyl, ferner z.B. Tritylreste, sowie durch die Reste $X_2$ und $X_3$ und/oder $X_4$ und $X_5$

zusammen gebildete Niederalkyliden-, 1-Phenyl-niederalkyliden- oder Cycloalkylidengruppen sowie in einem Rest $Ar_1$ vorhandene Hydroxy- und/oder Amino-Schutzgruppen dieser Art können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins wie Isopropylamin, abgespalten werden.

Durch Aminolyse können beispielsweise als Schutzgruppen von Hydroxygruppen vorliegende Acylreste und insbesondere durch $X_4$ und $X_5$ zusammen gebildete Niederalkyliden-, 1-Phenylniederalkyliden- oder Cycloalkylidengruppen wie z.B. die 1-Methyläthylidengruppe abgespalten werden, beispielsweise durch Umsetzen mit Ammoniak oder, besonders im Falle der vorgenannten zweiwertigen Schutzgruppen, mit primären Aminen, wie z.B. Isopropylamin oder Benzylamin, in einem geeigneten Reaktionsmedium, wie z.B. Isopropanol oder Dioxan. Als primäres Amin kann auch ein Ausgangsstoff der Formel II für das erstgenannte Herstellungsverfahren für die Verbindungen der Formel I fungieren. Deshalb erhält man bei der Umsetzung von Verbindungen der weiter unten angegebenen Formel IVe, in denen $X_4$ und $X_5$ zusammen eine der vorgenannten zweiwertigen Schutzgruppen, wie z.B. Isopropyliden bedeuten, mit der doppeltmolaren Menge von entsprechenden Ausgangsstoffen der Formel II, die ihrerseits auch unter die Formel IVa fallen, unter Abspaltung der aus $X_4$ und $X_5$ gebildeten Schutzgruppe direkt Endstoffe der Formel I, während die genannte Schutzgruppe bestehen bleibt und der entsprechende Ausgangsstoff der Formel IV entsteht, wenn man als halogenwasserstoffbindende Mittel anstelle eines Ueberschusses an der Verbindung der Formel II bzw. IVa z.B. gewisse anorganische Basen, wie Alkalimetallcarbonate, verwendet.

Acidolytisch abspaltbare Reste $X_2$, $X_3$ und/oder $X_4$ und/oder Hydroxy- und/oder Aminoschutzgruppen in einem Rest $Ar_1$ sind insbesondere

gewisse Acylreste von Halbestern der Kohlensäure, wie z.B. tert.-Niederalkoxycarbonyl oder gegebenenfalls substituierte Diphenylmethoxycarbonylreste, ferner auch der tert.-Butylrest; solche Reste können durch Behandeln mit geeigneten starken organischen Carbonsäuren, wie gegebenenfalls durch Halogen, insbesondere Fluor, substituierte Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_2$, $X_3$ und/oder $X_4$ und/oder Hydroxy- und/oder Amino-Schutzgruppen in einem Rest $Ar_1$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Schutzgruppen, welche an in einem Rest $Ar_1$ gegebenenfalls vorhandenen Hydroxy- und/oder Aminogruppen stehen, entsprechen den vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Die Ausgangsstoffe der Formel IV lassen sich in an sich bekannter Weise erhalten, indem man zur Herstellung eines Ausgangsstoffs, worin m für 0 steht, z.B. eine Verbindung der Formel $Ar_1$-H mit einem Halogenacetylhalogenid, z.B. Chloracetylchlorid, in Gegenwart einer geeigneten Lewissäure, z.B. Aluminiumchlorid, nach dem Friedel-Crafts-Verfahren umsetzt, die Carbonylgruppe in der so erhaltenen oder in anderer, üblicher Weise erhaltenen $Ar_1$-Halogenacetyl-Verbindung, z.B. mittels Natriumborhydrid, zur Carbinolgruppe reduziert und die erhaltene Verbindung mit einem Amin der Formel

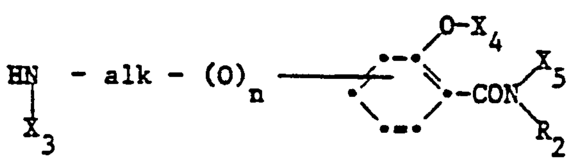

(IVa)

worin $X_3$ die angegebene Bedeutung hat, und $X_4$ oder $X_4$ und $X_5$ zusammen verschieden von Wasserstoff sind, umsetzt.

Ausgangsstoffe der Formel IV, worin m obige Bedeutung hat, sind durch Umsetzung einer Verbindung der oben erläuterten Formel II mit einer Verbindung der Formel IVa zugänglich. Diese Umsetzung wird in an sich bekannter Weise vorgenommen.

Man kann ferner z.B. die durch Umsetzung einer Verbindung der Formel

$$Ar_1-(CH_2)_m-\underset{\displaystyle OX_2}{\underset{|}{CH}} - CH_2- NH_2 \qquad \text{(IVb)}$$

mit einer Carbonylverbindung der Formel

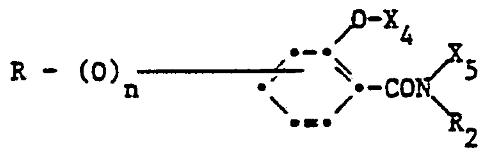 (IVc),

worin R einen dem Alkylenrest alk entsprechenden, eine vom Sauerstoffatom bzw. Phenylrest durch mindestens ein Kohlenstoffatom getrennte Carbonylgruppierung enthaltenden Alkylrest bedeutet, und $X_4$ oder $X_4$ und $X_5$ zusammen eine der angegebenen Schutzgruppen bedeuten, gebildete Schiff'sche Base mit einem Borhydrid, etwa Natriumborhydrid, zur Verbindung der Formel IV reduzieren. Die Reduktion kann auch mittels aktiviertem Wasserstoff in Gegenwart eines Hydrierkatalysators, z.B. eines Platin-auf-Kohle Katalysators erfolgen.

Amine der Formel IVb, worin $X_2$ Wasserstoff oder eine mittels Reduktion einschliesslich Hydrogenolyse abspaltbare und durch Wasserstoff ersetzbare Gruppe darstellt, können z.B. durch Umsetzung einer Verbindung der Formel

$$Ar_1-(CH_2)_m-\underset{}{\overset{X_1}{\overset{|}{CH}}}-CH_2-Z_1 \qquad (IVd),$$

worin $X_1$ und $Z_1$ die unter der Formel (II) angegebene Bedeutung haben, mit Ammoniak oder einem einen mittels Reduktion einschliesslich Hydrogenolyse, z.B. wie angegeben, abspaltbaren und durch Wasserstoff ersetzbaren Rest enthaltenden Amin, z.B. Benzylamin, hergestellt werden.

Carbonylverbindungen der Formel (IVc), worin n den Wert 1 darstellt, können durch Umsetzung einer Verbindung der Formel

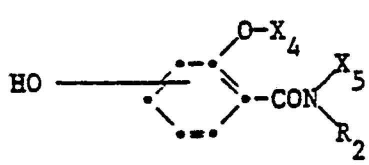 (IVe)

mit einer Verbindung der Formel R-Hal (IVf), worin R obige Bedeutung hat, und eine Verbindung (IVf) z.B. ein Halogenketon, z.B. Chloraceton darstellt, in üblicher Weise erhalten werden.

Aus den vorgenannten Verbindungen der Formel(IVe) kann man auch durch Umsetzung mit nicht-geminalen, insbesondere vicinalen Dihalogenniederalkanen, vor allem Dibrom- oder Brom-chlor-niederalkanen Verbindungen der Formel

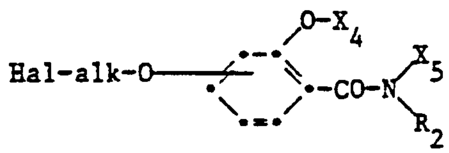

(IVg)

in der Hal ein Brom oder Chlor bedeutet und $R_2$ alk, $X_4$ und $X_5$ die oben angegebene Bedeutung haben, herstellen und diese mit Verbindungen der Formel IVb zu Ausgangsstoffen der Formel IV umsetzen, wobei, wie bereits erwähnt, gegebenenfalls direkt Endstoffe der Formel I entstehen.

Die neuen Verbindungen der Formel I können ebenfalls erhalten werden, indem man in einer Verbindung der Formel

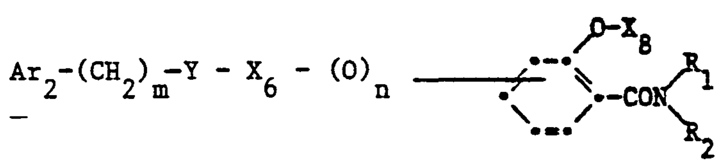

(V),

worin $X_6$ eine reduzierbare Gruppe der Formeln
$-CH=N-Alk-$ (Va), bzw. $-CH_2-N=alk_1-$ (Vb)
oder $-C(=X_7)-N(X_8)-alk-$ (Vc) bzw.
$-CH_2-N(X_8)-C(=X_7)-alk_2-$ (Vd) oder eine Gruppe
$-CH_2-N(X_8)-alk-$ (Ve) ist, wobei $alk_1$ für den einem Rest alk entsprechenden Alkyl-ylidenrest steht und $alk_2$ einem um eine an das Stickstoffatom gebundene Methylengruppe verkürzten Rest alk entspricht, $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt und Y für einen Rest der Formel -CO- (Vf) oder $-CH(OX_8)-$ (Vg) steht, in der $X_8$ die vorstehend angegebene Bedeutung hat, $Ar_2$ einem Rest Ar entspricht, jedoch gegebenenfalls

anstelle von jeweils einem oder zwei Hydroxy und/oder Amino eine oder zwei Gruppen-$OX_8$, und/oder $-N(X_8)-$, in welchen $X_8$ die vorstehend angegebene Bedeutung hat, trägt, m und n obige Bedeutungen haben, wobei stets $X_6$ eine reduzierbare Gruppe Va bis Vd und/oder Y eine Carbonylgruppe Vf ist, diese (Gruppe(n) reduziert und im gleichen Arbeitsgang die von Wasserstoff verschiedenen Gruppen $X_8$ durch Wasserstoff ersetzt, und, wenn erwünscht, die zusätzlichen, anschliessend an das erste Verfahren genannten Verfahrensschritte durchführt.

Eine hydrogenolytisch abspaltbare Gruppe $X_8$ ist in erster Linie eine α-Arylniederalkylgruppe, wie eine gegebenenfalls substituierte 1-Phenylniederalkylgruppe, worin Substituenten z.B. Niederalkoxy, wie Methoxy, sein können, und ganz besonders Benzyl.

Schutzgruppen, die an den den Rest $Ar_2$ gegebenenfalls substituierenden Hydroxy- und/oder Aminogruppen stehen, entsprechen den vorhin für $X_8$ genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Ausgangsstoffe der Formel V mit einer Gruppe $X_6$ der Formel Vb können auch in der isomeren Form von Ring-Tautomeren der Formel

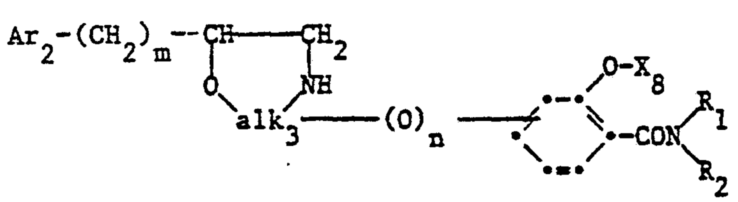

(Vh)

worin $alk_3$ der Bedeutung von $alk_1$ entspricht und das Sauerstoff- und Stickstoffatom des Ringes an das gleiche Kohlenstoffatom gebunden sind, vorliegen.

Eine Alkyl-ylidengruppe $alk_1$ ist z.B. 1-Aethyl-2-yliden oder 2-Methyl-1-äthyl-2-yliden, während eine Alkylidengruppe $alk_2$ z.B. Methylen, Aethyliden oder 1-Methyl-äthyliden darstellt.

Die Reduktion der Stickstoff-Kohlenstoff-Doppelbindung in Ausgangsstoffen der Formel V, die als $X_6$ eine Gruppe Va oder Vb enthalten, während $Ar_2$, Y, $X_8$ und n die unter der Formel V angegebene Bedeutung haben (oder in den isomeren Verbindungen der Formel Vg der Sauerstoff-Kohlenstoff-Stickstoff-Bindung) zur Stickstoff-Kohlenstoff-Einfachbindung kann in an sich bekannter Weise, z.B. durch Behandeln mit katalytisch aktiviertem Wasserstoff, wie Wasserstoff in Gegenwart eines geeigneten Hydrierkatalysators, z.B. eines Nickel-, Platin- oder Palladiumkatalysators, erfolgen, wobei hydrogenolytisch abspaltbare Gruppen $X_8$ zugleich abgespalten und durch Wasserstoff ersetzt werden; oder man arbeitet mit einem geeigneten Hydridreduktionsmittel, wie einem Alkalimetallborhydrid, z.B. Natriumborhydrid. In allen Fällen wird zugleich mit der Gruppe Va oder Vb auch, falls vorhanden, ein Carbonylrest Y zum Hydroxymethylenrest reduziert, und bei Anwendung von Hydridreduktionsmittel können auch an Sauerstoff gebundene Acylreste von Carbonsäuren, wie z.B. Essigsäure, als Reste $X_8$ vorliegen und im gleichen Arbeitsgang abgespalten werden.

Die Reduktion der Carbonylgruppe Y in Ausgangsstoffen der allgemeinen Formel V, die als Rest $X_6$ eine Gruppe Ve enthalten, während $Ar_2$, $X_8$ und n die unter der Formel V angegebene Bedeutung haben, kann in der vorstehend für die Reduktion der Gruppen Va und Vb angegebenen Weise erfolgen, wobei wiederum bei der katalytischen Hydrierung entsprechende Reste $X_8$ hydrogenolytisch abgespalten werden können.

Insbesondere geeignet zur Reduktion von Verbindungen der Formel V mit einer Gruppe der Formel Ve oder Vd und den unter der Formel V definierten Bedeutungen von $Ar_2$, Y und n sind Hydridreduktionsmittel, wie z.B. Natriumborhydrid oder Diboran.

Zugleich mit der Reduktion einer Gruppe Vc oder Vd erfolgt die Reduktion einer Carbonylgruppe Y, falls diese vorhanden ist, sowie die Abspaltung von an Sauerstoff gebundenen Acylresten von Carbonsäuren, wie z.B. Essigsäure, als Reste $X_8$. Andererseits ist durch Beschränkung der Menge Reduktionsmittel und geeignete Wahl der Reduktionsbedingungen dafür zu sorgen, dass die aromatisch gebundene Carboxamidgruppe nicht reduziert wird. Gruppierungen der Formeln Ve und Vd, worin $X_7$ jeweils eine Thioxogruppe bedeutet, werden durch reduktive Entschwefelung, z.B. durch Behandeln mit einem Hydrierkatalysator, wie Raney-Nickel, in die Gruppierung der Formel $-CH_2-NH-alk-$ umgewandelt. Die obigen Reduktionsreaktionen werden in an sich bekannter Weise, üblicherweise in Gegenwart eines inerten Lösungsmittels und, wenn notwendig, unter Kühlen oder Erwärmen, z.B. in einem Temperaturbereich von etwa -20° bis etwa +150°, und/oder in einem geschlossenen Gefäss unter Druck und/oder in einer Inertgas-, z.B. Stickstoffatmosphäre, durchführt.

Ein Ausgangsmaterial der Formel V, worin m für 0 steht, kann in an sich bekannter Weise, gegebenenfalls in situ, d.h. unter den Bedingungen des beschriebenen Verfahrens, hergestellt werden. So kann man eine Verbindung der Formel $Ar_2-H$ (Vi) mit einem Essigsäurehalogenid oder -anhydrid in Gegenwart einer Lewissäure acetylieren und in dem erhaltenen Zwischenprodukt die Acetylgruppe dann z.B. durch Behandeln mit einem geeigneten Oxidationsmittel, wie Selendioxid, in die Glyoxyloylgruppe umwandeln. Eine solche Glyoxylverbindung, oder, wenn erwünscht, ein geeignetes Derivat derselben, etwa ein Acetal, kann dann mit einem Amin der Formel

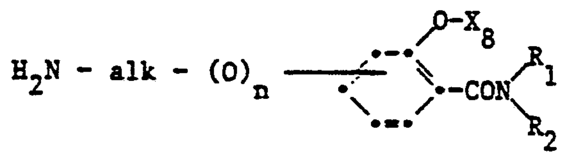

(Vj)

zu einem Ausgangsprodukt der Formel V mit der Gruppe $X_6$ der Formel Va umgesetzt werden, worin Y die Carbonylgruppe darstellt. Man kann ferner

eine Verbindung der Formel (Vi) mit einem Halogenacetylhalogenid, z.B. Chloracetylchlorid, in Gegenwart einer geeigneten Lewissäure, z.B. Aluminiumchlorid, nach der Friedel-Crafts-Methode zur entsprechenden $Ar_2$-Chloracetyl-Verbindung halogenacetylieren, in der so oder in anderer üblicher Weise erhältlichen Halogenacetylverbindung durch Behandeln mit einem geeigneten Hydridreduktionsmittel die Carbonyl- zur Carbinolgruppe reduzieren, und das Halogenatom durch Behandeln mit Ammoniak oder einem geeigneten Derivat davon, wie Hexamethylentetramin, und Zersetzen des gebildeten Reaktionsproduktes mit verdünnter Säure, etwa wässriger Salzsäure, in die primäre Aminogruppe umwandeln, wonach man ein Zwischenprodukt der Formel

$$Ar_2-(CH_2)_m-\underset{\displaystyle OH}{\underset{|}{CH}}-CH_2-NH_2 \qquad (Vk)$$

erhält, worin m für 0 steht. Amine der Formel Vk wiederum, worin m obige Bedeutung hat, sind z.B. durch Umsetzung einer Verbindung der Formel

$$Ar_2-(CH_2)_m-\overset{\displaystyle X_1}{\overset{|}{CH}}-CH_2-Z_1 \qquad (Vl)$$

worin $X_1$ und $Z_1$ die unter der Formel (II) angegebene Bedeutung haben, mit Ammoniak oder einer Ammoniak liefernden Verbindung, z.B. Hexamethylentetramin, in bekannter Weise erhältlich, wobei Verbindungen der Formel (Vl) analog dem für die Herstellung von Ausgangsstoffen der Formel (II) beschriebenen Verfahren erhalten werden können.

Amine der Formel Vk, worin m obige Bedeutung hat, können ausserdem durch Umsetzung eines Aldehyds der Formel

$$Ar-(CH_2)_m-CHO \qquad (Vm)$$

oder eines geeigneten Derivats davon, etwa eines Acetals, z.B. des Dimethylacetals, oder einer Bisulfitadditionsverbindung, mit einer geeigneten Silicium-organischen Cyanoverbindung, etwa einem Triniederalkyl-, z.B. Trimethylsilylcyanid, in üblicher Weise in Gegenwart eines Katalysators, wie Zinkjodid, zu einer Verbindung der Formel

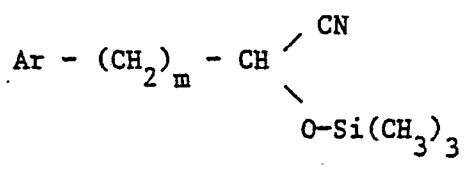 (Vn)

und deren Umwandlung mittels eines geeigneten Reduktionsmittels, z.B. eines Dileichtmetallhydrids, etwa Lithiumaluminiumhydrid, in einem Lösungsmittel, wozu üblicherweise eine ätherartige Flüssgikeit, z.B. Diäthyläther oder Tetrahydrofuran dient, in eine Verbindung der Formel Vk erhalten werden, worin m obige Bedeutung hat.

Durch Umsetzung eines Zwischenprodukts der Formel Vk mit einer Carbonylverbindung der Formel

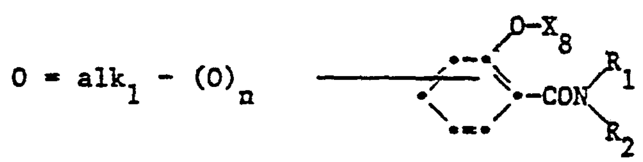 (Vo),

kann man zu Ausgangsstoffen der Formel V mit einer Gruppe $X_6$ der Formel (Vb) gelangen. Eine Modifizierung dieser Umsetzungen besteht darin, dass man in dem vorhin beschriebenen Zwischenprodukt das Halogenatom, statt es durch Behandeln mit Ammoniak etc. gegen die primäre Aminogruppe auszutauschen, durch Umsetzung mit einem 1-Aryl-niederalkylamin, z.B. Benzylamin oder einem Di-(1-aryl-niederalkyl)-amin z.B. Dibenzylamin gegen die entsprechende 1-Arylniederalkylamino bzw. Di-(1-arylniederalkyl)-aminogruppe austauscht und die erhaltene Verbindung, etwa die entsprechende Dibenzylaminoverbindung, mit der Oxoverbindung der Formel (Vl) unter den reduzieren-

den Bedingungen des Verfahrens umsetzt. Hierbei verwendet man als Reduktionsmittel in erster Linie katalytisch aktivierten Wasserstoff, z.B. Wasserstoff in Gegenwart eines Schwermetallhydrierkatalysators oder eines Gemisches davon, wie eines Palladium- und/oder Platinkatalysators. Unter solchen Reaktionsbedingungen werden hydrogenolytisch abspaltbare Gruppen $X_8$, z.B. Benzylgruppen, abgespalten, die gegebenenfalls vorhandene Carbonyl- zur Carbinolgruppe und gleichzeitig die Stickstoff-Kohlenstoff-Doppelbindung zur entsprechenden Stickstoff-Kohlenstoff-Einfachbindung reduziert.

Oxoverbindungen der Formel Vo wiederum, worin n für 1 steht, sind z.B. durch Umsetzung einer Dihydroxyverbindung der Formel

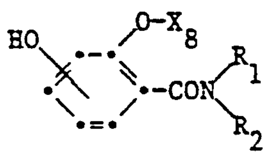

(Vp)

mit einer Halogenalkanon-Verbindung der oben erläuterten Formel R - Hal (IVf), z.B. Chloraceton, in Gegenwart eines alkalischen Kondensationsmittels, etwa Kaliumcarbonat, oder einer organischen Base, wie Triäthylamin, erhältlich.

Ausgangsprodukte der Formel V mit einer Gruppe $X_6$ der Formel Vc oder Vd können in an sich bekannter Weise hergestellt werden, indem man z.B. eine Formylverbindung der Formel $Ar_2-(CH_2)_m-CHO$ (Vq) mit Cyanwasserstoff umsetzt und im so erhältlichen Cyanhydrin-Zwischenprodukt die Cyan- zur Carboxylgruppe, z.B. unter sauren Bedingungen, hydrolysiert. Die so oder über die Zwischenstufen Imidchlorid, Imido-niederalkylester und Niederalkylester erhaltene $Ar_2$-2-hydroxyessigsäure wird dann in Gegenwart eines geeigneten Kondensationsmittels, z.B. eines Carbodiimids, wie Dicyclohexylcarbodiimid, mit einem Amin der Formel (Vj) umsetzt, wonach man ein Ausgangsmaterial der Formel V mit der Gruppe $X_6$ der Formel (Vc) erhält.

Ferner kann man durch Umsetzung einer Verbindung der Formel Vk mit einer Verbindung der Formel

$Hal - C(=O) - alk_2 - (O)_n$ 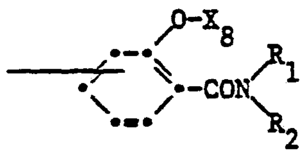 (Vr)

worin Hal für Halogen und insbesondere für Chlor steht, ein Ausgangsmaterial der Formel (V) mit der Gruppe $X_6$ der Formel (Vd) erhalten. Man kann ferner in der oben angegebenen $Ar_2$-Chloracetylverbindung das Chlor etwa durch Umsetzung mit Hexamethylentetramin gegen die primäre Aminogruppe austauschen und eine so erhaltene Verbindung mit einer Halogenverbindung der Formel Vr umsetzen. In einem so erhaltenen Ausgangsprodukt der Formel V mit einer Gruppe $X_6$ der Formel Vd und worin Y die Carbonylgruppe darstellt, kann man die Carbonyl- zur Carbinolgruppe und die aliphatisch gebundene Carbamoylgruppe zur Gruppe der Formel $-CH_2-NH-alk-$ gleichzeitig reduzieren, etwa mittels eines Hydridreduktionsmittels, insbesondere Diboran.

Die neuen Verbindungen der Formel I können ebenfalls erhalten werden, indem man eine Verbindung der Formel

$Ar_3-(CH_2)_m-CH(OH)-CH_2-NH-alk-(O)_n$ 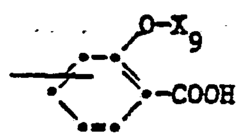 (VI)

worin $Ar_3$ die Bedeutung von Ar hat, oder einen Rest Ar darstellt, der durch 1 bis 2 mittels Aminolyse in Hydroxy und/oder Amino überführbare und/oder durch Gruppen der Formel -COOH substituiert ist, $X_9$ Wasserstoff oder eine mittels Aminolyse abspaltbare Gruppe bedeutet, und m und n obige Bedeutungen haben, oder ein reaktionsfähiges Derivat einer der in Formel VI definierten Carbonsäuren, mit einer Verbindung der Formel $HNR_1R_2$ (VII) umsetzt und gleichzeitig gegebenenfalls vorhandene, mittels Aminolyse in Hydroxy

und/oder Amino überführbare Reste in Hydroxy und/oder Amino überführt, und/oder gegebenenfalls vorhandene Reste $X_9$ abspaltet und durch Wasserstoff ersetzt, und, wenn erwünscht, die anschliessend an das erste Verfahren genannten zusätzlichen Verfahrensschritte durchführt. Hydrolytisch und insbesondere mittels Aminolyse in Hydroxy und/oder Amino überführbare Reste bzw. auf diese Weise abspaltbare Reste $X_9$ sind Acylreste von organischen Carbonsäuren, z.B. Aroyl, wie Benzoyl, oder Niederalkanoyl, wie Acetyl.

Reaktionsfähige Derivate der in Formel VI definierten Carbonsäuren sind z.B. die Halogenide, wie die Chloride oder Bromide, ferner die Azide, sowie Säureanhydride, insbesondere gemischte Säureanhydride mit z.B. Niederalkancarbonsäuren, wie Essigsäure oder Propionsäure, Niederalkoxyalkancarbonsäuren, wie 2-Methoxyessigsäure. Reaktionsfähige Derivate von Carbonsäuren der Formel VI sind insbesondere Ester, z.B. mit Niederalkanolen, wie Methanol, Aethanol, Isopropanol, tert.-Butanol, ferner mit Arylniederalkanolen, etwa gegebenenfalls durch Niederalkyl, z.B. Methyl, oder Niederalkoxy, z.B. Methoxy, substituiertem Benzylalkohol, oder Phenolen, die gegebenenfalls durch geeignete Substituenten aktiviert sind, z.B. durch Halogen, etwa 4-Halogen, wie 4-Chlor, Niederalkoxy, etwa 4-Niederalkoxy wie 4-Methoxy, 4-Nitro oder 2,4-Dinitro, wie etwa 4-Chlorphenol, 4-Methoxyphenol, 4-Nitro- oder 2,4-Dinitrophenol, ferner Ester mit Cycloalkanolen, wie etwa Cyclopentanol oder Cyclohexanol, die gegebenenfalls durch Niederalkyl, z.B. Methyl, substituiert sein können. Die Umsetzung wird in an sich bekannter Weise, üblicherweise in Gegenwart eines inerten Lösungsmittel, z.B. in einem Temperaturbereich von etwa -10° bis +50°C in einem geschlossenen Gefäss durchgeführt.

Die Ausgangsstoffe der Formel VI, worin m für 0 steht, lassen sich in an sich bekannter Weise erhalten, indem man eine Verbindung der Formel $Ar_3 - COCH_3$ bromiert, die Carbonylgruppe in der so erhaltenen

$Ar_3$-Halogenacetyl- Verbindung zur Carbinolgruppe, z.B. mittels Diboran, reduziert und die erhaltene Verbindung mit einem Amin der Formel

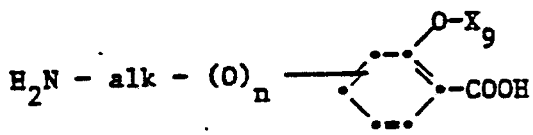

(VIa)

worin $X_9$ die angegebene Bedeutung hat, oder einem reaktionsfähigen Derivat davon, umsetzt. Man kann auch die $Ar_3$-Halogenacetyl-Verbindung mit dem Amin der Formel VIa umsetzen und nachträglich die Carbonylin die Carbinolgruppe umwandeln.

Für die Herstellung von Ausgangsstoffen der Formel VI, worin m obige Bedeutung hat, kann man ferner die durch Umsetzung einer Verbindung der Formel

$$Ar_3-(CH_2)_m-\underset{\underset{OH}{|}}{CH}-CH_2-NH_2 \qquad \text{(VIb)}$$

mit einer Carbonylverbindung der Formel

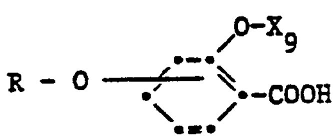

(VIc)

worin R dem einen Alkylenrest alk entsprechenden, eine Carbonylgruppe enthaltenden Alkylrest, d.h. einem Oxoalkylrest entspricht, gebildete Schiff'sche Base mit einem Borhydrid, etwa Natriumborhydrid reduzieren.

Die Reduktion kann auch mittels aktiviertem Wasserstoff in Gegenwart eines Hydrierkatalysators, z.B. eines Platin-auf-Kohle-Katalysators erfolgen.

Verbindungen der Formel VIb ihrerseits sind z.B. durch Umsetzung einer Verbindung der Formel

$$Ar_3-(CH_2)_m-\overset{\overset{X_1}{|}}{CH}-CH_2-Z_1 \qquad \text{(VId),}$$

worin $X_1$ und $Z_1$ zusammen Epoxy, oder $X_1$ Hydroxy und $Z_1$ eine reaktionsfähige veresterte Hydroxygruppe, z.B. Halogen, wie Chlor, bedeutet, mit Ammoniak, oder, falls $X_1$ Hydroxy und $Z_1$ z.B. Chlor bedeutet, mit Hexamethylentetramin und Zersetzung des gebildeten Addukts mit verdünnter Mineralsäure, wie verd. Salzsäure, erhältlich, wobei Ausgangsstoffe der Formel VId analog dem für die Herstellung von Ausgangsstoffen der Formel II beschriebenen Verfahren hergestellt werden können.

Carbonylverbindungen der Formel (VIc) wiederum, worin n 1 bedeutet, können durch Umsetzung einer Verbindung der Formel

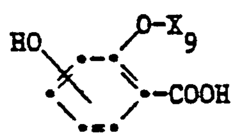 (VIe)

mit einer Verbindung der Formel R-Hal (IVf), worin Hal für Halogen, insbesondere Chlor steht, in an sich bekannter Weise erhalten werden.

Die neuen Verbindungen der Formel I, worin $R_1$ und $R_2$ jeweils Wasserstoff bedeuten, können ebenfalls erhalten werden, indem man in einer Verbindung der Formel

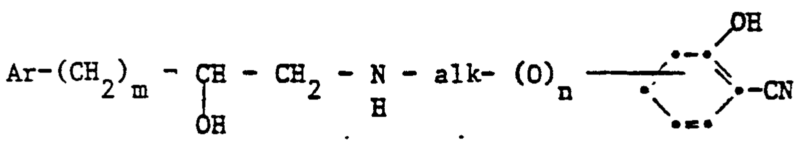 (VII),

worin eine oder beide Hydroxygruppen und/oder im Rest Ar vorhandene Hydroxy- und/oder Aminogruppen gegebenenfalls durch solche mittels Hydrolyse abspaltbaren und durch Wasserstoff ersetzbaren Gruppen geschützt sind, die unter den Bedingungen des Verfahrens abgespalten und durch Wasserstoff ersetzt werden, oder im Rest Ar gegebenenfalls zusammen mit den genannten geschützten Hydroxy- und/oder Aminogruppen die Gruppe -CN vorhanden ist, die Gruppen -CN mittels Hydrolyse in die Gruppe $-CONH_2$ und gleichzeitig gegebenenfalls geschützte Hydroxy- und/oder Aminogruppen in freie Hydroxy- und/oder Aminogruppen

umwandelt, und, wenn erwünscht, die anschliessend an das erste Verfahren genannten zusätzlichen Verfahrensschritte durchführt.

Die Ausgangsstoffe der Formel VII lassen sich in üblicher Weise herstellen, indem man eine Verbindung der Formel

$$Ar-(CH_2)_m-\underset{\displaystyle OH}{\underset{|}{CH}}-CH_2-NH_2 \qquad (VIIa)$$

mit einer Verbindung der Formel

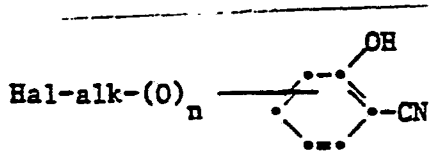

(VIIb)

worin Hal für Chlor, Brom oder Jod steht, umsetzt.Die Umsetzung wird vorteilhafterweise in Gegenwart eines basischen Mittels in an sich bekannter Weise durchgeführt.

Ausgangsstoffe der Formel (VIIa) ihrerseits, worin m obige Bedeutung hat, sind durch Umsetzung einer Verbindung der Formel (II), worin $X_1$ und $Z_1$ zusammen die Epoxygruppe bedeuten, mit Ammoniak, oder wenn $X_1$ Hydroxy und $Z_1$ als reaktionsfähige veresterte Hydroxygruppe z.B. Halogen, wie Chlor, bedeutet, z.B. mit Hexamethylentetramin und anschliessender Umwandlung der erhaltenen Addukts mit verdünnter Mineralsäure, wie verdünnter Salzsäure, erhältlich.

Ausgangstoffe der Formel (VII), worin m für 0 steht, lassen sich ferner in üblicher Weise herstellen, indem man z.B. eine Verbindung der Formel Ar-$COCH_3$ (VII c) halogeniert, z.B. bromiert, etwa mittels Brom in einem inerten Lösungsmittel, etwa Chloroform,in der so erhaltenen Ar-halogenacetyl-, z.B. -bromacetyl-Verbindung das Halogen durch die Aminogruppe, z.B. durch Umsetzen mit Hexamethylentetramin in einem Lösungsmittel, etwa einem Chlorkohlenwasserstoff, wie Chloroform ersetzt, das erhaltene Addukt mit verdünnter Mineralsäure, z.B. Salzsäure zersetzt, in der erhaltenen Verbindung der Formel $Ar-CO-CH_2-NH_2$ (VIId) die Carbonylgruppe zur Carbinolgruppe, z.B. mittels Diboran, reduziert und die erhaltene Verbindung der Formel

$$\mathrm{Ar - \underset{\underset{OH}{|}}{CH} - CH_2 - NH_2} \qquad \mathrm{(VIIe)}$$

mit einer Verbindung der oben erläuterten Formel VIIb in der dort angegebenen Weise umsetzt.

Eine Verbindung der Formel $VII^b$ wiederum kann durch Einwirken von Essigsäureanhydrid auf das dem Cyanid entsprechende Oxim erhalten werden. Dies geschieht zweckmässigerweise durch Kochen unter Rückfluss. Das Oxim kann seinerseits aus dem entsprechenden Aldehyd durch Kochen mit Hydroxylamin-hydrochlorid in Gegenwart von alkoholischer Natriumcarbonatlösung unter Rückfluss hergestellt werden. Der entsprechende Aldehyd wiederum kann durch Umsetzung von 2,4-Dihydroxybenzaldehyd mit einem $\alpha,\beta$-, $\alpha,\gamma$- oder $\alpha,\omega$-Dihalogenniederalkan, vorzugsweise in Gegenwart eines basischen Mittels, hergestellt werden. Man kann auch auch in analoger Weise ein Hydroxysalicylonitril, z.B. das 2,4-Dihydroxybenzonitril [Chem.Ber. 24, 3657 (1891)] oder das 2,5-Dihydroxybenzonitril [Helv. Chim. Acta 30, 149, 153 (1947)] mit einem nicht-geminalen Dihalogenniederalkan zu einer Verbindung der Formel $VII^b$ umsetzen.

Die neuen Verbindungen der Formel I, worin m für 1, 2 oder 3 steht, können ebenfalls erhalten werden, indem man in einer Verbindung der Formel

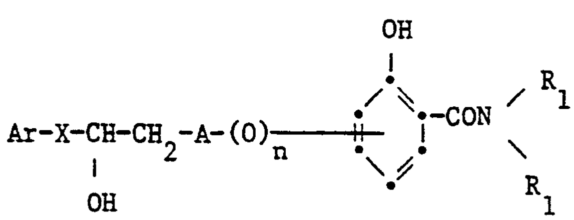

(VIII),

worin A für die Gruppe $-\underset{H}{N}-$alk-oder $-N=alk_1-$ steht, worin $alk_1$ die eine Methylengruppe weniger aufweisende Gruppe alk darstellt, und X eine mittels Reduktion, einschliesslich Hydrogenolyse, in eine Methylen-, 1,2-Aethylen- oder 1,3-Propylengruppe überführbare Gruppe darstellt, und eine oder beide Hydroxygruppen und/oder im Rest Ar gegebenenfalls vorhandene Hydroxy- und/oder Aminogruppen durch solche mittels Reduktion, einschliesslich Hydrogenolyse, abspaltbaren und durch Wasserstoff ersetzbaren Gruppen geschützt sind, die unter den Bedingungen des Verfahrens abgespalten und durch Wasserstoff ersetzt werden, die Gruppe X mittels Reduktion einschliesslich Hydrogenolyse in eine Methylen-, 1,2-Aethylen- oder 1,3-Propylengruppe und gleichzeitig eine gegebenenfalls vorhandene ungesättigte Gruppe A in die gesättigte Gruppe A, und gleichzeitig gegebenenfalls vorhandene geschützte Hydroxy- und/oder Aminogruppen in die freien Hydroxy- und/oder Aminogruppen umwandelt, und, wenn erwünscht, die anschliessend an das erste Verfahren genannten zusätzlichen Verfahrensschritte durchführt.

Eine Gruppe X stellt insbesondere eine anstelle einer Methylengruppe stehende Carbonylgruppe dar, die entsprechend der Bedeutung von m als 1, 2 oder 3 entweder alleine steht oder zusammen mit einer oder zwei Methylengruppen jede der möglichen Stellungen einnimmt. Eine Gruppe X stellt ausserdem eine zwei- oder dreifach ungesättigte Gruppe mit 2 bis 3 Kohlenstoffatomen, z.B. eine 1,2-Aethenylen-, 1,2-Propenylen-, 2,3-Propenylen- oder 1,2-Aethinylen- oder 1,3-Propinylengruppe dar.

Die Reduktion der Gruppe X erfolgt in üblicher Weise z.B. mittels aktiviertem Wasserstoff, etwa Wasserstoff in Gegenwart eines Hydrierkatalysators, z.B. eines Nickel- oder gegebenenfalls auf einem Trägermaterial, wie Aktivkohle, niedergeschlagenen Edelmetallkatalysators wie Platin oder Palladium, oder eines Platin- oder Palladium-auf-Kohle-Katalysators. Hierbei werden

gegebenenfalls vorhandene hydrogenolytisch abspaltbare Hydroxy- und/oder Aminoschutzgruppen abgespalten und durch Wasserstoff ersetzt. Die Reduktion, einschliesslich Hydrogenolyse, wird in an sich bekannter Weise, üblicherweise in Gegenwart eines inerten Lösungsmittels bei normalem oder erhöhtem Druck und normaler oder erhöhter Temperatur vorgenommen.

Ausgangsstoffe der Formel VIII, worin X eine Gruppe der Formeln -CO- (VIIIa), $-CO-CH_2-$ (VIIIb), oder $-CO-CH_2-CH_2-$(VIIIc) bedeutet, und im Rest Ar, wie vorhin angegeben, geschützte Hydroxy- und/oder Aminogruppen vorhanden sind, können in an sich bekannter Weise erhalten werden, in dem man in einer Verbindung der Formel

$$Ar - X - CH = CH_2 \qquad (IX)$$

die Gruppe $-CH=CH_2$ zur Gruppe $-\overset{O}{\overbrace{CH - CH_2}}$ epoxidiert, (vgl. hierzu: Cahnmann, Bull.Soc.Chim France [5], 4, 1937, 226, 230.) z.B., wie unter den Ausgangsstoffen der Formel (II) beschrieben, durch Umsetzung mit einer Peroxy-Verbindung, wie Wasserstoffperoxid, oder einer organischen Persäure, wie z.B. einer gegebenenfalls substituierten, wie halogenierten aliphatischen oder aromatischen Persäure, z.B. Peressigsäure oder Trifluorperessigsäure, Perbenzoesäure oder m-Chlorperbenzoesäure in einem wasserfreien Lösungsmittel, z.B. Chloroform oder Dioxan. Anschliessend kann man die erhaltene Verbindung oder eine hieraus z.B. durch Umsetzung mit einer Halogenwasserstoffsäure, z.B. Chlorwasserstoffsäure in einem Lösungsmittel, wie Dioxan, erhaltene Verbindung der allgemeinen Formel

$$Ar - X - \overset{X_1}{\overset{|}{CH}} - CH_2 - Z_3 \qquad (X),$$

worin $X_1$ und $Z_3$ zusammen Epoxy oder $X_1$ Hydroxy und $Z_3$ Halogen z.B. Chlor,

bedeutet, mit Ammoniak, oder falls $X_2$ und $Z_3$ Halogen ist, mit Hexamethylentetramin und anschliessender Zersetzung des erhaltenen Addukts mit einer verdünnten Mineralsäure, z.B. Salzsäuren, in eine Verbindung der Formel

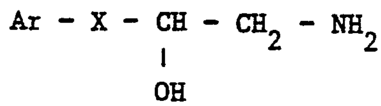
(XI)

umwandeln. Aus dieser lässt sich durch Umsetzung mit einer Verbindung der Formel

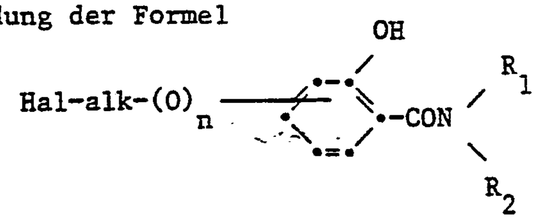
(XII),

worin Hal für Halogen und insbesondere für Chlor steht, ein Ausgangsmaterial der Formel VIII mit der Gruppe A der Formel $-\underset{H}{N}-alk-$, oder durch Umsetzung mit einer Verbindung der Formel

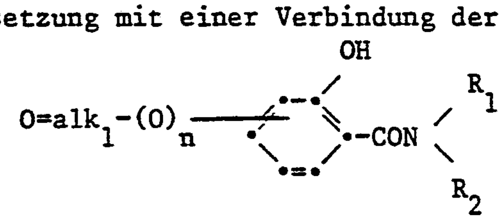
(XIII),

worin $alk_1$ obige Bedeutung hat, ein Ausgangsmaterial der Formel VIII, worin die Gruppe A der Formel $-N{=}alk_1-$ entspricht, herstellen. Diese Umsetzungen werden in an sich bekannter Weise vorgenommen, z.B. verwendet man bei der Umsetzung mit einer Verbindung der Formel XII ein alkalisches Kondensationsmittel, z.B. ein Alkali- oder Erdalkalicarbonat, wie Natrium- bzw. Calciumcarbonat.

Ausgangsstoffe der Formel VIII, worin X eine zwei- oder dreifach ungesättigte Gruppe mit 2 bis 3 Kohlenstoffatomen bedeutet, und A die angegebene Bedeutung hat, können erhalten werden, indem man eine Verbindung der Formel

$$Ar - X - CHO \qquad (XIV),$$

worin X eine zwei- oder dreifach ungesättigte Gruppe mit 2-3 Kohlenstoffatomen bedeutet, oder ein geeignetes Derivat davon, etwa ein Acetal, z.B. das Dimethylacetal, oder eine Bisulfitadditionsverbindung, mit einer geeigneten Silicium-organischen Cyanverbindung, etwa einem Triniederalkyl-, z.B. Trimethylsilylcyanid, in üblicher Weise in Gegenwart eines Katalysators, wie Zinkjodid, in eine Verbindung der Formel

$$Ar - X - CH \begin{matrix} \diagup CN \\ \diagdown Si(CH_3)_3 \end{matrix} \qquad (XV)$$

umwandelt, und in dieser die Gruppe, -CN mittels Reduktion, unter gleichzeitigem Ersatz der Trimethylsilylgruppe durch Hydroxy in die Gruppe $-CH_2NH_2$ überführt. Als Reduktionsmittel kann man z.B. ein Dileichtmetallhydrid, etwa Lithiumaluminiumhydrid, in einem geeigneten Lösungsmittel verwenden, wozu üblicherweise eine ätherartige Flüssigkeit, z.B. Diäthyläther oder Tetrahydrofuran verwendet werden kann. Die hiernach erhaltene Verbindung der Formel

$$Ar - X - \underset{\substack{| \\ OH}}{CH} - CH_2 - NH_2 \qquad (XVI)$$

kann man anschliessend mit einer Verbindung der oben erläuterten Formel XII zu einer Verbindung der Formel (VIII) umsetzen, worin A die Gruppe der Formel $-\underset{H}{N}-alk-$ darstellt; oder man setzt eine Verbindung der Formel XVI mit einer Verbindung der oben erläuterten Formel (XIII) um, wonach ein Ausgangsmaterial der Formel VIII erhalten wird, worin A die Gruppe der Formel $-N=alk_1-$ bedeutet.

Diese Umsetzungen werden in üblicher Weise durchgeführt.

Bei der Auswahl des geeigneten obigen Verfahrens zur Herstellung von Verbindungen der Formel I muss darauf geachtet werden, dass vorhandene Substituenten, in erster Linie der Reste Ar, nicht umgewandelt oder abgespalten werden, falls solche Umwandlungen bzw. Abspaltungen nicht erwünscht sind. So können insbesondere funktionell abgewandelte Carboxylgruppen, wie veresterte oder amidierte Carboxylgruppen, sowie Cyangruppen, als Substituenten der Reste Ar während Solvolysen, insbesondere Hydrolysen, ferner auch bei Reduktionen an der Reaktion beteiligt sein und umgewandelt werden. Andererseits können gleichzeitige Umwandlungen von Substituenten erwünscht sein; z.B. können ungesättigte Substituenten, wie Niederalkenyl, unter den Bedingungen eines erfindungsgemäss eingesetzten Reduktionsverfahrens, z.B. zu Niederalkyl, reduziert werden.

In erhaltenen Verbindungen kann man im Rahmen der Definition der Verbindungen der Formel I in üblicher Weise verfahrensgemäss erhaltene Verbindungen in andere Endstoffe überführen, z.B. indem man geeignete Substituenten abwandelt, einführt oder abspaltet.

So kann man in erhaltenen Verbindungen im Rest Ar stehende ungesättigte Substituenten wie Niederalkinyl bzw. Niederalkinyloxy, oder Niederalkenyl bzw. Niederalkenyloxy, z.B. durch Behandeln mit katalytisch aktiviertem Wasserstoff, reduzieren.

In Verbindungen mit Halogenatomen als Substituenten des Restes Ar kann man das Halogen, z.B. durch Behandeln mit Wasserstoff in Gegenwart eines üblichen Hydrierkatalysators, wie Raney-Nickel oder Palladium auf Kohle, durch Wasserstoff ersetzen.

In Verbindungen mit einer veresterten Carboxylgruppe als Substituent im Rest Ar kann diese in üblicher Weise, z.B. durch Ammonolyse oder Aminolyse mit Ammoniak oder einem primären oder sekundären Amin in die entsprechende Carbamoylgruppe übergeführt werden.

Verbindungen mit einer Carbamoylgruppe im Rest Ar und von Wasserstoff verschiedenen Gruppen $R_1$ und $R_2$ können in üblicher Weise, z.B. durch Einwirkung wasserentziehender Mittel, wie Phosphorpentoxid oder Phorphoroxychlorid, vorzugsweise bei höheren Temperaturen zu den entsprechenden Cyanverbindungen dehydratisiert werden.

In Verbindungen mit einer Cyangruppe als Substituenten im Rest Ar kann diese in üblicher Weise, z.B. durch Addition von Alkoholen in Gegenwart einer wasserfreien Säure, wie Chlorwasserstoff, und nachträglicher Hydrolyse des entstandenen Imidoesters zu den entsprechenden Verbindungen mit veresterten Carboxylgruppen alkoholysiert werden.

In Verbindungen mit einer Nitrogruppe als Substituenten im Rest Ar kann man diese zur Aminogruppe reduzieren, z.B. mittels katalytisch aktiviertem Wasserstoff, wie Wasserstoff in Gegenwart eines Hydrierkatalysators, etwa Raney-Nickel oder eines Palladium-auf-Kohle-Katalysators, oder mittels eines Metalls, z.B. Eisen oder gegebenenfalls amalgamierten Zink in einer Säure, z.B. einer Mineralsäure, wie Salzsäure oder einer Carbonsäure, wie Essigsäure oder Gemischen davon.

In Verbindungen mit einer primären Aminogruppe als Substituenten im Rest Ar kann man diese in eine Niederalkylamino- bzw. Diniederalkylaminogruppe überführen z.B. durch Umsetzung mit einem entsprechenden geeigneten Alkylierungsmittel, etwa einem reaktionsfähig veresterten Niederalkanol, z.B. dem Halogenid, etwa dem Chlorid, Bromid oder Jodid, oder einem Ester mit einer starken organischen Sulfonsäure, z.B. dem Methansulfonsäureester, zweckmässigerweise in Gegenwart eines basischen Mittels, wie eines Alkalihydroxids oder -carbonats.

In Verbindungen mit einer primären Aminogruppe im Rest Ar kann man diese in die Ureido-, N'-Niederalkylureido- oder N,N'-Diniederalkylureidogruppe umwandeln, z.B. durch Umsetzung mit Cyansäure oder einem

Salz davon, etwa Kaliumcyanat, im sauren Medium, wie wässriger Salzsäure, oder mit einem Niederalkyl- bzw. N,N-Diniederalkylharnstoff bei erhöhter Temperatur.

Zu Verbindungen mit einer primären Aminogruppe im Rest Ar kann man diese in eine Niederalkylsulfonylaminogruppe umwandeln, z.B. durch Umsetzung mit einem Niederalkylsulfonylhalogenid in Gegenwart eines basischen Mittels, z.B. Pyridin.

Wie bei den Herstellungsverfahren muss auch bei der Durchführung der Zusatzschritte darauf geachtet werden, dass unerwünschte Nebenreaktionen, welche die Umwandlung zusätzlicher Gruppierungen zur Folge haben können, nicht eintreten.

Die oben beschriebenen Reaktionen können gegebenenfalls gleichzeitig oder nacheinander, ferner in beliebiger Reihenfolge durchgeführt werden. Falls notwendig, erfolgen sie in Anwesenheit von Verdünnungsmitteln, Kondensationsmitteln und/oder katalytisch wirkenden Mitteln, bei erniedrigter oder erhöhter Temperatur, im geschlossenen Gefäss unter Druck und/oder in einer Inertgasatmosphäre.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten oder Ionenaustauschern, in die freien Verbindungen übergeführt werden. Andererseits können erhaltene freie Basen mit organischen oder anorganischen Säuren, z.B. mit den genannten Säuren, Säureadditionssalze bilden, wobei zu deren Herstellung insbesondere solche

Säuren verwendet werden, die sich zur Bildung von pharmazeutisch annehmbaren Salzen eignen.

Diese oder andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate, oder sofern sie mindestens zwei asymmetrische Kohlenstoffatome enthalten, auch als Racematgemische vorliegen. Die Ausgangsstoffe können auch als bestimmte optische Antipoden eingesetzt werden.

Erhaltene Racematgemische können auf Grund der physikalischchemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-O,O'-(p-Toluoyl)-weinsäure, Aepfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, wie oben, z.B. analog wie in den Beispielen beschrieben, erhalten werden. Neue Ausgangsstoffe bilden ebenfalls einen Gegenstand der Erfindung. Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen, enthalten, die sich zur enteralen, z.B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium- oder

Calciumstearat, und/oder Polyäthylenglykol, aufweisen. Tabletten können ebenfalls Bindemittel, z.B.Magnesiumaluminiumsilikat,. Stärken, wie Mais-, Weizen-, Reis- oder Pfeilwurzstärke, Gelatine, Traganth, Methylcellulsoe, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Dosierung kann von verschiedenen Faktoren, wie Applikationsweise, Spezies, Alter und/oder individuellem Zustand abhängen. So liegen die täglich in einer oder mehreren, vorzugsweise höchstens 4 Einzeldosen zu verabreichenden Dosen bei oraler Applikation an Warmblüter für β-Rezeptoren-Blocker der Formel I zwischen 0,03mg/kg und 3 mg/kg und für Warmblüter von etwa 70 kg Körpergewicht vorzugsweise zwischen etwa 0,004g und etwa 0,08 g,und für β-Rezeptoren-Stimulatoren der Formel I zwischen 0,01 mg/kg und 1 mg/kg bzw. für Warmblüter von etwa 70 kg Körpergewicht zwischen etwa 0,002 g und etwa 0,04 g.

Die folgenden Beispiele dienen zur Illustration der Erfindung; Temperaturen werden in Celsiusgraden angegeben.

Bei Verbindungen, die zwei Asymmetriezentren besitzen und daher als Diastereomeren-Gemische vorliegen können, wurde der relative Gehalt der beiden Diastereomeren (Enantiomeren-Paare) mittels $^{13}$C-NMR-Spektroskopie anhand der relativen Intensität der beiden C-Methyl-Signale bei 13,3 ± 0,1 ppm bezw. 13,6 ± 0,1 ppm festgestellt. Die $^{13}$C-Spektren wurden in DMSO-$d_6$ auf einem Varian-XL-100-Instrument bei 25,16 MHz unter Verwendung von Tetramethylsilan als internem Standart aufgenommen. (Digital-Auflösung 0,8 Punkte/Hz). Salze wurden als solche untersucht. Basen müssen mit Säuren (z.B. 1 Aequivalent Fumarsäure) neutralisiert werden, um eine Separation der Methyl-Signale zu erzielen.

Beispiel 1: Eine Lösung von 1,96 g 5-(2-Aminoäthoxy)-salicylamid in 15 ml wasserfreiem Dimethylsulfoxid wird mit 2,4 g 2,4-Dichlor-1-(epoxyäthyl)-benzol versetzt und 30 Minuten in einem Bad von 80° gerührt. Das Reaktionsgemisch wird auf 30 ml Eiswasser gegossen und mit 100 ml Aethylacetat extrahiert. Die organische Phase wird abgetrennt, über Magnesiumsulfat getrocknet und eingedampft. Der Eindampfrückstand wird mit 20 ml Petroläther verrührt, die erhaltenen Kristalle abgesaugt und aus Methanol umkristallisiert, wonach man den α-[N-[2-(3-Carbamoyl-4-hydroxy-phenoxy)-äthyl]-aminomethyl]-2,4-dichlorbenzylalkohol vom Smp. 139-140° erhält.

Das als Ausgangsmaterial benötigte 5-(2-Aminoäthoxy)-salicylamid kann wie folgt hergestellt werden:

a) Nach der von Irvine et al., Synthesis 1972, 568 beschriebenen Methode wird 2,5-Dihydroxy-benzamid unter Verwendung eines Ueberschusses von Aceton in 2,3-Dihydro-2,2-dimethyl-6-hydroxy-4H-1,3-benzoxazin-4-on vom Smp. 215-216° übergeführt.

b) Ein Gemisch von 70 g 2,3-Dihydro-2,2-dimethyl-6-hydroxy-4H-1,3-benzoxazin-4-on, 75 g getrocknetem Kaliumcarbonat und 400 ml 1,2-Dibromäthan wird während 14 Stunden unter Rühren und Rückfluss gekocht. Das Gemisch wird abgekühlt und filtriert, der Rückstand mit 400 ml Wasser gründlich verrührt, abgesaugt und der Rückstand im Vakuum bei 80° getrocknet. Man erhält rohes 6-(2-Bromäthoxy)-2,3-dihydro-2,2-dimethyl-4H-1,3-benzoxazin-4-on vom Smp. 190-200°, das für weitere Umsetzungen genügend rein ist. Durch Umkristallisation aus Methanol erhält man ein reines Produkt vom Smp. 205-208°.

c) Ein Gemisch von 60 g 6-(Bromäthoxy)-2,3-dihydro-2,2-dimethyl-4H-1,3-benzoxazin -4-on und 110 ml Benzylamin wird in einem Bad von 80° während 30 Minuten gerührt. Das Reaktionsgemisch wird hierauf unter Eiskühlung mit konz. Salzsäure auf pH 3-4 gebracht und kristallisieren gelassen. Nach 2-4 Stunden werden die Kristalle abgesaugt, mit je 50 ml Wasser und Aethylacetat gewaschen und getrocknet. Das so erhaltene 5-[(2-Benzylamino)-äthoxy]-salicylamid-hydrochlorid schmilzt bei 214-216°. Die daraus freigesetzte Base schmilzt bei 107-108° (aus Aethylacetat-Aether).

d) Katalytische Debenzylierung von 5-[(2-Benzylamino)-äthoxy]-salicylamid in Methanol mittels eines Palladium-auf-Kohle-Katalysators (5 %-ig) führt zum 5-(2-Aminoäthoxy)-salicylamid vom Smp. 140°.

Beispiel 2: Analog Beispiel 1 erhält man aus 17,4 g 4-Chlor-1-(epoxyäthyl)-3-nitrobenzol und 11,4 g 5-(2-Aminoäthoxy)-salicylamid in 50 ml Dimethylsulfoxid den α-[N-[2-(3-Carbamoyl-4-hydroxy-phenoxy)-äthyl]-aminomethyl]-4-chlor-3-nitrobenzylalkohol vom Smp. 167-168° (aus Aethylacetat).

Beispiel 3: 8 g 2-Chlor-5-(epoxyäthyl)-benzolsulfonamid und 5 g 5-(2-Aminoäthoxy)-salicylamid werden in 40 ml Dimethylsulfoxid gelöst und die Lösung unter Rühren während 65 Minuten auf 90° erhitzt. Anschliessend wird abgekühlt, das Reaktionsgemisch auf Eiswasser gegossen und mit Aethylacetat extrahiert. Die organische Phase wird danach mit 1-n. wässriger Methansulfonsäurelösung ausgezogen, die saure Phase mit Ammoniak alkalisch gestellt und mehrfach mit Aethylacetat extrahiert. Nach dem Trocknen der organischen Phase über Natriumsulfat wird das Lösungsmittel am Rotationsverdampfer entfernt und der Rückstand aus Isopropanol umkristallisiert. Nach nochmaliger Umkristallisation aus Methanol erhält man den α-[N-[2-(3-Carbamoyl-4-hydroxy-phenoxy)-äthyl]-aminomethyl]-3-sulfamoyl-4-chlor-benzylalkohol vom Smp. 193-195°.

Das als Ausgangsmaterial benötigte 2-Chlor-5-(epoxyäthyl)-benzolsulfonamid kann wie folgt hergestellt werden:

a) Eine Lösung von 20 g 5-(Bromacetyl)-2-chlorbenzolsulfonamid (DE-OS 26 01 598) in 800 ml Methanol wird bei 0-5° innerhalb 1 1/2 Stunden portionenweise mit 13,5 g Natriumborhydrid versetzt. Man rührt noch 3 Stunden bei Raumtemperatur weiter, entfernt das Lösungsmittel am Rotationsverdampfer und verteilt den Rückstand zwischen Wasser und Aethylacetat. Die organische Phase wird getrocknet, eingedampft und der Rückstand aus Aethylacetat/Aether umkristallisiert, wobei man das 2-Chlor-5-(epoxyäthyl)-benzolsulfonamid vom Smp. 126-129° erhält.

<u>Beispiel 4</u>: Eine Lösung von 21,9 g 4-(2-Aminoäthoxy)-salicylamid und 30,3 g α-(Brommethyl)-4-nitrobenzylalkohol in 250 ml Aethanol wird nach Zusatz von 20 g Kaliumbicarbonat 12 Stunden unter Rückfluss und Rühren gekocht. Nach dem Abkühlen und Filtrieren wird das Filtrat eingedampft und der Rückstand zwischen 50 ml 2-n. Salzsäure und 100 ml Aethylacetat verteilt. Die wässrige Phase wird eingedampft und der Rückstand aus Methanol umkristallisiert. Man erhält den α-[N-[2-(4-Carbamoyl-3-hydroxy-phenoxy)-äthyl]-aminomethyl]-4-nitrobenzylalkohol als Hydrochlorid; Smp. 231-233° (aus Methanol).

Das als Ausgangsmaterial benötigte 4-(2-Aminoäthoxy)-salicylamid wird wie folgt erhalten:

a) 16,2 g des nach Beispiel 8b) erhaltenen 2,3-Dihydro-2,2-dimethyl-7-hydroxy-4H-1,3-benzoxazin-4-on werden analog Beispiel 1b) mit 84 ml 1,2-Dibromäthan umgesetzt und ergeben das 2,3-Dihydro-2,2-dimethyl-7-(2-bromäthoxy)-4H-1,3-benzoxazin-4-on vom Smp. 156-158° (aus Isopropanol).

b) 53 g 2,3-Dihydro-2,2-dimethyl-7-(2-bromäthoxy)-4H-1,3-benzoxazin-4-on und 94 g Benzylamin werden unter Rühren 3 Stunden gekocht. Das Reaktionsgemisch wird mit konz. Ammoniak alkalisch gestellt, und die organische Phase bei max. 50° eingedampft. Das so erhaltene 4-[2-(Benzylamino)-äthoxy]-salicylamid bildet ein Oel, dessen Hydrochlorid bei 252-254° schmilzt (aus Methanol).

c) Katalytische Debenzylierung von 4-[(2-Benzylamino)-äthoxy]-salicylamid analog Beispiel 1 ergibt das 4-(2-Aminoäthoxy)-salicylamid vom Smp. 126-130° (aus Isopropanol).

Beispiel 5: Ein Gemisch von 16,4 g N-[5-(2-Brom-1-hydroxyäthyl)-2-chlor-phenyl]-methansulfonamid, 14,7 g 5-(2-Aminoäthoxy)-salicylamid und 5 g Kaliumbicarbonat in 100 ml Isopropanol wird während 25 Stunden unter Rühren und Rückfluss gekocht. Das Reaktionsgemisch wird filtriert, das Filtrat eingedampft und zwischen Aether und 2-n. Salzsäure verteilt. Die wässrige Phase wird mit festem Kaliumbicarbonat alkalisch gestellt und das Gemisch mit Aethylacetat extrahiert. Aus dem nach dem Eindampfen erhaltenen Schaum kristallisiert allmählich aus wenig Aethylacetat der α-[N-[2-(3-Carbamoyl-4-hydroxy-phenoxy)-äthyl]-aminomethyl]-3-(methylsulfonylamino)-4-chlor-benzylalkohol vom Smp. 139-141°. Er bildet ein neutrales Fumarat vom Smp. 190-192° (aus Methanol).

Das als Ausgangsmaterial benötigte N-[5-(2-Brom-1-hydroxyäthyl)-2-chlor-phenyl]-methansulfonamid wird auf folgende Weise hergestellt:

a) Unter Eiskühlung werden zu einer Lösung von 162,5 g 3-Amino-4-chlor-acetophenon in 900 ml Pyridin 110 g Methansulfonsäurechlorid getropft. Nach weiterem Rühren bei Zimmertemperatur während 3 Stunden wird der entstandene Niederschlag abfiltriert und das Filtrat eingedampft. Der Rückstand kristallisiert beim Versetzen mit Wasser. Das erhaltene rohe N-(2-Chlor-5-acetyl-phenyl)-methansulfonamid wird aus Aethanol umkristallisiert; Smp. 112-114°.

b) Durch Bromieren der gemäss Beispiel 5a) erhaltenen Verbindung mit der äquivalenten Menge Brom in Chloroform erhält man das N-(5-Bromacetyl-2-chlor-phenyl)-methansulfonamid vom Smp. 120-123°.

c) Durch Reduktion der gemäss Beispiel 5b) erhaltenen Verbindung mit der äquivalenten molaren Menge Natriumborhydrid in Dioxan-Wasser (9:1) erhält man das N-[5-(2-Brom-1-hydroxyäthyl)-2-chlor-phenyl]-methansulfonamid vom Smp. 130-134°.

Beispiel 6: 4,7 g 4-Amino-α-(aminomethyl)-3,5-dichlor-benzylalkohol, 6,0 g 5-(2-Bromäthoxy)-salicylamid und 12,9 g N,N-Diisopropyl-äthylamin werden in einem Gemisch von 20 ml Dimethylformamid und 50 ml Dioxan 3 Stunden unter Rückfluss gekocht. Nach dem Abdampfen des Lösungsmittels wird der Rückstand analog Beispiel 5 aufgearbeitet. Man erhält den α-[N-[2-(3-Carbamoyl-4-hydroxy-phenoxy)-äthyl]-aminomethyl]-3,5-dichlor-4-amino-benzylalkohol als bräunliche Kristalle vom Smp. 196-201°.

Das als Ausgangsmaterial benötigte 5-(2-Bromäthoxy)-salicylamid kann wie folgt erhalten werden:

a) 30,0 g des nach Beispiel 1b) erhaltenen 6-(2-Bromäthoxy)-2,3-dihydro-2,2-dimethyl-4H-1,3-benzoxazin-4-on werden in einem Gemisch von 100 ml Dioxan und 100 ml 6-n. Salzsäure unter Rühren 1 1/2 Stunden am Rückfluss gekocht. Die nach dem Eindampfen des Reaktionsgemisches erhaltenen Kristalle werden mit 50 ml Wasser gewaschen und im Vakuum getrocknet. Das so erhaltene 5-(2-Bromäthoxy)-salicylamid schmilzt bei 141-143°.

Der als Ausgangsmaterial benötigte 4-Amino-α-(aminomethyl)-3,5-dichlor-benzylalkohol wird auf folgende Weise hergestellt:

b) Eine Suspension von 17,9 g Phthalimid in 70 ml Dimethylformamid wird portionenweise unter Rühren mit 6,5 g Natriumhydrid-Suspension (55 % in Paraffinöl) versetzt. Nach einer Stunde wird unter weiterem Rühren und Kühlung bei 10-20° eine Lösung von 34,5 g 4-Amino-ω-brom-3,5-dichlor-acetophenon in 50 ml Dimethylformamid zugetropft, das Reaktionsgemisch noch 3-4 Stunden nachgerührt und dann auf 1200 ml Wasser gegossen. Die ausgefallenen Kristalle werden abgesaugt und im Vakuum bei 100° getrocknet. Man erhält rohes N-[2-(4-Amino-3,5-dichlor-phenyl)-2-oxo-äthyl]-phthalimid vom Smp. 255-260°.

c) Eine Suspension von 23,6 g N-[2-(4-Amino-3,5-dichlor-phenyl)-2-oxo-äthyl]-phthalimid in einem Gemisch von 150 ml Dioxan und 20 ml Wasser wird portionenweise mit 5,2 g Natriumborhydrid versetzt und hierauf 48 Stunden bei 80-90° gerührt. Nach 24 Stunden werden nochmals 5,2 g Natriumborhydrid zugegeben.

Das überschüssige Natriumborhydrid wird unter Eiskühlung mit 2-n. Salzsäure zersetzt und das Reaktionsgemisch eingedampft. Der Eindampfrückstand wird mit konz. Ammoniaklösung alkalisch gestellt und mit Aethylacetat extrahiert. Nach dem Entfernen des Lösungsmittels erhält man den rohen 4-Amino-3,5-dichlor-α-[N-(2-hydroxymethyl-benzoyl)-aminomethyl]-benzylalkohol vom Smp. 165-170°, der ohne weitere Reinigung weiterverarbeitet wird.

d) 19,4 g des Rohproduktes gemäss Beispiel 6c) und 4,8 g Natriumhydroxid werden in 200 ml Aethanol 5 Stunden unter Rückfluss gekocht. Durch Verteilen des Eindampfrückstandes zwischen 200 ml Aethylacetat und 30 ml Wasser und üblichem Aufarbeiten erhält man den 4-Amino-α-(aminomethyl)-3,5-dichlor-benzylalkohol vom Smp. 124-127°.

Beispiel 7: Ein Gemisch von 8,7 g N-[3-(2-Amino-1-hydroxy-äthyl)-phenyl]-methansulfonamid und 9,0 g 5-(2-Bromäthoxy)-salicylamid in 50 ml Dioxan und 20 ml Triäthylamin wird 4 Stunden unter Rühren und Rückfluss gekocht. Der durch Eindampfen erhaltene Schaum wird mit 100 ml gesättigter Kaliumbicarbonat-Lösung und 500 ml Aethylacetat bis zur vollständigen Lösung verrührt. Die organische Phase ergibt nach dem Trocknen über Magnesiumsulfat und Eindampfen 12 g eines grünlichen Schaumes, aus dem durch Lösen in einem Gemisch von Methanol und Isopropanol (ca. 1:10) der α-[N-[2-(3-Carbamoyl-4-hydroxy-phenoxy)-äthyl]-aminomethyl]-3-(methylsulfonylamino)-benzylalkohol vom Smp. 135-137° auskristallisiert.

Beispiel 8: 23 g roher α-[N-[2-(2,3-Dihydro-2,2-dimethyl-4-oxo-4H-1,3-benzoxazin-7-yloxy)-1-methyl-äthyl]-aminomethyl]-4-(methylsulfonylamino)-benzylalkohol werden in einer Mischung von 50 ml Isopropylamin und 200 ml Isopropanol 2 Stunden unter Rückfluss gekocht und hierauf eingedampft, und der Rückstand mit 5 ml Isopropanol und 20 ml Aether verrührt. Die sich allmählich bildenden Kristalle werden abgesaugt und stellen den α-[N-[2-(4-Carbamoyl-3-hydroxy-phenoxy)-1-methyl-äthyl]-aminomethyl]-4-(methylsulfonylamino)-benzylalkohol als Diastereoisomeren-Gemisch dar. Ein 1:1-Gemisch der Diastereoisomeren schmilzt bei 140-145°.

Das Ausgangsmaterial wird auf folgende Weise hergestellt:

a) Eine Lösung von 11,5 g N-[4-(2-Amino-1-hydroxy-äthyl)-phenyl]-methansulfonamid und 12,4 g 2,3-Dihydro-2,2-dimethyl-7-(2-oxo-propoxy)-4H-1,3-benzoxazin-4-on in 250 ml Methanol wird nach Zusatz von 0,13 g konz. Schwefelsäure über 1g Platin-auf-Kohle-Katalysator bis zur Aufnahme der berechneten Menge Wasserstoff unter Normalbedingungen hydriert. Nach Abfiltrieren des Katalysators wird die Lösung eingedampft, wonach man den rohen α-[N-[2-(2,3-Dihydro-2,2-dimethyl-4-oxo-4H-1,3-benzoxazin-7-yloxy)-1-methyl-äthyl]-aminomethyl]-4-(methylsulfonylamino)-benzylalkohol als Oel erhält, welches als solches weiterverarbeitet wird.

Das als weiteres Ausgangsmaterial benötigte 2,3-Dihydro-2,2-dimethyl-7-(2-oxo-propoxy)-4H-1,3-benzoxazin-4-on kann wie folgt erhalten werden:

b) Analog Beispiel 1a) erhält man aus 2,4-Dihydroxy-benzamid das 2,3-Dihydro-2,2-dimethyl-7-hydroxy-4H-1,3-benzoxazin-4-on vom Smp. 249-251°.

c) Aus 168 g 2,3-Dihydro-2,2-dimethyl-7-hydroxy-4H-1,3-benzoxazin-4-on, 305 g Kaliumcarbonat und 88 ml Chloraceton in 1,2 Liter Acetonitril durch Kochen während 28 Stunden und nachfolgendem Aufarbeiten erhält man das 2,3-Dihydro-2,2-dimethyl-7-(2-oxo-propoxy)-4H-1,3-benzoxazin-4-on vom Smp. 160-162° (aus Isopropanol).

<u>Beispiel 9</u>: Analog Beispiel 8 und 8a) erhält man aus α-(Aminomethyl)-3,4-methylendioxy-benzylalkohol (Smp. 75-78°; vgl. H. Tatsuno et al., J. Med. Chem. <u>20</u>, 394, 1977) und 2,3-Dihydro-2,2-dimethyl-6-(2-oxo-propoxy)-4H-1,3-benzoxazin-4-on den α-[N-[2-(3-Carbamoyl-4-hydroxy-phenoxy)-1-methyl-äthyl]-aminomethyl]-3,4-methylendioxy-benzylalkohol als Diastereoisomeren-Gemisch. Durch fraktionierte Kristallisation des Hydrochlorids aus Methanol-Wasser (4:1) erhält man ein reines Enantiomeren-Paar vom Smp. 211-212°.

Das als Ausgangsmaterial benötigte 2,3-Dihydro-2,2-dimethyl-6-(2-oxo-propoxy)-4H-1,3-benzoxazin-4-on kann wie folgt erhalten werden:

a) 70 g des nach Beispiel 1a) erhaltenen 2,3-Dihydro-2,2-dimethyl-6-hydroxy-4H-1,3-benzoxazin-4-on werden in 400 ml Acetonitril mit 100 g Kaliumcarbonat und 32 ml Chloraceton 30 Stunden unter Rückfluss gerührt. Nach Zusatz von weiteren 3,2 ml Chloraceton wird das

Reaktionsgemisch während weiteren 15-20 Stunden erhitzt. Das noch warme Reaktionsgemisch wird filtriert, der Rückstand mit Aceton gründlich gewaschen und das vereinigte Filtrat eingedampft. Der kristalline Rückstand wird aus Toluol umkristallisiert und ergibt das 2,3-Dihydro-2,2-dimethyl-6-(2-oxo-propoxy)-4H-1,3-benzoxazin-4-on vom Smp. 125-126°.

Beispiel 10: Analog Beispiel 9 erhält man unter Verwendung von 2,3-Dihydro-2,2-dimethyl-7-(2-oxo-propoxy)-4H-1,3-benzoxazin-4-on den α-[N-[2-(4-Carbamoyl-3-hydroxy-phenoxy)-1-methyl-äthyl]-aminomethyl]-3,4-methylendioxy-benzylalkohol als Diastereoisomeren-Gemisch (ca. 1:1) vom Smp. 164-174°. Es bildet ein neutrales Fumarat vom Smp. 193-204° (aus Methanol-Wasser), das mittels $^{13}C$-Kernresonanz-Spektrum als ein Gemisch der Diastereomeren (ca. 1:1) identifiziert wird.

Beispiel 11: Eine Lösung von 0,795 g 1-(3-Sulfamoyl-4-chlor-phenyl)-2-amino-äthanol und 0,707 g (2,3-Dihydro-2,2-dimethyl-4H-1,3-benzoxazin-4-on-6-yloxy)-acetaldehyd in 20 ml Methanol wird mit 2 g Molekularsieben (3 Å) und 0,44 g Natriumcyanoborhydrid versetzt und die Suspension bei Raumtemperatur gerührt. Durch tropfenweise Zugabe von methanolischer Salzsäure wird der pH-Wert bei 6-7 gehalten. Nach 12 Stunden wird filtriert, das Filtrat zur Zersetzung des Cyanoborhydrids mit 1 ml konz. Salzsäure versetzt, 10 Minuten gerührt und anschliessend mit konz. Ammoniak auf pH 9 eingestellt. Die Suspension wird vom Lösungsmittel befreit, der Rückstand mit einem Gemisch von Chloroform/Methanol/Ammoniak (40:10:1) aufgeschlämmt, filtriert und das Filtrat erneut eingeengt. Der Rückstand wird an 150 g Kieselgel mit einem Gemisch von Chloroform/Methanol/Ammoniak (40:10:1) chromatographiert, die Hauptfraktion eingedampft und der Rückstand während 15 Minuten in einem Gemisch von 20 ml Dioxan und 1 ml konz. Salzsäure auf 80° erhitzt. Nach Entfernung des Lösungsmittels wird der Rückstand in Aethylacetat aufgenommen und mit 2-n. Ammoniak extrahiert. Die organische Phase wird getrocknet, eingedampft und der Rückstand aus Isopropanol-Aethylacetat umkristallisiert, wonach man den α-[N-[2-(3-Carbamoyl-4-hydroxy-phenoxy)-äthyl]-aminomethyl]-3-sulfamoyl-4-chlor-benzylalkohol erhält, Smp. 193-195°.

Der als Ausgangsmaterial benötigte 2,3-Dihydro-2,2-dimethyl-4H-1,3-benzoxazin-4-on-6-yloxy)-acetaldehyd kann wie folgt erhalten werden:

a) Eine Lösung von 9,65 g des nach Beispiel 1a) erhaltenen 2,3-Dihydro-2,2-dimethyl-6-hydroxy-4H-1,3-benzoxazin-4-on und 9,1 g Allylbromid in 150 ml Acetonitril wird unter Zugabe von 10,3 g trockenem Kaliumcarbonat 5 Stunden unter Rückfluss gerührt. Das Reaktionsgemisch wird warm filtriert, das Filtrat eingedampft und die verbleibenden Kristalle nach Verreiben mit Aether abgesaugt, wonach man das rohe 2,3-Dihydro-2,2-dimethyl-6-allyloxy-4H-1,3-benzoxazin-4-on vom Smp. 137-138° erhält.

b) Eine Lösung von 4,7 g 2,3-Dihydro-2,2-dimethyl-6-allyloxy-4H-1,3-benzoxazin-4-on in einem Gemisch von 50 ml Dioxan und 15 ml Wasser wird unter Rühren mit ca. 20 mg Osmiumtetroxid versetzt. Nach 15 Minuten wird portionenweise 8,6 g Natriummetaperjodat zugegeben, wobei die Temperatur bis 45° ansteigt. Nach 2 Stunden wird das Reaktionsgemisch filtriert, das Filtrat eingedampft und der Rückstand zwischen 20 ml Wasser und 200 ml Aethylacetat verteilt. Die organische Phase wird abgetrennt, getrocknet und eingedampft. Das verbleibende Oel wird über 100 g Kieselgel chromatographiert. Durch Eluieren mit Aethylacetat und Abdampfen zur Trockne wird (2,3-Dihydro-2,2-dimethyl-4H-1,3-benzoxazin-4-on-6-yloxy)-acetaldehyd vom Smp. 153-163° erhalten.

Beispiel 12: Eine Lösung von 9,2 g α-(Aminomethyl)-4-(methylsulfonylamino)-benzylalkohol und 8,5 g 4-(2-Oxo-propoxy)-salicylamid in 180 ml Methanol wird unter Zusatz von 0,1 g konz. Schwefelsäure über 1 g Platin-auf-Kohle-Katalysator (5 %) hydriert. Nach Filtrieren und Eindampfen des Filtrats erhält man einen Schaum, der aus Isopropanol nach

Stehen während mehreren Tagen den α-[N-[2-(4-Carbamoyl-3-hydroxy-phenoxy)-1-methyl-äthyl]-aminomethyl]-4-(methylsulfonylamino)-benzylalkohol als Diastereoisomeren-Gemisch (ca. 1:1) vom Smp. 141-152° ergibt. Die Isolierung der reinen Diasteromeren ist in Beispiel 28 beschrieben.

Das als Ausgangsmaterial benötigte 4-(2-Oxo-propoxy)-salicylamid kann wie folgt hergestellt werden:

a) 66 g rohes nach Beispiel 9a) erhaltenes 2,3-Dihydro-2,2-dimethyl-6-(2-oxopropoxy)-4H-1,3-benzoxazin-4-on wird in einem Gemisch von 150 ml Dioxan und 400 ml 2-n. Salzsäure 1 Stunde auf dem siedenden Wasserbad hydrolysiert. Nach dem Abdampfen des Lösungsmittels und Verreiben des Rückstandes mit Wasser erhält man rohes 4-(2-Oxo-propoxy)-salicylamid vom Smp. 128-140°. Nach Umkristallisation aus Aethanol schmilzt das Produkt bei 145-148°.

Beispiel 13: Analog Beispiel 12 erhält man unter Verwendung der äquivalenten Menge des nach Beispiel 10a) erhaltenen 5-(2-Oxo-propoxy)-salicylamid den α-[N-[2-(3-Carbamoyl-4-hydroxy-phenoxy)-1-methyl-äthyl]-aminomethyl]-4-(methylsulfonylamino)-benzylalkohol als Oel, welcher ein neutrales Fumarat als Gemisch der Diastereoisomeren im Verhältnis von ca. 6:4 gemäss $^{13}C$-Spektroskopie-Untersuchung vom Smp. 210-215° bildet.

Beispiel 14: Eine Lösung von 24,1 g 5-(2-Amino-1-hydroxy-äthyl)-salicylamid und 25,6 g 5-(2-Oxo-propoxy)-salicylamid wird analog Beispiel 12 über 2 g Platin-auf-Kohle-Katalysator (5 %), der in 2 Portionen zugegeben wird, bis zur Aufnahme der berechneten Menge Wasserstoff hydriert. Der Eindampfrückstand der filtrierten Lösung wird unter Zugabe von Fumarsäure in Wasser gelöst, die Lösung mit Aethylacetat extrahiert und die wässrige Phase abgetrennt. Diese wird mit gesättigter Kaliumbicarbonat-Lösung alkalisch gestellt und der ausgefallene Niederschlag mit viel Aethylacetat durch Rühren während 1 Stunde extrahiert. Aus der Aethylacetatlösung erhält man nach dem

Trocknen über Magnesiumsulfat und Eindampfen den α-[N-[2-(3-Carbamoyl-4-hydroxy-phenoxy)-1-methyl-äthyl]-aminomethyl]-3-carbamoyl-4-hydroxy-benzylalkohol, welcher nach dem Umkristallisieren aus Aethylacetat bei 143-148° unter Zersetzung schmilzt und ein Gemisch der Diastereoisomeren im Verhältnis von ca. 1:1 darstellt.

Beispiel 15: Eine Lösung von 10,0 g α-(Aminomethyl)-4-(2-methoxy-äthoxy)-benzylalkohol und 9,9 g 5-(2-Oxo-propoxy)-salicylamid wird in 200 ml Methanol analog Beispiel 12 hydriert und analog Beispiel 14 aufgearbeitet. Man erhält den α-[N-[2-(3-Carbamoyl-4-hydroxy-phenoxy)-1-methyl-äthyl]-aminomethyl]-4-(2-methoxy-äthoxy)-benzylalkohol, der nach Umkristallisation aus Aethylacetat bei 100-106° schmilzt und ein Diastereoisomeren-Gemisch im Verhältnis von ca. 1:1 darstellt.

Der als Ausgangsmaterial verwendete α-(Aminomethyl)-4-(2-methoxy-äthoxy)-benzylalkohol kann auf folgende Weise hergestellt werden:

a) Eine Suspension von 21 g 2-Methoxy-äthylbromid, 13,6 g 4-Hydroxy-acetophenon und 27,6 g Kaliumcarbonat in 250 ml Aceton wird 20 Stunden unter Rühren und Rückfluss gekocht. Uebliches Aufarbeiten und Destillation des Rohproduktes bei 0,001 Torr ergibt das 4-(2-Methoxy-äthoxy)-acetophenon vom Kp. 113-116°/0,001 Torr. als farbloses Oel, welches kristallin erstarrt.

b) Bromierung der gemäss Beispiel a) erhaltenen Verbindung mit der äquivalenten Menge Brom in Chloroform bei 20-30° während 2 Stunden ergibt das ω-Brom-4-(2-methoxy-äthoxy)-acetophenon, welches als solches weiterverwendet wird.

c) Eine Lösung von 53 g der rohen gemäss Beispiel b) erhaltenen Verbindung in 350 ml Dimethylformamid wird auf 0-5° gekühlt und unter Rühren portionenweise mit 15,4 g Natriumazid versetzt. Nach

Abklingen der exothermen Reaktion wird die Eiskühlung entfernt und das Reaktionsgemisch 3 Stunden nachgerührt. Die Lösung wird anschliessend auf 3 Liter Eiswasser gegossen und das sich abscheidende gelbe Oel mit Aether extrahiert. Die Aetherlösung (500 ml) wird zweimal mit 100 ml Wasser, dann mit 100 ml gesättigter Natriumchloridlösung gewaschen, über Magnesiumsulfat gründlich getrocknet, filtriert und das Filtrat mit einer Suspension von 35 g Lithiumaluminiumhydrid in 500 ml Aether während 2 Stunden bei 10-12° reduziert. Uebliches Aufarbeiten ergibt rohen α-(Aminomethyl)-4-(2-methoxy-äthoxy)-benzylalkohol als gelbliches Oel, welches als Rohprodukt weiterverwendet werden kann. Bei der Destillation im Kugelrohr siedet es bei 130-140°/0,08 Torr.

Beispiel 16: Analog der Arbeitsweise des Beispiels 12 erhält man aus 7,5 g α-(Aminomethyl)-4-methyl-benzylalkohol und 10,45 g 5-(2-Oxo-propoxy)-salicylamid den α-[N-[2-(3-Carbamoyl-4-hydroxy-phenoxy)-1-methyl-äthyl]-aminomethyl]-4-methyl-benzylalkohol vom Smp. 120-134° (aus Aethylacetat), welcher ein Gemisch der Diastereoisomeren im Verhältnis von ca. 1:1 darstellt..

Beispiel 17: Eine Lösung von 6,1 g α-Aminomethyl-4-methoxybenzylalkohol (S.M. Albónico et al., J. Chem. Soc. 1967, 1327) und 7,7 g 5-(2-Oxo-propoxy)-salicylamid in 130 ml Methanol wird unter Zusatz von 0,09 g konz. Schwefelsäure und 1,2 g Platin-auf-Kohle-Katalysator (5 %) analog Beispiel 12 hydriert. Das Rohprodukt wird aus 200 ml Aethylacetat, dann aus Acetonitril umkristallisiert. Man erhält das höher schmelzende Enantiomeren-Paar des α-[N-[2-(3-Carbamoyl-4-hydroxy-phenoxy)-1-methyl-äthyl]-aminomethyl]-4-methoxy-benzylalkohol, Smp. 144-146°. Aus der Aethylacetat-Mutterlauge wird durch Einengen das andere Enantiomeren-Paar erhalten und durch Kristallisation aus Acetonitril gereinigt, Smp. 129-131°.

Beispiel 18: Eine Lösung von 7 g α-(Aminomethyl)-3-sulfamoyl-4-chlorbenzylalkohol und 5,8 g 5-(2-Oxo-propoxy)-salicylamid in 130 ml

Methanol wird mit 70 mg konz. Schwefelsäure versetzt und anschliessend unter Stickstoff 2 Stunden bei Raumtemperatur geschüttelt. Man setzt 0,7 g Platin-auf-Kohle-Katalysator (5 %) zu und hydriert unter Normaldruck bis zum Stillstand der Wasserstoffaufnahme. Der Katalysator wird abfiltriert, das Filtrat vom Lösungsmittel befreit und der zurückbleibende Schaum aus Isopropanol, dann aus Methanol/Isopropanol umkristallisiert, wonach man den α-[N-[2-(3-Carbamoyl-4-hydroxy-phenoxy)-1-methyl-äthyl]-aminomethyl]-3-sulfamoyl-4-chlor-benzylalkohol als ca. 1:1 Diastereomerengemisch vom Smp. 103-106° mit 0,5 Mol Kristallisopropanol erhält.

Der als Ausgangsmaterial benötigte α-(Aminomethyl)-3-sulfamoyl-4-chlorbenzylalkohol kann wie folgt erhalten werden:

a) Zu einer Lösung von 28 g 5-(Bromacetyl)-2-chlorbenzolsulfonamid (DE-OS 26 01 598) in 40 ml Dimethylformamid werden bei 0-5° im Verlaufe von 30 Minuten portionenweise 8 g Natriumazid zugegeben. Man rührt weitere 2 Stunden bei dieser Temperatur und giesst das Reaktionsgemisch anschliessend auf ein Eis/Wasser-Gemisch. Das dabei auskristallisierende 5-(Azidoacetyl)-2-chlor-benzolsulfonamid, welches sich ab 152° zersetzt, wird abgesaugt, mit viel Wasser gewaschen und im Hochvakuum getrocknet. Das erhaltene Rohprodukt wird direkt in der nächsten Stufe eingesetzt.

b) Eine Lösung von 29,5 g rohem 5-(Azidoacetyl)-2-chlorbenzolsulfonamid in 800 ml Methanol und 46,4 ml Salzsäure wird in Gegenwart von 3 g eines Platin-auf-Kohle-Katalysators (5 %) bei Raumtemperatur und Normaldruck bis zur beendeten Wasserstoffaufnahme hydriert. Der Katalysator wird abfiltriert, das Filtrat eingedampft und der Rückstand in Aceton aufgeschlämmt. Das erhaltene 5-(Aminoacetyl)-2-chlorbenzolsulfonamid-hydrochlorid wird abfiltriert und als Rohprodukt in der nächsten Stufe eingesetzt.

c) 5 g 5-(Aminoacetyl)-2-chlorbenzolsulfonamid-hydrochlorid werden in 100 ml Methanol aufgeschlämmt und portionenweise mit 1,6 g Natriumborhydrid versetzt. Die Lösung wird 1/2 Stunde bei Raumtemperatur gerührt, am Rotationsverdampfer eingedampft und der Rückstand mit Aethylacetat und Wasser versetzt. Die organische Phase wird über Natriumsulfat getrocknet, eingedampft und der Rückstand aus Aethylacetat umkristallisiert, wobei man den α-(Aminomethyl)-3-sulfamoyl-4-chlorbenzylalkohol vom Smp. 179-182° erhält.

Beispiel 19: Analog der im Beispiel 18 beschriebenen Verfahrensweise erhält man aus α-(Aminomethyl)-3-sulfamoyl-4-chlorbenzylalkohol und 4-(2-Oxo-propoxy)-salicylamid den α-[N-[2-(3-Hydroxy-4-carbamoyl-phenoxy)-1-methyl-äthyl]-aminomethyl]-3-sulfamoyl-4-chlor-benzylalkohol vom Smp. 112-116°, der als Diastereomerengemisch (ca. 1:1) vorliegt.

Beispiel 20: Eine Lösung von 20 g rohem α-[N-[2-(3-Carbamoyl-4-hydroxy-phenoxy)-äthyl]-benzylaminomethyl]-3-sulfamoyl-4-methoxy-benzylalkohol in 200 ml Methanol wird nach Zusatz von 2 g Palladium-auf-Kohle-Katalysator (5 %) bis zur Aufnahme der berechneten Menge Wasserstoff hydriert. Durch Filtration und Eindampfen des Filtrats erhält man einen dickflüssigen Rückstand, aus dem durch Lösen in einem Methanol-Isopropanol-Gemisch der α-[N-[2-(3-Carbamoyl-4-hydroxy-phenoxy)-äthyl]-aminomethyl]-3-sulfamoyl-4-methoxy-benzylalkohol auskristallisiert, welcher nach dem Umkristallisieren aus Methanol den Smp. 160-162° zeigt. Eine Kristallmodifikation vom Smp. 191-194° entsteht, wenn die Verbindung aus Acetonitril umkristallisiert wird.

Das Ausgangsmaterial kann auf folgende Weise hergestellt werden:

a) 2-Methoxy-5-(bromacetyl)-benzolsulfonamid wird in Methanol mit der 5-fachen molaren Menge Natriumborhydrid zum 5-(Epoxyäthyl)-2-methoxy-benzolsulfonamid reduziert (Smp. 158-160° aus Methanol).

b) Eine Lösung von 7,7 g 5-(Epoxyäthyl)-2-methoxy-benzolsulfonamid und 8,6 g des nach Beispiel 1c) erhaltenen 5-[(2-Benzylamino)-äthoxy]-salicylamid in 150 ml Isopropanol wird 20 Stunden unter Rückfluss gekocht. Durch Eindampfen der Lösung erhält man den α-[N-[2-(3-Carbamoyl-4-hydroxy-phenoxy)-äthyl]-benzylaminomethyl]-3-sulfamoyl-4-methoxybenzylalkohol als orangefarbenes Harz, das ohne weitere Reinigung weiterverarbeitet wird.

Beispiel 21: Eine Lösung von 5,5 g 1-[N-Benzyl-2-(3-carbamoyl-4-hydroxy-phenoxy)-äthylamino]-4-(2-methoxyphenyl)-2-butanol in 60 ml Methanol wird unter Zusatz von 0,6 g Palladium-auf-Kohle-Katalysator (5%) unter Normalbedingungen bis zur beendeten Wasserstoffaufnahme hydriert, wobei das Reduktionsprodukt teilweise aus der Lösung ausfällt. Die Suspension wird mit 400 ml Methanol verdünnt, erwärmt und durch ein Filterhilfsmittel filtriert. Das Filtrat wird mit einem Ueberschuss methanolischer Salzsäure versetzt und am Rotationsverdampfer vom Lösungsmittel befreit. Der Rückstand wird aus Acetonitril/Aethylacetat umkristallisiert wonach man 1-[2-(3-Carbamoyl-4-hydroxy-phenoxy)-äthylamino]-4-(2-methoxyphenyl)-2-butanol-hydrochlorid vom Smp. 124-126° erhält.
Das als Ausgangsmaterial benötigte 1-[N-Benzyl-2-(3-carbamoyl-4-hydroxy-phenoxy)-äthylamino]-4-(2-methoxyphenyl)-2-butanol kann wie folgt hergestellt werden:

a) Zu einer Lösung von 5 g 1-(3-Butenyl)-2-methoxybenzol (vgl. W.S. Johnson: JACS 86, 1975, (1964) in 25 ml Methylenchlorid wird unter Rühren bei Raumtemperatur eine Lösung von 7,3 g m-Chlorperbenzoesäure in 60 ml Methylenchlorid langsam zugetropft. Nach 21/2 Stunden wird abfiltriert, das Filtrat mit 10%-iger Natriumsulfitlösung gewaschen, über Natriumsulfat getrocknet und eingeengt. Das zurückbleibende rohe 1-(3,4-Epoxybutyl)-2-methoxybenzol wird als solches weiterverarbeitet.

b) 2,45 g rohes 1-(3,4-Epoxybutyl)-2-methoxybenzol und 3,9 g des nach Beispiel 4b erhaltenen 4-[2-(Benzylamino)-äthoxy]-salicylamid werden in 65 ml Isopropanol gelöst und die Lösung während 80 Stunden unter Rückfluss erhitzt. Anschliessend wird das Lösungsmittel entfernt und der Rückstand über 300 g Kieselgel mit einem Gemisch von Chloroform/Methanol/Ammoniak = 350:50:1 chromatographiert, wobei man das 1-[N-Benzyl-2-(4-hydroxy-3-carbamoyl-phenoxy)-äthyl-amino]-4-(2-methoxyphenyl)-2-butanol als gelbliches Oel erhält, welches als solches weiterverarbeitet wird.

Beispiel 22: Eine Lösung von 2,9 g 5-(2-Aminoäthoxy)-salicylamid in 10 ml Dimethylsulfoxid wird mit 2,7 g (2,3-Epoxypropyl)-benzol versetzt und hierauf 40 Minuten in einem ca. 90° warmen Bad unter Rühren erhitzt. Diese Lösung wird abgekühlt und mit 40 ml Wasser verdünnt. Die ausgefallenen Kristalle werden abgesaugt, mit wenig Wasser gewaschen und im Vakuum getrocknet. Man erhält so das 1-[2-(3-Carbamoyl-4-hydroxy-phenoxy)-äthylamino]-3-phenyl-2-propanol vom Smp. 142-143° (aus wenig Isopropanol).

Beispiel 23: Eine Lösung von 11,4 g 1-[2-(3-Carbamoyl-4-hydroxy-phenoxy)-N-benzyl-äthylamino]-3-(2-methoxyphenyl)-2-propanol in 130 ml Methanol wird bei Raumtemperatur in Gegenwart von 1,2 g Palladium-auf-Kohle-Katalysator bis zur beendeten Wasserstoffaufnahme hydriert. Man filtriert vom Katalysator ab und wäscht den Filterrückstand mit viel Methanol nach, dampft das Filtrat unter vermindertem Druck zur Trockne, kristallisiert den Rückstand aus Methanol um, suspendiert die erhaltene Kristalle in Methanol und versetzt die Suspension mit methanolischer-Salzsäure, dampft die Lösung unter vermindertem Druck zur Trockne und kristallisiert den Rückstand aus Acetonitril um. Man erhält das 1-[2-(3-Carbamoyl-4-hydroxy-phenoxy)-äthylamino]-3-(2-methoxyphenyl)-2-propanol als Hydrochlorid vom Smp. 152-154°.

Das Ausgangsmaterial kann wie folgt hergestellt werden:
Eine Lösung von 5 g 2-Methoxy-1-(2,3-epoxypropyl)-benzol (vgl. Compt. rendues, 219, 163-64 (1944)) und 8,7 g 5-(2-N-Benzylaminoäthoxy)-salicylamid in 130 ml Isopropanol wird während 20 Stunden unter Rückfluss gekocht. Anschliessend wird das Lösungsmittel am Rotationsverdampfer entfernt, der Rückstand an 500 g Kieselgel chromatographiert und mit einem Gemisch von Chloroform: Methanol, Ammoniak 350:50:1 eluiert. Die Fraktionen 4-20 werden gesammelt, vereinigt und zur Trockne verdampft, wonach man das 1-[2-(3-Carbamoyl-4-hydroxy-phenoxy)-N-benzyl-äthylamino]-3-(2-methoxyphenyl)-2-propanol als öligen Rückstand erhält.

Beispiel 24: 4,18 g α-Aminomethyl-2-methoxy-benzylalkohol werden in 250 ml Methanol und 4,28 ml 5,9-n. methanolischer Salzsäure gerührt (~pH6). Die Reaktionslösung wird mit 0,59 g Kaliumhydroxid (fest) versetzt (~pH9). Nach Zugabe von 25 g Molekularsieb 3 Å und 5,60 g (2,3-Dihydro-2,2-dimethyl-4H-1,3-benzoxazin-4-on-6-yloxy)-acetaldehyd wird eine Lösung aus 0,59 g Natriumcyanoborhydrid in 60 ml Methanol zugetropft. Das Reaktionsgemisch wird 24 Stunden bei Raumtemperatur gerührt, filtriert und mit 50 ml 5,9-n. methanolischer Salzsäure 1 Stunde unter Rückfluss gekocht. Die Reaktionslösung wird vom Lösungsmittel befreit und der Rückstand über 500 g Kieselgel mit einem Gemisch von Chloroform/Methanol/Ammoniak (40:5:0,5) chromatographiert. Die Hauptfraktion wird eingedampft. Durch Kristallisation aus Isopropanol wird der α-[N-[2-(3-Carbamoyl-4-hydroxy-phenoxy)-aethyl]-aminomethyl]-2-methoxy-benzylalkohol vom Smp. 167-168° erhalten.

Das als Ausgangsmaterial benötigte α-Aminomethyl-2-methoxy-benzylalkohol kann wie folgt erhalten werden:

a) Eine Lösung von 13,8 g α-Brom-2-methoxy-acetophenon in 30 ml Dimethylformamid wird unter Rühren bei 0-5° während 30 Minuten mit 5,85 g Natriumazid versetzt. Nach 2 Stunden Rühren bei 0° wird das

Reaktionsgemisch auf 100 ml Eis/Wasser gegossen und das kristalline α-Azido-2-methoxy-acetophenon abgesaugt. Der Filterrückstand wird in 300 ml Toluol gelöst, die Lösung getrocknet ($MgSO_4$) und innerhalb 1 Stunde zu einer Suspension von 10 g Lithiumaluminiumhydrid in 200 ml Tetrahydrofuran bei 0-5° getropft. Das Reaktionsgemisch wird 1 1/2 Stunden bei 0-5° gerührt und dann bei 0-5° tropfenweise mit 10 ml Wasser, 10 ml konz. Natronlauge und dann mit 30 ml Wasser versetzt. Der Niederschlag wird abfiltriert und das Filtrat eingedampft. Der Rückstand wird aus Aethylacetat kristallisiert wobei der α-Aminomethyl-2-methoxy-benzylalkohol vom Smp. 122-123° erhalten wird.

Beispiel 25: Eine Lösung von 100 g rohem 2-[N-Benzyl-N-[2-(4-carbamoyl-3-hydroxy-phenoxy)-äthyl]-amino]-4-methylsulfonylamino-acetophenon in 1000 ml Methanol wird unter Zusatz von 100 ml Essigsäure und 20 g Palladium-auf-Kohle-Katalysator (10 %) bei 30-40° unter Normaldruck bis zum Stillstand der Wasserstoffaufnahme hydriert (80 % der Theorie). Nach Filtration und Abdampfen des Lösungsmittels erhält man ein Oel, welches nach Neutralisation auf pH 3-4 mit 6-n. Salzsäure kristallisiert. Die Kristalle werden abgesaugt und aus Wasser umkristallisiert. Man erhält so den α-[N-[2-(4-Carbamoyl-3-hydroxy-phenoxy)-äthyl]-aminomethyl]-4-methylsulfonylamino-benzylalkohol als Hydrochlorid vom Smp. 239-240°.

Das Ausgangsmaterial kann auf folgende Weise hergestellt werden:

a) Ein Gemisch von 58,6 g 2-Brom-4-methylsulfonylamino-acetophenon, 57,2 g 4-[2-Benzylamino-äthoxy]-salicylamid und 30 g Kaliumbicarbonat wird in 400 ml Dioxan 4 Stunden bei 20-30° gerührt, filtriert und eingedampft. Der Eindampfrückstand wird zwischen Aethylacetat und 2-n. Kaliumbicarbonat-lösung verteilt, die organische Phase abgetrennt und mit 2-n. Salzsäure dreimal extrahiert. Durch Neutralisieren des sauren Extrakts mit Kaliumbicarbonat und Extrahieren mit Aethylacetat erhält man rohes 2-[N-Benzyl-N-[2-(4-carbamoyl-3-hydroxy-phenoxy)-äthyl]-amino]-4-methylsulfonylamino-acetophenon als braunes Oel, welches als solches weiterverarbeitet wird.

Beispiel 26: Analog den Beispielen 25 und 25a erhält man unter Verwendung von 5-[2-Benzylamino-äthoxy]-salicylamid den α-[N-[2-(3-Carbamoyl-4-hydroxyphenoxy)-äthyl]-aminomethyl]-4-methansulfonyl-amino-benzylalkohol als Base vom Smp. 172-174° (aus Aethanol), indem man das eingedampfte Hydriergemisch mit 2-n. Ammoniak-Lösung alkalisch stellt und mit viel Aethylacetat die Base extrahiert.

Beispiel 27: Eine Lösung von 8,5 g α-(Aminomethyl)-4-(N-methyl-sulfamoyl)-benzylalkohol und 7,7 g 4-(2-Oxo-propoxy)-salicylamid wird analog Beispiel 12 hydriert und aufgearbeitet. Durch fraktioniertes Umkristallisieren aus Aethanol und Aethylacetat erhält man 3 Kristallfraktionen, die im Dünnschichtchromatogramm einheitliche Flecken ergeben (System: Aethylacetat, Aethanol, konz. Ammoniak 24:12:4), und die zusammen aus Aethanol umkristallisiert den α-[N-[2-(4-Carbamoyl-3-hydroxy-phenoxy)-1-methyl-äthyl]-aminomethyl]-4-(N-methyl-sulfamoyl)-benzylalkohol als Diastereomerengemisch (3:1) vom Smp. 136-150° ergeben. Durch weiteres Umkristallisieren aus Aethanol erhält man ein reines Diastereomeres vom Smp. 138-140° ($^{13}$C-NMR-Signal bei 15,3 ppm nach Zusatz von Fumarsäure).

Das Ausgangsmaterial kann auf folgende Weise hergestellt werden:

a) 4-Chlorsulfonyl-acetophenon wird mit einem Ueberschuss von Methylamin zu 4-(N-Methyl-sulfamoyl)-acetophenon umgesetzt: Smp. 111-112° (aus Isopropanol).

b) 60 g der erhaltenen Verbindung werden in 1000 ml Essigsäure unter Zusatz von 1 g Aluminiumbromid mit 44,8 g Brom zu 2-Brom-4-(N-methyl-sulfamoyl)-acetophenon umgesetzt, einem gelblichen Oel, das roh weiterverwendet wird.

c) 80 g der erhaltenen Verbindung werden in 760 ml Aceton gelöst und mit 109 g Dibenzylamin versetzt. Das Reaktionsgemisch wird 16 Stunden bei 20-30° gerührt. Das auskristallisierte Dibenzylamin-hydrobromid wird abfiltriert und das Filtrat eingedampft. Der Rückstand wird mit 500 ml Aether versetzt, vom Ungelösten abfiltriert und die Aetherlösung eingedampft. Das so als Oel erhaltene rohe 2-Dibenzylamino-4-(N-methylsulfamoyl)-acetophenon kann als Rohprodukt mit Natriumborhydrid reduziert werden. Durch Umkristallisation aus Isopropanol wird ein reines Produkt vom Smp. 93-94° erhalten.

d) 100 g der erhaltenen rohen Verbindung werden in einem Gemisch von 1800 ml Isopropanol und 200 ml Methanol gelöst und die Lösung unter Rühren und Kühlen bei 10-15° mit 9,5 g Natriumborhydrid protionenweise versetzt. Nach 2-3 Stunden wird das Reaktionsgemisch mit Essigsäure auf pH 5-6 gebracht, unter vermindertem Druck eingedampft und zwischen Aethylacetat und Wasser verteilt. Die organische Phase wird abgetrennt, mit Wasser gewaschen, getrocknet ($MgSO_4$) und eingedampft. Das verbleibende Oel kristallisiert allmählich aus wenig Aether. Man erhält so den α-(Dibenzylaminomethyl)-4-(N-methylsulfamoyl)-benzylalkohol vom Smp. 105-106°.

e) Durch katalytische Debenzylierung der erhaltenen Verbindung in Methanol über Palladium-auf-Kohle-Katalysator erhält man den α-(Aminomethyl)-4-(N-methylsulfamoyl)-benzylalkohol vom Smp. 130-132° (aus Methanol-Toluol).

Beispiel 28: 110,0 g α-(Aminomethyl)-4-(methylsulfonylamino)-benzylalkohol werden mit 100,0 g 4-(2-Oxopropoxy)-salicylamid, wie in Beispiel 12 beschrieben, umgesetzt. Die Hydrierlösung wird auf ca. 300 ml eingeengt und während 15-20 Stunden kristallisieren gelassen.

Die Kristalle des erhaltenen α-[N-[2-(4-Carbamoyl-3-hydroxy-phenoxy)-1-methyläthyl]-aminomethyl]-4-(methylsulfonylamino)-benzylalkohols werden abgesaugt und zeigen einen Smp. von 156-162°. Sie bestehen nach $^{13}$C-NMR-Analyse aus einem Gemisch von 75 % des höher schmelzenden Enantiomeren-Paars A und 25 % des tiefer schmelzenden Enantiomeren-Paars B. Durch weiteres, dreimaliges Umkristallisieren aus Methanol erhält man das Enantiomeren-Paar A vom Smp. 165-167 (> 90 % A). Es bildet ein Hydrochlorid vom Smp. 219-220° (aus Methanol); $^{13}$C-NMR: C-$\underline{CH}_3$-Signal bei 13,15 ppm.

Durch weiteres Einengen der ersten Mutterlauge erhält man eine weitere kristalline Fraktion vom Smp. 138-144°, worin das Diastereomeren-Verhältnis ~1:1 beträgt. Die davon abfiltrierte Mutterlauge kristallisiert nicht mehr. Sie enthält nach $^{13}$C-NMR-Analyse das Enantiomeren-Paar B zu 60-70 % angereichert. Diese Mutterlauge (~80 g braunes Oel) wird über 4 kg Kieselgel chromatographiert. Mit 70 Fraktionen Aethylacetat à 150 ml werden nicht identifizierte Verunreinigungen eluiert. Weitere Elution mit Methanol (80 Fraktionen à 150 ml) ergeben ein braunes Oel, das allmählich zum Teil kristallisiert. Durch mehrmaliges Umkristallisieren aus Aethanol erhält man das Enantiomeren-Paar B vom Smp. 144-145° (>90 % B). Es bildet ein Hydrochlorid vom Smp. 206-209° (aus Aethanol), welches im $^{13}$C-NMR das Signal der C-Methylgruppe bei 13,65 ppm zeigt (>95 %).

Beispiel 29: Eine Lösung von 19,6 g 5-(2-Aminoäthoxy)-salicylamid in 100 ml Dimethylsulfoxid wird mit 19,0 g 3,4-(Epoxybutyl)-benzol 1 Stunde in einem Bad von 120° gerührt und anschliessend auf 200 ml Eiswasser gegossen. Durch dreimaliges Extrahieren mit je 400 ml Aethylacetat, Waschen des Extraktes mit Wasser, Trocknen ($MgSO_4$) und Eindampfen erhält man einen halbfesten Rückstand, aus dem durch Umkristallisation aus wenig Methanol das 1-[2-(3-Carbamoyl-4-hydroxy-phenoxy)-äthylamino]-4-phenyl-2-butanol vom Smp. 159-160° erhalten wird.

Beispiel 30: Eine Lösung von 29 g rohem 1-[N-Benzyl-2-(3-carbamoyl-4-hydroxy-phenoxy)-äthylamino]-4-(2-methyl-phenyl)-2-butanol in Methanol wird über 3,0 g Palladium-auf-Kohle-Katalysator (5 %) bis zum Stillstand der Wasserstoff-Aufnahme unter Normalbedingungen hydriert. Durch Filtration und Eindampfen erhält man rohes 1-[2-(3-Carbamoyl-4-hydroxy-phenoxy)-äthylamino]-4-(2-methylphenyl)-2-butanol, das nach Umkristallisation aus Isopropanol bei 140-142° schmilzt. Es bildet ein Hydrochlorid vom Smp. 155-157° (aus Isopropanol).

Die Ausgangsstoffe können auf folgende Weise erhalten werden:

a) Zu 2-Methyl-benzylmagnesiumbromid (hergestellt aus 92,5 g α-Brom-o-xylol, 12,5 g Magnesium-Spänen in 250 ml absolutem Aether) tropft man eine Lösung von 46,3 g Epichlorhydrin in 175 ml Benzol. Hierauf wird der Aether abdestilliert und das Reaktionsgemisch nach Erreichen von 65° Innentemperatur noch 1 Stunde unter Rückfluss gekocht. Unter Eiskühlung wird das Reaktionsgemisch zuerst mit 50 ml Wasser vorsichtig zersetzt und dann mit 125 ml 2-n. Schwefelsäure sauer gestellt. Unlösliches wird abfiltriert,die Wasserphase zweimal mit je 200 ml Aethylacetat extrahiert, die organischen Phasen vereinigt, mit Wasser gewaschen, getrocknet ($MgSO_4$) und eingedampft. Man erhält rohes 1-Chlor-4-(2-methylphenyl)-2-butanol als gelbes Oel, welches als solches weiterverarbeitet wird.

b) 98 g der erhaltenen Verbindung werden in 700 ml Dichlormethan gelöst, mit 16,7 g Tetrabutylammonium-hydrogensulfat und 700 ml 2-n. Natronlauge versetzt und das Gemisch 7 Stunden bei Raumtemperatur gerührt. Trennen der Schichten, Trocknen ($MgSO_4$) und Eindampfen der Dichlormethan-lösung ergeben ein Oel, das durch Kugelrohrdestillation bei 135-145° Badtemperatur und 0,5 mm Druck zwei Fraktionen ergibt. Die flüchtigere Komponente zeigt im $^1H$-NMR-Spektrum die für 2-(3,4-Epoxy-butyl)-toluol erwarteten Signale: ($CDCl_3$; $\delta$ in ppm) 1,8 (m, 2H); 2,3 (s, 3H); 2,45 (q, 1H); 2,7 (m, 2H); 2,85 (s; 1H)

2,95 (m, 1H); 7,1 (s,4H).

c) Eine Lösung von 15,0 g des erhaltenen Epoxids und 18,6 g 5-(2-Benzylamino-äthoxy)-salicylamid in 130 ml Isopropanol wird 24 Stunden unter Rückfluss gekocht und hierauf eingedampft. Man erhält rohes 1-[N-Benzyl-2-(3-carbamoyl-4-hydroxy-phenoxy)-äthylamino]-4-(2-methylphenyl)-2-butanol als gelbes Oel, welches als solches weiterverarbeitet wird.

Beispiel 31: 20 g Rohes 1-[N-Benzyl-2-(3-carbamoyl-4-hydroxy-phenoxy)-äthylamino]-4-[4-(2-methoxy-äthoxy)-phenyl]-2-butanol wird analog Beispiel 30 katalytisch debenzyliert. Man erhält das 1-[2-(3-Carbamoyl-4-hydroxy-phenoxy)-äthylamino]-4-[4-(2-methoxy-äthoxy)-phenyl]-2-butanol vom Smp. 151-153° (aus Methanol). Es bildet ein Hydrochlorid vom Smp. 207-208° (aus Methanol).

a) 76 g 4-Hydroxybenzoesäure-methylester werden mit 92 g (2-Methoxyäthyl)-methansulfonat in 1500 ml Acetonitril in Anwesenheit von 138 g Kaliumcarbonat durch Kochen während 18 Stunden unter Rückfluss alkyliert, wonach man 4-(2-Methoxyäthoxy)-benzoesäuremethylester vom Smp. 35-38° erhält.

b) 66 g der erhaltenen Verbindung werden mit 12 g Lithiumaluminiumhydrid in 700 ml Tetrahydrofuran reduziert, wonach man rohen 4-(2-Methoxyäthoxy)-benzylalkohol als Oel erhält, der als solcher weiterverwendet wird.

c) In eine Lösung von 49 g der erhaltenen Verbindung in 550 ml Toluol wird während 1 Stunde bei ca. -5° Chlorwasserstoffgas in schnellem Strom eingeleitet. Das Reaktionsgemisch wird anschliessend noch 1 Stunde bei ca. 0 bis 5° gerührt, hierauf mit gesättigter wässriger Natriumchloridlösung gewaschen, getrocknet ($MgSO_4$) und im Vakuum unterhalb 50° eingedampft. Das so erhaltene rohe 4-(2-Methoxyäthoxy)-benzylchlorid wird ohne weitere Reinigung weiterverarbeitet.

d) Aus 45 g der erhaltenen Verbindung und 6,5 g Magnesium-Spänen wird in einem Gemisch von je 100 ml Aether und Tetrahydrofuran die Grignard-Verbindung hergestellt und hierauf analog Beispiel 30a) mit 21 g Epichlorhydrin umgesetzt. Man erhält so rohes 1-Chlor-4-[4-(2-methoxy-äthoxy)-phenyl]-2-butanol als Oel, welches analog Beispiel 30b) zu 1-(3,4-Epoxybutyl)-4-(2-methoxy-äthoxy)-benzol umgesetzt wird. $^1$H-NMR($CDCl_3$): 1,6-1,8 (m); 2,45 (q); 2,8-3,0 (m). Anhand dieser Signale wird ein Gehalt aus Epoxy-Verbindung von 50 % berechnet.

e) Aquivalente Mengen der erhaltenen Verbindung und 5-(2-Benzylamino-äthoxy)-salicylamid werden analog Beispiel 30c) umgesetzt und ergeben das 1-[N-Benzyl-2-(3-carbamoyl-4-hydroxy-phenoxy)-äthylamino]-4-[4-(2-methoxy-äthoxy)-phenyl]-2-butanol als Oel, welches als solches weiterverarbeitet wird.

Beispiel 32: Eine Lösung von 2,1 g rohem α-[N-[2-(4-Carbomethoxy-3-hydroxy-phenoxy)-1-methyläthyl]-aminomethyl]-4-(methylsulfonylamino)-benzylalkohol in 20 ml Dioxan wird mit 40 ml konz. Ammoniaklösung 3 Tage bei 20-30° stehen gelassen. Durch Eindampfen der Lösung erhält man einen Schaum, der aus Aethanol allmählich kristallisiert und den α-[N-[2-(4-Carbamoyl-3-hydroxy-phenoxy)-1-methyl-äthyl]-aminomethyl]-4-(methylsulfonylamino)-benzylalkohol als Diastereoisomeren-Gemisch (ca. 1:1) vom Smp. 141-150° darstellt.

Der Ausgangsstoff kann auf folgende Weise hergestellt werden:

a) Eine Lösung von 3,4 g 2,4-Dihydroxybenzoesäuremethylester, 4,0 g Triäthylamin und 2,8 g Chloraceton in 40 ml Acetonitril wird 16 Stunden unter Rückfluss gekocht. Nach Zusatz von 1,4 g Chloraceton und 1,3 g Triäthylamin wird das Reaktionsgemisch weitere 5 Stunden gekocht, dann eingedampft, der Rückstand in 50 ml Toluol gelöst, die Lösung mit

10 ml Wasser, dann mit 10 ml gesättigter wässeriger Natriumchloridlösung gewaschen und über 200 g Silicagel chromatographiert. Die mit Toluol zuerst eluierten Fraktionen enthalten den 4-(2-Oxo-propoxy)-salicylsäuremethylester der aus Isopropanol umkristallisiert wird und bei 106-108° schmilzt.

b) Eine Lösung von 2,3 g α-(Aminomethyl)-4-(methylsulfonylamino)-benzylalkohol und 1,9 g 4-(2-Oxopropoxy)-salicylsäuremethylester in 50 ml Methanol wird analog Beispiel 12 hydriert. Durch Filtrieren und Eindampfen erhält man rohen α-[N-[2-(3-Carbomethoxy-4-hydroxy-phenoxy)-1-methyl-äthyl]-aminomethyl]-4-(methylsulfonylamino)-benzylalkohol als gelbliches Oel,das allmählich kristallin erstarrt. Es kann roh weiterverarbeitet werden. Durch Umkristallisation aus Aethylacetat-Aether erhält man Kristalle vom Smp. 113-121° (Diastereomeren-Gemisch).

Beispiel 33: 1,2g roher α-[N-[2-(4-Cyan-3-hydroxy-phenoxy)-äthyl]-aminomethyl]-4-methylsulfonylamino-benzylalkohol wird in 15 ml konz. Salzsäure gelöst und die Lösung 24 Stunden bei 20-25° stehen gelassen. Durch Eindampfen im Vakuum erhält den α-[N-[2-(4-Carbamoyl-3-hydroxy-phenoxy)-äthyl]-aminomethyl]-4-methylsulfonylamino-benzylalkohol als Hydrochlorid, das nach Umkristallisation aus Wasser bei 239-240° schmilzt.

Das Ausgangsmaterial kann auf folgende Weise hergestellt werden:

a) Ein Gemisch von 27,4 g 2,4-Dihydroxy-benzonitril, 30,8 g Kaliumcarbonat, 1000 ml 1,2-Dibromäthan und 100 ml Dimethylformamid wird unter Rühren am absteigenden Kühler so erhitzt, dass eine Innentemperatur von 127-129° erreicht wird. Das Erhitzen wird während 6 Stunden fortgesetzt. Das Destillat wird verworfen. Das Reaktionsgemisch wird filtriert und das Filtrat am Vakuum eingedampft. Durch Verteilen des Rückstandes zwischen Aethylacetat und Wasser, Trocknen ($MgSO_4$), Eindampfen der organischen Phase und Kristallisieren erhält

man das 4-(2-Bromäthoxy)-2-hydroxy-benzonitril vom Smp. 160-175° (aus Aethylacetat), das für die folgende Reaktion genügend rein ist.

b) Ein Gemisch von 2,8 g α-(Aminomethyl)-4-methylsulfonylamino-benzylalkohol, 2,4 g 4-(2-Bromäthoxy)-2-hydroxy-benzonitril, 4,1 g Triäthylamin und 20 ml Dioxan wird 3 Stunden unter Rühren und Rückfluss erhitzt. Aufarbeitung analog Beispiel 7 ergibt rohen α-[N-[2-(4-Cyan-3-hydroxy-phenoxy)-äthyl]-aminomethyl-4-methylsulfonylamino-benzylalkohol als braunen Schaum, der roh weiterverarbeitet wird.

Beispiel 34: Eine Lösung von 10,5 g rohem 1-[N-Benzyl-2-(3-carbamoyl-4-hydroxy-phenoxy)-äthylamino]-4-(4-benzyloxy-phenyl)-2-butanol in 55 ml Methanol wird analog Beispiel 30 katalytisch debenzyliert. Man erhält so das 1-[2-(3-Carbamoyl-4-hydroxy-phenoxy)-äthylamino]-4-(4-hydroxyphenyl)-2-butanol.

Das Ausgangsmaterial kann auf folgende Weise hergestellt werden:
a) 18 g 4-Benzyloxy-benzylalkohol wird mit 21 ml Thionylchlorid in 30 ml Pyridin zu 4-Benzyloxy-benzylchlorid chloriert, Smp. der Rohsubstanz 72-78°;

b) 15 g des erhaltenen 4-Benzyloxy-benzylchlorid werden analog Beispiel 31d) zur Grignard-Verbindung und diese mit 7 g Epichlorhydrin zum 1-(3,4-Epoxybutyl)-4-benzyloxy-benzol umgesetzt.

c) Umsetzung von 15 g des erhaltenen Epoxids mit 18,6 g 5-(2-Benzylaminoäthoxy)-salicylamid analog Beispiel 30c) ergibt das rohe 1-[N-Benzyl-2-(3-carbamoyl-4-hydroxy-phenoxy)-äthylamino]-4-(4-benzyloxyphenyl)-2-butanol als Oel, das als solches weiterverarbeitet wird.

Beispiel 35: 8,4 g α-[N-[2-(4-Carbomethoxy-3-hydroxy-phenoxy)-1-methyläthyl]-aminomethyl]-4-(methylsulfonylamino)-benzylalkohol wird in

50 ml Dioxan gelöst und mit 200 ml einer 40 %-igen wässrigen Methylamin-Lösung versetzt. Die Lösung wird 2-3 Tage bei Raumtemperatur stehen gelassen. Aufarbeitung analog Beispiel 32 ergibt den α-[N-[2-(4-Methylcarbamoyl-3-hydroxy-phenoxy)-1-methyl-äthyl]-aminomethyl]-4-(methylsulfonylamino)-benzylalkohol als Diastereomerengemisch.

Beispiel 36: Tabletten enthaltend 20 mg an aktiver Substanz werden in folgender Zusammensetzung in üblicher Weise hergestellt:

Zusammensetzung:

| | |
|---|---|
| α-[N-[2-(3-Carbamoyl-4-hydroxy-phenoxy)-1-methyl-äthyl]-aminomethyl]-3-sulfamoyl-4-chlor-benzylalkohol | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

α-[N-[2-(3-Carbamoyl-4-hydroxy-phenoxy)-1-methyl-äthyl]-aminomethyl]-3-sulfamoyl-4-chlor-benzylalkohol wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 37: Tabletten enthaltend 1 mg an aktiver Substanz werden in folgender Zusammensetzung in üblicher Weise hergestellt:

Zusammensetzung:

| | |
|---|---|
| α-[N-[2-(3-Hydroxy-4-carbamoyl-phenoxy)-1-methyl-äthyl]-aminomethyl]-3-sulfamoyl-4-chlor-benzylalkohol | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

α-[N-[2-(3-Hydroxy-4-carbamoyl-phenoxy)-1-methyl-äthyl]-aminomethyl]-3-sulfamoyl-4-chlor-benzylalkohol wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 38: Kapseln enthaltend 10 mg an aktiver Substanz werden wie folgt auf übliche Weise hergestellt:

Zusammensetzung:

| | |
|---|---|
| α-[N-[2-(3-Carbamoyl-4-hydroxy-phenoxy)-äthyl]-aminomethyl]-3-sulfamoyl-4-chlor-benzylalkohol | 2500 mg |
| Talkum | 200 mg |
| Kolloidale Kieselsäure | 50 mg |

Herstellung:
Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 39: Eine sterile Lösung von 5,0 g α-[N-[2-(3-Carbamoyl-4-hydroxy-phenoxy)-1-methyl-äthyl]-aminomethyl]-3-sulfamoyl-4-chlor-benzylalkohol-methansulfonat in 5000 ml destilliertem Wasser wird in Ampullen zu 5 ml abgefüllt, die in 5 ml Lösung 5 mg Wirkstoff enthalten.

Beispiel 40: 3,62 g α-[N-[2-(3-Carbamoyl-4-hydroxy-phenoxy)-1-methyl-äthyl]-aminomethyl]-3-sulfamoyl-4-chlor-benzylalkohol werden unter Zusatz von 100,0 ml 0,10-n. Salzsäure mit 18000 ml destilliertem Wasser auf ein Volumen von 18100 ml gelöst. Die sterilisierte Lösung wird in Ampullen à 5,0 ml abgefüllt, in denen 1 mg Wirkstoff enthalten ist.

Beispiel 41: Anstelle der in den Beispielen 37 bis 40 als aktive Substanz verwendeten Verbindungen können auch folgende Verbindungen der Formel I, oder deren pharmazeutisch annehmbare nicht-toxische Säureadditionssalze als Wirkstoffe in Tabletten, Dragées, Kapseln, Ampullenlösungen etc. verwendet werden:

α-[N-[2-(3-Carbamoyl-4-hydroxy-phenoxy)-äthyl]-aminomethyl]-2,4-dichlorbenzylalkohol,

α-[N-[2-(3-Carbamoyl-4-hydroxy-phenoxy)-äthyl]-aminomethyl]-4-chlor-3-nitro-benzylalkohol,

α-[N-[2-(4-Carbamoyl-3-hydroxy-phenoxy)-äthyl]-aminomethyl]-4-nitro-benzylalkohol,

α-[N-[2-(3-Carbamoyl-4-hydroxy-phenoxy)-äthyl]-aminomethyl]-3-(methylsulfonylamino)-4-chlor-benzylalkohol,

α-[N-[2-(3-Carbamoyl-4-hydroxy-phenoxy)-äthyl]-aminomethyl]-3,5-dichlor-4-amino-benzylalkohol,

α-[N-[2-(3-Carbamoyl-4-hydroxy-phenoxy)-äthyl]-aminomethyl]-3-(methylsulfonylamino)-benzylalkohol,

α-[N-[2-(4-Carbamoyl-3-hydroxy-phenoxy)-1-methyl-äthyl]-aminomethyl]-4-(methylsulfonylamino)-benzylalkohol,

α-[N-[2-(3-Carbamoyl-4-hydroxy-phenoxy)-1-methyl-äthyl]-aminomethyl]-3,4-methylendioxy-benzylalkohol,

α-[N-[2-(4-Carbamoyl-3-hydroxy-phenoxy)-1-methyl-äthyl]-aminomethyl]-3,4-methylendioxy-benzylalkohol,

α-[N-[2-(3-Carbamoyl-4-hydroxy-phenoxy)-1-methyl-äthyl]-aminomethyl]-4-(methylsulfonylamino)-benzylalkohol,

α-[N-[2-(3-Carbamoyl-4-hydroxy-phenoxy)-1-methyl-äthyl]-aminomethyl]-3-carbamoyl-4-hydroxy-benzylalkohol,

α-[N-[2-(3-Carbamoyl-4-hydroxy-phenoxy)-1-methyl-äthyl]-aminomethyl]-4-(2-methoxy-äthoxy)-benzylalkohol,

α-[N-[2-(3-Carbamoyl-4-hydroxy-phenoxy)-1-methyl-äthyl]-aminomethyl]-4-methyl-benzylalkohol,

α-[N-[2-(3-Carbamoyl-4-hydroxy-phenoxy)-1-methyl-äthyl]-aminomethyl]-4-methoxy-benzylalkohol,

α-[N-[2-(3-Carbamoyl-4-hydroxy-phenoxy)-äthyl]- aminomethyl]-3-sulfamoyl-4-methoxy-benzylalkohol.

1-[2-(3-Carbamoyl-4-hydroxy-phenoxy)-äthylamino]-4-(2-methoxyphenyl)-2-butanol,

1-[2-(3-Carbamoyl-4-hydroxy-phenoxy)-äthylamino]-3-phenyl-2-propanol, oder das

1-[2-(3-Carbamoyl-4-hydroxy-phenoxy)-äthylamino-3-(2-methoxyphenyl)-2-propanol

α-[N-[2-(3-Carbamoyl-4-hydroxy-phenoxy)-äthyl]-aminomethyl]-2-methoxy-benzylalkohol,

α-[N-[2-(4-Carbamoyl-3-hydroxy-phenoxy)-äthyl]-aminomethyl]-4-methyl-sulfonylamino-benzylalkohol,

α-[N-[2-(3-Carbamoyl-4-hydroxy-phenoxy)-äthyl]-aminomethyl]-4-methan-sulfonylamino-benzylalkohol,

α-[N-[2-(4-Carbamoyl-3-hydroxy-phenoxy)-1-methyl-äthyl]-aminomethyl]-4-(methylsulfonylamino)-benzylalkohol als Enantiomerenpaar vom Smp. 165-167°, oder als Enantiomerenpaar vom Smp. 144-145°,

1-[2-(3-Carbamoyl-4-hydroxy-phenoxy)-äthylamino]-4-phenyl-2-butanol,

1-[2-(3-Carbamoyl-4-hydroxy-phenoxy)-äthylamino]-(2-methylphenyl)-2-butanol,

1-[2-(3-Carbamoyl-4-hydroxy-phenoxy)-äthylamino]-4-[4-(2-methoxy-äthoxy)-phenyl]-2-butanol.

Patentansprüche

1. Neue Derivate des 2-Amino-äthanols der Formel

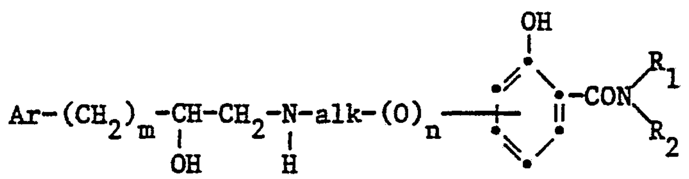

(I)

in welcher Ar gegebenenfalls substituiertes Phenyl, m eine Zahl von 0 bis 3, n die Zahl 0 oder 1 und alk Alkylen mit 2-5 Kohlenstoffatomen bedeuten, wobei das Stickstoffatom und das Sauerstoffatom, oder, falls n für Null steht, der Phenylrest, durch mindestens zwei Kohlenstoffatome voneinander getrennt sind, und $R_1$ und $R_2$ unabhängig voneinander je Wasserstoff oder Niederalkyl, oder zusammen Niederalkylen, Oxaniederalkylen, Thianiederalkylen, Azaniederalkylen oder N-Niederalkylazaniederalkylen darstellt mit der Massgabe, dass, falls m für 0 steht, der Phenylrest Ar mindestens einen Substituenten enthält und ein durch eine oder zwei Hydroxygruppen oder geschützte Hydroxygruppen substituierter Phenylrest Ar mindestens einen zusätzlichen, von diesen verschiedenen Substituenten enthält, in der Form von Racematgemischen, Racematen oder optischen Antipoden.

2. Verbindungen der Formel I, worin Ar gegebenenfalls ein- oder mehrfach, vorzugsweise höchstens dreifach, substituiert ist, wobei als Substituenten gegebenenfalls, insbesondere in untenstehend angegebener Weise, substituiertes Niederalkyl, Niederalkenyl oder Niederalkoxy, weiter Niederalkenyloxy, Niederalkinyl, Niederalkinyloxy, Niederalkylidendioxy, Niederalkylendioxy, Cyan und/oder Nitro, und/oder direkt oder an vorgenanntes Niederalkyl, Niederalkenyl oder Niederalkoxy gebundenes Niederalkanoyl, verestertes oder amidiertes Carboxyl, insbesondere Niederalkoxycarbonyl, Carbamoyl, Niederalkylcarbamoyl oder (Hydroxyniederalkyl)-carbamoyl, Niederalkylsulfinyl, Niederalkylsulfonyl, Sulfamoyl oder Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Acylamino, wie Niederalkanoylamino oder Niederalkoxycarbonylamino, gegebenenfalls durch Niederalkyl, Hydroxyniederalkyl oder Cycloalkyl substituiertes Ureido, Niederalkylsulfonylamino, Hydroxy, Phenylniederalkoxy, z.B. Benzyloxy, Niederalkanoyloxy oder, als nicht direkt gebundenen Substituent, wiederum Niederalkoxy vorliegen können, m für eine Zahl von 0 bis 3 steht, und n für die Zahl 0 oder 1 steht und alk einen Alkylenrest mit 2 bis 4 Kohlenstoffatomen bedeutet, wobei das Stickstoffatom und das Sauerstoffatom oder, falls n für 0 steht, der Phenylrest, durch 2 bis 3 Kohlenstoffatome der Reste alk voneinander getrennt sind, $R_1$ und $R_2$ die unter Formel I angegebene Bedeutung haben, jedoch vorzugsweise für Wasserstoff oder Niederalkyl, z.B. Methyl oder Aethyl, stehen oder zusammen mit dem Stickstoffatom der Amidgruppe Morpholino oder Alkylenamino mit 5 bis 6 Ringgliedern, wie 1-Pyrrolidinyl oder Piperidino bilden, mit der Massgabe, dass, falls m für 0 steht, der Phenylrest Ar mindestens einen Substituenten und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, in der Form von Racematgemischen, Racematen oder optischen Antipoden.

3. Verbindungen der Formel I, worin Ar gegebenenfalls ein- bis dreifach substituiert ist, wobei als Substituenten gegebenenfalls in untenstehend angegebener Weise substituiertes Niederalkyl, Niederalkenyl oder Niederalkoxy, weiter Niederalkenyloxy, Niederalkinyl, Niederalkinyloxy, Niederalkylidendioxy, Cyan und/oder Nitro und/oder direkt oder an vorgenanntes Niederalkyl, Niederalkenyl oder Niederalkoxy gebundenes Niederalkanoyl, Niederalkoxycarbonyl, Carbamoyl, Niederalkylcarbamoyl, (Hydroxyniederalkyl)-carbamoyl, Niederalkylsulfinyl, Niederalkylsulfonyl, Sulfamoyl, Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Niederalkanoylamino, Niederalkoxycarbonylamino, gegebenenfalls durch Niederalkyl, Hydroxyniederalkyl oder Cycloalkyl substituiertes Ureido, Niederalkylsulfonylamino, Phenylniederalkoxy, z.B. Benzyloxy, Niederalkanoyloxy oder, als nicht direkt gebundener Substituent wiederum Niederalkoxy vorliegen können, m für eine Zahl von 0 bis 3 steht, und n die Zahl 0 oder 1 und alk einen Alkylenrest mit 2 bis 4 Kohlenstoffatomen bedeutet, wobei das Stickstoffatom und das Sauerstoffatom, oder, falls n für 0 steht, der Phenylrest, durch 2 bis 3 Kohlenstoffatome des Restes alk voneinander getrennt sind, $R_1$ und $R_2$ die unter Formel I angegebene Bedeutung haben, jedoch vorzugsweise für Wasserstoff oder Niederalkyl, insbesondere Methyl oder Aethyl, stehen oder zusammen mit dem Stickstoffatom der Aminogruppe 1-Pyrrolidinyl, Piperidino oder Morpholino bilden, mit der Massgabe dass, falls m für 0 steht, der Phenylrest Ar mindestens einen Substituenten und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, in der Form von Racematgemischen, Racematen oder optischen Antipoden.

4. Verbindungen der Formel I, worin Ar gegebenenfalls ein- bis dreifach substituiert ist, wobei als Substituenten gegebenenfalls in untenstehend angegebener Weise substituiertes Niederalkyl, Niederalkenyl oder Niederalkoxy, weiter Niederalkenyloxy, Niederalkinyloxy, Niederalkylidendioxy, Nitro und/oder Cyan und/oder direkt oder an vorgenanntes Niederalkyl, Niederalkenyl oder Niederalkoxy gebundenes Niederalkanoyl, Niederalkoxycarbonyl, Carbamoyl, Niederalkylcarbamoyl, (Hydroxyniederalkyl)-carbamoyl, Niederalkylsulfinyl, Niederalkylsulfonyl, Sulfamoyl oder Niederalkylsulfamoyl, und/oder direkt oder an vorgenanntes Niederalkyl oder in höherer als der 1-Stellung an vorgenanntes Niederalkoxy gebundenes Halogen, Niederalkylthio, Amino, Niederalkylamino, Diniederalkylamino, Alkylen- oder Oxaalkylenamino, z.B. 1-Pyrrolidinyl, Piperidino oder Morpholino, Pyrrol-1-yl, Niederalkanoylamino, Niederalkoxycarbonylamino, gegebenenfalls durch Niederalkyl oder Cycloalkyl substituiertes Ureido, Niederalkylsulfonylamino, Hydroxy, Phenylniederalkoxy, z.B. Benzyloxy, Niederalkanoyloxy oder, als nicht direkt gebundener Substituent, wiederum Niederalkoxy vorliegen können, m für eine Zahl von 0 bis 3 steht, mit der Massgabe, dass, falls m für 0 steht, der Phenylester Ar mindestens einen Substituenten, und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält, n die Zahl 0 oder 1 und alk einen Alkylenrest mit 2 bis 4 Kohlenstoffatomen bedeutet, wobei das Stickstoffatom und das Sauerstoffatom, oder, falls n für 0 steht, der Phenylrest, durch 2 bis 3 Kohlenstoffatome des Restes alk voneinander getrennt sind, und $R_1$ und $R_2$ unabhängig voneinander je Wasserstoff oder Niederalkyl, insbesondere Methyl oder Aethyl darstellen, oder zusammen mit dem Stickstoffatom der Amidgruppe Morpholino bilden, in der Form von Racematgemischen, Racematen oder optischen Antipoden.

5. Verbindungen der Formel I, worin Ar gegebenenfalls ein- bis dreifach substituiert ist, wobei als Substituenten gegebenenfalls in untenstehend angegebener Weise substituiertes Niederalkyl oder Niederalkoxy, weiter Niederalkenyl, Niederalkenyloxy, Niederalkinyloxy, Niederalkylidendioxy, Nitro und/oder Cyan und/oder direkt oder an vorgenanntes Niederalkyl oder Niederalkoxy gebundenes Niederalkanoyl, Niederalkoxycarbonyl, Carbamoyl, Niederalkalkylcarbamoyl oder (Hydroxyniederalkyl)-carbamoyl, Niederalkylsulfinyl, Niederalkylsulfonyl, Sulfamoyl und/oder direkt oder an vorgenanntes Niederalkyl oder in höherer als der 1-Stellung an vorgenanntes Niederalkoxy gebundenes Halogen, Niederalkylthio, Amino, Alkylen- oder Oxaalkylenamino, z.B. 1-Pyrrolidinyl, Piperidino oder Morpholino, Pyrrol-1-yl, Niederalkanoylamino oder Niederalkoxycarbonylamino, oder gegebenenfalls durch Niederalkyl substituiertes Ureido, Niederalkylsulfonylamino, Hydroxy, weiter Phenylniederalkoxy, z.B. Benzyloxy, oder, als nicht direkt gebundener Substituent, wiederum Niederalkoxy, vorliegen kann,

m für eine Zahl von 0 bis 3 steht, mit der Massgabe, dass, falls m für 0 steht, der Phenylrest Ar mindestens einen Substituenten, und ein durch eine oder zwei Hydroxy-, 1-Phenylniederalkoxy- oder Niederalkanoyloxygruppen substituierter Phenylrest Ar mindestens einen zusätzlichen, von diesen verschiedenen Substituenten enthält,
n die Zahl 0 oder 1 und alk einen Alkylenrest mit 2 bis 4 Kohlenstoffatomen bedeutet, wobei das Stickstoffatom und das Sauerstoffatom oder, falls n für 0 steht, der Phenylrest, durch 2 bis 3 Kohlenstoffatome des Restes alk voneinander getrennt sind, und $R_1$ und $R_2$ unabhängig voneinander je Wasserstoff oder Niederalkyl, jedoch vorzugsweise Wasserstoff oder Methyl darstellen, in der Form von Racematgemischen, Racematen oder optischen Antipoden.

6. Verbindungen der Formel I, worin Ar gegebenenfalls ein- bis dreifach substituiert ist, wobei als Substituenten gegebenenfalls in untenstehend angegebener Weise substituiertes Niederalkyl, z.B. Methyl, oder Niederalkoxy, z.B. Methoxy oder Aethoxy, weiter Niederalkenyl, z.B. Allyl, Niederalkenyloxy, z.B. Allyloxy, Niederalkinyloxy, z.B. Propargyloxy, Nitro, Niederalkylidendioxy, z.B. Methylendioxy, und/oder Cyan und/oder direkt oder an vorgenanntes Niederalkyl oder Niederalkoxy gebundenes Niederalkanoyl, z.B. Acetyl, Carbamoyl, Niederalkylcarbamoyl, z.B. N-Methylcarbamoyl, oder (Hydroxyniederalkyl)-carbamoyl, z.B. N-Hydroxymethyl-carbamoyl, Niederalkylsulfonyl, z.B. Methylsulfonyl, Sulfamoyl, und/oder direkt oder an vorgenanntes Niederalkyl, z.B. Methyl, gebundenes Fluor oder Chlor, z.B. Trifluormethyl, Hydroxy, oder als direkt gebundene Substituenten Phenylniederalkoxy, z.B. Benzyloxy, Niederalkoxyniederalkoxy, z.B. 2-Methoxyäthoxy, Benzyloxy, Amino, Niederalkanoylamino, z.B. Acetylamino, Niederalkoxycarbonylamino, z.B. Methoxycarbonylamino, oder Niederalkylsulfonylamino, z.B. Methylsulfonylamino vorliegen können, m für eine Zahl von 0 bis 3 steht, mit der Massgabe, dass, falls m für 0 steht, der Phenylrest Ar mindestens einen Substituenten, und ein durch ein oder zwei Hydroxy- oder 1-Phenylniederalkoxygruppen substituierter Phenylrest Ar mindestens einen zusätzlichen, von diesen verschiedenen Substituenten enthält,

n die Zahl 1 und alk einen Alkylenrest mit 2 bis 4 Kohlenstoffatomen bedeutet, wobei das Stickstoffatom und das Sauerstoffatom durch 2 bis 3 Kohlenstoffatome des Restes alk voneinander getrennt sind, und $R_1$ und $R_2$ Wasserstoff darstellen, und der die Amid- und die benachbarte Hydroxygruppe tragende Phenylrest vorzugsweise in seiner 4- oder 5-Stellung mit dem restlichen Molekül verbunden ist, in der Form von Racematgemischen, Racematen, oder optischen Antipoden.

7. α-[N-[2-(4-Carbamoyl-3-hydroxy-phenoxy)-1-methyl-äthyl]-aminomethyl]-4-(methylsulfonylamino)-benzylalkohol.

8. α-[N-[2-(4-Carbamoyl-3-hydroxy-phenoxy)-1-methyl-äthyl]-aminomethyl]-4-(methylsulfonylamino)-benzylalkohol als Enantiomerenpaar vom Smp. 165-167°, oder als Hydrochlorid vom Smp. 219-220°.

9. α-[N-[2-(4-Carbamoyl-3-hydroxy-phenoxy)-1-methyl-äthyl]-aminomethyl]-4-(methylsulfonylamino)-benzylalkohol als Enantiomerenpaar vom Smp. 144-145°, oder als Hydrochlorid vom Smp. 206-209°.

10. Salze von Verbindungen der Ansprüche 1 bis 9.

11. Pharmazeutisch annehmbare, nicht-toxische Säureadditionssalze von Verbindungen der Ansprüche 1 bis 9.

12. Pharmazeutische Präparate enthaltend eine Verbindung der Ansprüche 1 bis 9 und 11 zusammen mit inerten Hilfs- und Trägerstoffen.

13. Verbindungen der Formel I als Blocker cardialer β-Rezeptoren.

14. Verwendung von Verbindungen der Formel I zur Herstellung von Arzneimitteln.

15. Verwendung von Verbindungen der Formel I zur Bekämpfung von Rhythmusstörungen, (Arrhythmien), Angina pectoris, und der Hypertonie.

16. Verfahren zur Herstellung von neuen Derivaten des 2-Aminoäthanols der Formel

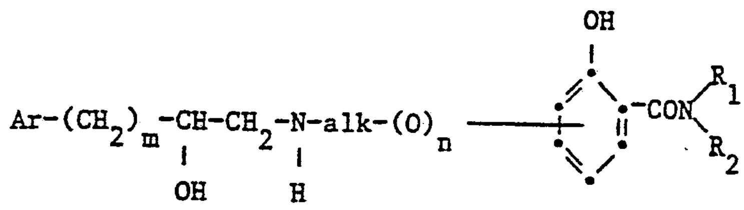

(I)

in welcher Ar gegebenenfalls substituiertes Phenyl, m eine Zahl von 0 bis 3, n die Zahl 0 oder 1 und alk Alkylen mit 2-5 Kohlenstoffatomen bedeuten, wobei das Stickstoffatom und das Sauerstoffatom oder, falls n für Null steht, der Phenylrest durch mindestens zwei Kohlenstoffatome voneinander getrennt sind, und $R_1$ und $R_2$ unabhängig voneinander je Wasserstoff oder Niederalkyl, oder zusammen Niederalkylen, Oxaniederalkylen, Thianiederalkylen, Azaniederalkylen oder N-Niederalkylazaniederalkylen darstellt mit der Massgabe, dass falls m für 0 steht, der Phenylrest Ar mindestens einen Substituenten enthält und ein durch eine oder zwei Hydroxygruppen oder geschützte Hydroxygruppen substituierter Phenylrest Ar mindestens einen zusätzlichen, von diesen verschiedenen Substituenten enthält, in der Form von Racematgemischen, Racematen, optischen Antipoden oder deren Salzen, dadurch gekennzeichnet, dass man

a) eine Verbindung der Formel

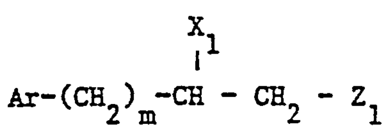

(II)

mit einer Verbindung der Formel

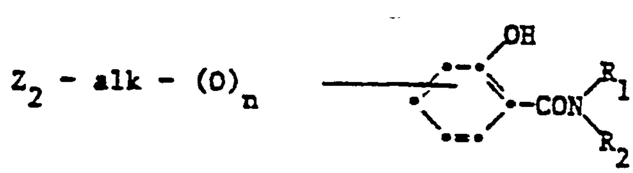

(III)

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, Ar, alk, m und n obige Bedeutung haben, umsetzt, oder

b) in einer Verbindung der Formel

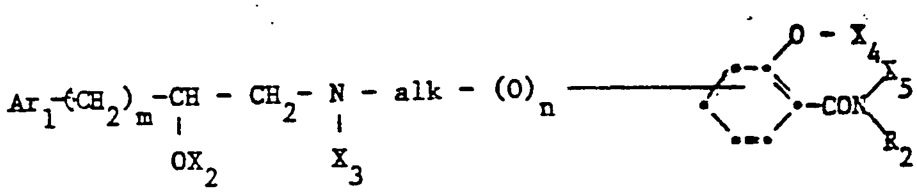 (IV),

worin $Ar_1$ die Bedeutung von Ar hat oder einen Rest Ar bedeutet, der durch jeweils 1 bis 2 in Hydroxy und/oder Amino überführbare Gruppen substituiert ist, $X_2$, $X_3$ und $X_4$ jeweils Wasserstoff oder einen durch Wasserstoff ersetzbaren Substituenten bedeuten und $X_5$ für $R_1$ steht, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$ und $X_4$ von Wasserstoff verschieden ist, oder mindestens $Ar_1$ einen Rest Ar bedeutet, der durch jeweils 1 bis 2 in Hydroxy und/oder Amino überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen, und m und n obige Bedeutungen haben, oder in einem Salz davon, das von Wasserstoff verschiedene $X_2$, $X_3$, oder $X_4$, bzw. $X_2$ und $X_3$ zusammen oder $X_4$ und $X_5$ zusammen durch Wasserstoff ersetzt, und/oder in einem Rest $Ar_1$ vorhandenes substituiertes Hydroxy und/oder Amino in freies Hydroxy und/oder Amino überführt, oder

c) in einer Verbindung der Formel

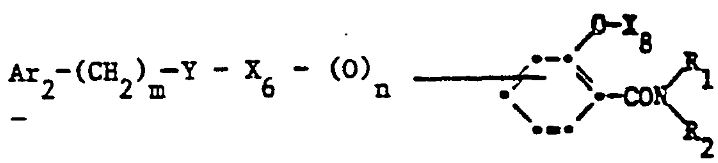 (V),

worin $X_6$ eine reduzierbare Gruppe der Formeln $-CH = N - Alk -$ (Va), bzw. $-CH_2 - N = alk_1-$ (Vb) oder $- C (=X_7) - N (X_8) - alk -$ (Vc) bzw. $- CH_2 - N (X_8) - C (=X_7) - alk_2 -$ (Vd) oder eine Gruppe $-CH_2 - N (X_8) - alk -$ (Ve) ist, wobei $alk_1$ für den einem Rest alk entsprechenden Alkyl-ylidenrest steht und $alk_2$ einem um eine an das Stickstoffatom gebundene Methylengruppe verkürzten Rest alk entspricht, $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt und Y für einen Rest der Formel $-CO-$ (Vf) oder $-CH(OX_8)-$ (Vg) steht, in der $X_8$ die vorstehend angegebene Bedeutung hat, $Ar_2$ einem Rest Ar entspricht, jedoch gegebenenfalls anstelle von jeweils einem oder zwei Hydroxy und/oder Amino eine oder zwei Gruppen$-OX_8$, und/oder $-N(X_8)-$, in welchen $X_8$ die vorstehend angegebene Bedeutung hat, trägt, m und n obige Bedeutungen haben, wobei stets $X_6$ eine reduzierbare Gruppe Va bis Vd und/oder Y eine Carbonylgruppe Vf ist, diese (Gruppe(n) reduziert und im gleichen Arbeitsgang die von Wasserstoff verschiedenen Gruppen $X_8$ durch Wasserstoff ersetzt, oder

d) eine Verbindung der Formel

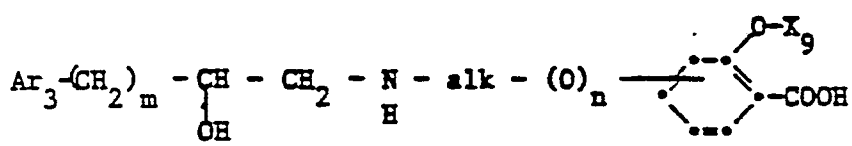

(VI)

worin $Ar_3$ die Bedeutung von Ar hat, oder einen Rest Ar darstellt, der durch 1 bis 2 mittels Aminolyse in Hydroxy und/oder Amino überführbare und/oder durch Gruppen der Formel $-COOH$ substituiert ist, $X_9$ Wasserstoff oder eine mittels Aminolyse abspaltbare Gruppe bedeutet, und m und n obige Bedeutungen haben, oder ein reaktionsfähiges Derivat einer der in Formel VI definierten Carbonsäuren, mit einer Verbindung der Formel $HNR_1R_2$ (VII) umsetzt und gleichzeitig gegebenenfalls vorhandene, mittels Aminolyse in Hydroxy

und/oder Amino überführbare Reste in Hydroxy und/oder Amino überführt, und/oder gegebenenfalls vorhandene Reste $X_9$ abspaltet und durch Wasserstoff ersetzt, oder

e) zur Herstellung von Verbindungen der Formel I, worin $R_1$ und $R_2$ jeweils Wasserstoff bedeuten, in einer Verbindung der Formel

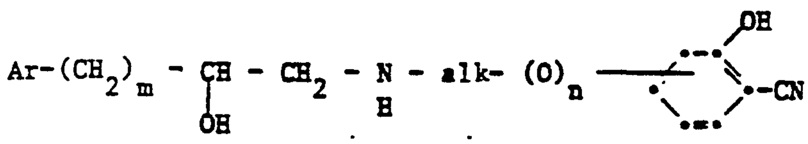 (VII),

worin eine oder beide Hydroxygruppen und/oder im Rest Ar vorhandene Hydroxy- und/oder Aminogruppen gegebenenfalls durch solche mittels Hydrolyse abspaltbaren und durch Wasserstoff ersetzbaren Gruppen geschützt sind, die unter den Bedingungen des Verfahrens abgespalten und durch Wasserstoff ersetzt werden, oder im Rest Ar gegebenenfalls zusammen mit den genannten geschützten Hydroxy- und/oder Aminogruppen die Gruppe -CN vorhanden ist, die Gruppen -CN mittels Hydrolyse in die Gruppe $-CONH_2$ und gleichzeitig gegebenenfalls geschützte Hydroxy- und/oder Aminogruppen in freie Hydroxy- und/oder Aminogruppen umwandelt, oder

f) zur Herstellung von Verbindungen der Formel I, worin m für 1, 2 oder 3 steht, in einer Verbindung der Formel

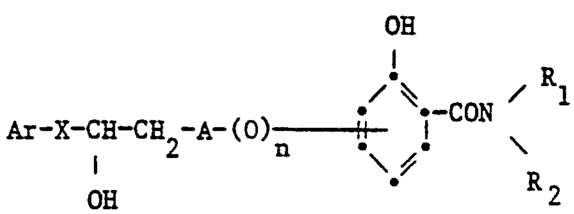 (VIII)

worin A für die Gruppe $-\underset{H}{N}-$alk-oder $-N{=}alk_1-$ steht, worin $alk_1$ die eine Methylengruppe weniger aufweisende Gruppe alk darstellt, und X eine mittels Reduktion, einschliesslich Hydrogenolyse, in eine Methylen-, 1,2-Aethylen- oder 1,3-Propylengruppe überführbare Gruppe darstellt, und eine oder beide Hydroxygruppen und/oder im Rest Ar gegebenenfalls vorhandene Hydroxy- und/oder Aminogruppen

durch solche mittels Reduktion, einschliesslich Hydrogenolyse, abspaltbaren und durch Wasserstoff ersetzbaren Gruppen geschützt sind, die unter den Bedingungen des Verfahrens abgespalten und durch Wasserstoff ersetzt werden, die Gruppe X mittels Reduktion einschliesslich Hydrogenolyse in eine Methylen-, 1,2-Aethylen- oder 1,3-Propylengruppe und gleichzeitig eine gegebenenfalls vorhandene ungesättigte Gruppe A in die gesättigte Gruppe A, und gleichzeitig gegebenenfalls vorhandene geschützte Hydroxy- und/oder Aminogruppen in die freien Hydroxy- und/oder Aminogruppen umwandelt, und, wenn erwünscht, eine erhaltene Verbindung in eine andere Verbindung der Formel I umwandelt, und/oder, wenn erwünscht, eine erhaltene freie Verbindung in ein Salz oder ein erhaltenes Salz in eine freie Verbindung überführt, und/oder, wenn erwünscht, ein erhaltenes Racematgemisch in die Racemate oder ein erhaltenes Racemat in die optischen Antipoden auftrennt.

0030030

Europäisches Patentamt

# EUROPÄISCHER RECHERCHENBERICHT

Nummer der Anmeldung

EP 80107499.8

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| | GB - A - 1 541 933 (SCHERICO LTD)<br>+ Ansprüche +<br>-- | 12-15 | C 07 C 143/80<br>C 07 C 103/29<br>C 07 C 143/74<br>C 07 D 317/58<br>C 07 C 103/85<br>A 61 K 31/16 |
| | DE - A1 - 2 843 016 (YAMANOUCHI) (26-04-1979)<br>+ Beispiele 21,23; Ansprüche 6,8 +<br>-- | 1,12, 16 | |
| P | EP - A1 - 0 005 848 (CIBA-GEIGY AG) (12-12-1979)<br>+ Ansprüche 1,29,30,32,33 +<br>& IL-A-57471 (30-11-1979)<br>& PT-A-69723 (01-06-1979)<br>---- | 1,11, 12,16 | RECHERCHIERTE SACHGEBIETE (Int Cl.³)<br>C 07 C 103/00<br>C 07 C 143/00<br>A 61 K 31/00 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| WIEN | 20-02-1981 | HOFBAUER |

EPA form 1503.1 06.78